(19) Europäisches Patentamt
European Patent Office
Office européen des brevets

(11) EP 4 556 478 B1

(12) EUROPEAN PATENT SPECIFICATION

(45) Date of publication and mention of the grant of the patent:
**24.06.2026 Bulletin 2026/26**

(21) Application number: **23851819.5**

(22) Date of filing: **08.08.2023**

(51) International Patent Classification (IPC):
***C07D 487/04*** *(2006.01)* ***C07D 519/00*** *(2006.01)*
***A61K 31/4439*** *(2006.01)* ***A61K 31/444*** *(2006.01)*
***A61P 35/00*** *(2006.01)* ***A61P 19/02*** *(2006.01)*
***A61P 19/10*** *(2006.01)* ***A61P 31/12*** *(2006.01)*
***A61P 3/00*** *(2006.01)* ***A61P 9/00*** *(2006.01)*
***A61P 13/12*** *(2006.01)* ***A61P 17/00*** *(2006.01)*

(52) Cooperative Patent Classification (CPC):
**C07D 487/04; A61P 9/00; C07D 519/00**

(86) International application number:
**PCT/CN2023/111704**

(87) International publication number:
**WO 2024/032598 (15.02.2024 Gazette 2024/07)**

# (54) TGF-BETA INHIBITOR COMPOUND AND USE THEREOF

TGF-BETA-HEMMERVERBINDUNG UND VERWENDUNG DAVON

COMPOSÉ INHIBITEUR DE TGF-BETA ET SON UTILISATION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.08.2022 CN 202210943348**

(43) Date of publication of application:
**21.05.2025 Bulletin 2025/21**

(73) Proprietor: **Medinno Pharmaceutical Technology (Zhuhai) Co., Ltd.**
**Zhuhai, Guangdong 519000 (CN)**

(72) Inventors:
- **LU, Liang**
  **Zhengzhou, Henan 450000 (CN)**
- **ZHAO, Saisai**
  **Zhengzhou, Henan 450000 (CN)**
- **ZHANG, Longzheng**
  **Zhengzhou, Henan 450000 (CN)**
- **HUANG, Hai**
  **Zhengzhou, Henan 450000 (CN)**
- **ZHANG, Jixuan**
  **Zhengzhou, Henan 450000 (CN)**
- **ZHU, Junjie**
  **Zhengzhou, Henan 450000 (CN)**
- **WANG, Xiaolong**
  **Zhengzhou, Henan 450000 (CN)**
- **CHEN, Jiaxin**
  **Zhengzhou, Henan 450000 (CN)**
- **LING, Shancun**
  **Zhengzhou, Henan 450000 (CN)**
- **LIAO, Xinwei**
  **Zhengzhou, Henan 450000 (CN)**

(74) Representative: **Kraus & Lederer PartGmbB**
**Thomas-Wimmer-Ring 15**
**80539 München (DE)**

(56) References cited:
**WO-A1-2004/013135** **WO-A1-2004/016606**
**WO-A1-2018/086609** **WO-A1-2021/027594**
**CN-A- 111 606 908** **CN-A- 112 341 477**
**CN-A- 114 075 200** **CN-A- 114 380 818**
**CN-A- 114 524 818**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

The present disclosure provides a class of novel compounds with pharmacological activity, which may be used to inhibit fibroblast growth factor receptor, TGF. The present disclosure further relates to a composition comprising the compound, and use of the compound and the composition in the preparation of a medicament for the treatment and/or prevention of TGF-β -related diseases or disorders.

### BACKGROUND

Transforming Growth Factor-β (TGF-β) is a multifunctional cytokine that participates in the regulation of cell proliferation, differentiation, and apoptosis through complex receptor signaling pathways on the cell surface. TGF-β, along with various related proteins such as activins, inhibins, and bone morphogenetic proteins, belongs to the Transforming Growth Factor-β superfamily (TGF-β superfamily, TGF-βs).

TGF-β has three main cell receptors: Type I receptor (TGFβR1), Type II receptor (TGFβR2), and Type III receptor (TGFβR3). The Type I and Type II receptors are transmembrane serine/threonine kinases that convey signals simultaneously, while the Type III receptor does not transmit signals, the main function of which is to deliver TGF-β to the Type II receptor, indirectly affecting signal transduction by providing ligands to the receptor II. There are three subtypes of TGF-β (TGF-β1, TGF-β2, and TGF-β3), which exist together with the receptors in most cells. Each subtype is expressed in a tissue-specific and developmentally regulated manner.

TGF-β and related factors such as activins regulate a multitude of cellular processes, including cell cycle arrest in epithelial and hematopoietic cells, control of mesenchymal cell proliferation and differentiation, inflammatory cell recruitment, immunosuppression, wound healing, and extracellular matrix production. Studies have shown that abnormal TGF-β signaling is associated with many diseases, such as cancer, renal fibrosis, liver fibrosis, pulmonary fibrosis, viral infections, chronic glomerulonephritis, acute glomerulonephritis, diabetic nephropathy, osteoporosis, arthritis, wound healing, ulcers, corneal trauma, valvular heart stenosis, congestive cardiac necrosis, neurofunctional impairment, Alzheimer's disease, peritoneal or subcutaneous adhesions, atherosclerosis, and tumor metastatic growth, etc.

Thus, there is a desire to develop a new TGF-β inhibitor for the prevention and/or treatment of various diseases involving this signaling pathway.

The patent applications disclosing TGF-β inhibitors include WO2004013135, WO2004016606 and WO2018086609.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

SUMMARY

### I. Compounds

In a first aspect, the present disclosure provides a compound of Formula (I)

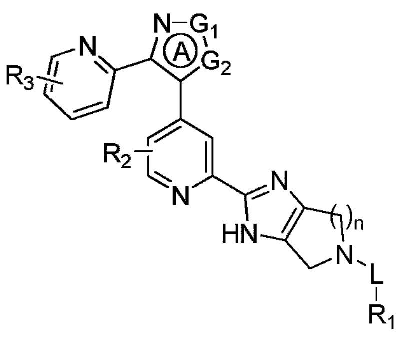

(I)

or an isotopically labeled compound thereof, or an optical isomer thereof, a geometric isomer thereof, a tautomer thereof or a mixture of the optical isomer, geometric isomer, and tautomer, or a pharmaceutically acceptable salt thereof wherein

n is 1;

L is (C=O), (O=S=O), ((C=O)- $CH_2$), $CH_2$ or a bond; and

$R_1$ is selected from H, halogen, -OH, $-NO_2$, -CN, $-SF_5$, -SH, -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicyclic aryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroalicyclic group), -$C_{1-4}$ alkyl-($C_{8-15}$ tricyclic alicyclic group), -$C_{1-4}$ alkyl-(8-15 membered tricyclic heteroalicyclic group), -$C_{1-4}$ alkyl-($C_{5-8}$ aryl), -$C_{1-4}$ alkyl-(5-10 membered heteroaryl), -$NR_{10}$)$R_{11}$), -N($R_{10}$)(C(=O)$R_{11}$), -N($R_{10}$)(C(=O)-O$R_{11}$), -N($R_{12}$)(C(=O)-N($R_{10}$)($R_{11}$)), - C(=O)-N($R_{10}$)($R_{11}$), -C(=O)-$R_{12}$, -C(=O)-O$R_{12}$, -OC(=O)$R_{12}$, -N($R_{10}$)(S(=O)$_2R_{11}$), -S(=O)$_2$-N($R_{10}$)($R_{11}$), -S$R_{12}$, and -O$R_{12}$, wherein the -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicycloaryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroalicyclic group), -$C_{1-4}$ alkyl-($C_{8-15}$ tricyclic alicyclic group), -$C_{1-4}$ alkyl-(8-15 membered tricyclic heteroalicyclic group), -$C_{1-4}$ alkyl-($C_{5-8}$ aryl) and -$C_{1-4}$ alkyl-(5-10 membered heteroaryl) are each optionally substituted with 0, 1, 2, 3 or 4 $R^{1a}$; and $R^{1a}$ is independently selected from halogen, -OH, - $NO_2$, -CN, $-SF_5$, -SH, -S-$C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10 membered heteroalicyclic group, $C_{5-8}$ aryl, 5-7 membered heteroaryl, -N($R_{13}$)($R_{14}$), -N($R_{13}$)(C(=O)$R_{14}$), -N($R_{13}$)(C(=O)-O$R_{14}$), -N($R_{15}$)(C(=O)-N($R_{13}$)($R_{14}$)), -C(=O)-N($R_{13}$)($R_{14}$), -C(=O)-$R_{15}$, -C(=O)-O$R_{15}$, - OC(=O)$R_{15}$, -N($R_{13}$)(S(=O)$_2R_{14}$), -S(=O)$_2$-N($R_{13}$)($R_{14}$), -S$R_{15}$, and -O$R_{15}$; and

$R_{10}$, $R_{11}$, $R_{12}$, $R_{13}$, $R_{14}$ and $R_{15}$, at each occurrence, are each independently selected from H, $C_{1-6}$ alkyl, $C_{1-6}$ haloalkyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroalicyclic group), - $C_{1-4}$ alkyl-($C_{8-15}$ tricyclic alicyclic group), -$C_{1-4}$ alkyl-(8-15 membered tricyclic heteroalicyclic group), -$C_{1-4}$ alkyl-($C_{5-8}$ aryl), and -$C_{1-4}$ alkyl-(5-10 membered heteroaryl), wherein each option listed in the group is optionally substituted with 0, 1, 2, 3, or 4 substituents each independently selected from a group consisting of: halogen, -OH, $-NH_2$, - NH($CH_3$), -N($CH_3$)$_2$, -CN, $-NO_2$, $-SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy; or $R_{10}$, $R_{11}$, and the atom(s) attached thereto together form a 3-14-membered ring; or $R_{13}$, $R_{14}$, and the atom(s) attached thereto together form a 3-14-membered ring;

1, 2, or 3 $R_2$(s) are present in formula (I), and each $R_2$ is H;

1, 2, or 3 $R_3$(s) are present in formula (I), and each $R_3$ is methyl;

and

the ring A is an aromatic ring containing nitrogen and satisfies: 1) G1 = $NR_4$, G2 = $CR_5$; or 2) G1 = $CR_4$, G2 = $NR_5$; or 3) G1 = $CR_4$, G2 = O or S; wherein $R_4$ and $R_5$ are each independently selected from H, halogen, -CN, -OH, $-NO_2$, $C_{1-6}$ alkyl, $C_{1-6}$ haloalkyl, $C_{1-6}$ alkoxy; or $R_4$ and $R_5$ together with the atom(s) to which they are attached form a 3-8 membered ring.

Unless otherwise specified, the various technical terms used in the present application should be interpreted according to the meanings generally understood by those skilled in the art. To avoid ambiguity, the definitions of some terms are provided below.

Unless otherwise indicated, the "compound" used in the term "a compound as shown by Formula (I)" or "a compound according to the present application" also encompasses any optical isomer, geometric isomer, tautomer or a mixture of various isomers of the compound.

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers may be separated by a chiral chromatography or by chiral synthesis.

The term "geometric isomer" refers that when a double bond is present in a compound, the compound may exist as a cis isomer, a trans isomer, an E isomer, or a Z isomer. A geometric isomer comprises a cis isomer, trans isomer, E isomer, Z isomer, or a mixture thereof.

The term "tautomer" refers to an isomer that is formed by rapid movement of an atom at two positions in a single

molecule. It will be understood by those skilled in the art that tautomers may be mutually transformed, and in a certain state, may coexist by reaching an equilibrium state.

Unless otherwise indicated, reference to "a compound as shown by Formula (I)" or "a compound according to the present application" herein also encompasses isotopically-labeled compounds obtained by replacing any atom of the compound with its isotopic atom.

Examples of isotopes suitable for inclusion in the compounds of the invention include isotopes of hydrogen, such as $^{2}H$ (D) and $^{3}H$ (T), of carbon, such as $^{11}C$, $^{13}C$ and $^{14}C$, of chlorine, such as $^{36}Cl$, of fluorine, such as $^{18}F$, of iodine, such as $^{123}I$ and $^{125}I$, of nitrogen, such as $^{13}N$ and $^{15}N$, of oxygen, such as $^{15}O$, $^{17}O$ and $^{18}O$, and of sulphur, such as $^{35}S$.

The isotopically-labelled compounds, for example, those incorporating a radioactive isotope, are useful in drug and/or substrate tissue distribution studies. The radioactive isotopes deuterium, i.e. $^{2}H$, and carbon-14, i.e. $^{14}C$, are particularly useful for this purpose in view of their ease of incorporation and ready means of detection.

Substitution with heavier isotopes such as deuterium, i.e. D, may afford certain therapeutic advantages resulting from greater metabolic stability, for example, increased in vivo half-life or reduced dosage requirements, and hence may be preferred in some circumstances. Thus, in some embodiments, the compounds according to the present application are isotopically labeled compounds, wherein H is optionally replaced by D at each occurrence.

Substitution with positron emitting isotopes, such as $^{11}C$, $^{18}F$, $^{15}O$ and $^{13}N$, may be useful in Positron Emission Topography (PET) studies for examining substrate receptor occupancy.

The isotopically-labeled compounds can generally be prepared by conventional techniques known to those skilled in the art or using an appropriate isotopically-labeled reagents in place of the non-labeled reagent previously employed.

The compounds according to the present application may exist in the form of a pharmaceutically acceptable salt.

The term "pharmaceutically acceptable" means that the corresponding compound, vehicle or molecule is suitable for administration to humans. Preferably, the term refers to the use for mammalian, preferably human approved by any national regulatory agency such as CFDA (China), EMEA (Europe), FDA (USA), etc.

The pharmaceutically acceptable salt include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Moreover, the compounds according to the present application may exist in unsolvated form as well as solvated forms with pharmaceutically acceptable solvents such as water, ethanol and the like. The compounds may exist in one or more crystalline forms, or in an amorphous form. These forms are included within the scope of the invention.

The present application further comprises prodrug of the compounds according to the present application. The "prodrug" refers to a derivative that is converted into a compound according to the present disclosure by a reaction with enzymes, gastric acid, and the like in a living body under physiological conditions, for example, through oxidation, reduction, hydrolysis, and the like catalyzed by enzymes. Thus, some derivatives of the compounds according to the present application may have little or no pharmacological activity on their own, and may be converted into compounds according to the present application with the desired activity when administered to the body or on the body of a patient.

The present application further comprises metabolite of the compounds according to the present application. The "metabolite" refers to all molecules derived from any compound according to the present disclosure in a cell or organism, preferably a human.

As used herein, the term "substituted" means that one or more (preferably 1 to 5, more preferably 1 to 3, most preferably 1 or 2) hydrogen atoms in a group are independently replaced by a corresponding number of substituents.

As used herein, the term "independently" means that when the number of chemical or group substituents is more than one, these chemical or group substituents may be the same or different.

As used herein, the term "optional" or "optionally" means that the event described therein may or may not occur. For example, an "optionally substituted" group means that the group may be unsubstituted or substituted.

The term "halogen" or "halo" refers to -F, -Cl, -Br, or -I.

As used herein, the term "alkyl" refers to saturated aliphatic hydrocarbons, including straight and branched

chains. In some embodiments, the alkyl group has 1-8, or 1-6, or 1-4, or 1-3 carbon atoms. For example, the term "$C_{1-8}$ alkyl" refers to a straight or branched chain group of atoms having 1-8 carbon atoms. The term "$C_{1-8}$ alkyl" includes the terms "$C_{1-6}$ alkyl", "$C_{1-3}$ alkyl" and "$C_{1-4}$ alkyl" in its definition. Examples of alkyl groups include, but are not limited to, methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl, and the like. The alkyl group may be optionally substituted with one or more (for example, 1 to 5) suitable substituent(s).

As used herein, the term "alkenyl" refers to an aliphatic hydrocarbon having at least one carbon-carbon double bond, including straight and branched chains having at least one carbon-carbon double bond. In some embodiments, alkenyl groups have 2-8 carbon atoms, 2-6 carbon atoms, 3-6 carbon atoms, or 2-4 carbon atoms. For example, the term "$C_{2-8}$ alkenyl" refers to a linear or branched unsaturated atomic group (having at least one carbon-carbon double bond) having 2-8 carbon atoms. The double bond may or may not be the point of attachment of another group. Alkenyl groups include, but are not limited to, vinyl, 1-propenyl, 2-propenyl, 2-methyl-2-propenyl, butenyl, pentenyl, 3-hexenyl, and the like. Alkenyl groups may be optionally substituted with one or more (for example, 1 to 5) suitable substituent(s). When the compound of formula (I) contains an alkenyl group, the alkenyl group may be present in the pure E form, the pure Z form, or any mixture thereof.

As used herein, the term "alkynyl" refers to an aliphatic hydrocarbon having at least one carbon-carbon triple bond, including straight and branched chains having at least one carbon-carbon triple bond. In some embodiments, an alkynyl group has 2-8 carbon atoms, 2-6 carbon atoms, 3-6 carbon atoms, or 2-4 carbon atoms. For example, the term "$C_{2-8}$ alkynyl" refers to a linear or branched unsaturated atomic group (having at least one carbon-carbon triple bond) having 2-8 carbon atoms. The triple bond may or may not be the point of attachment of another group. Alkynyl groups include, but are not limited to, ethynyl, 1-propynyl, 2-propynyl, 2-methyl-2-propynyl, butynyl, pentynyl, 3-hexynyl, and the like. The alkynyl group may be optionally substituted with one or more (for example, 1 to 5) suitable substituent(s).

As used herein, the term "$C_{3-8}$ alicyclic group" refers to an alicyclic group having 3-8 ring-forming carbon atoms. The term "$C_{3-7}$ alicyclic group" refers to an alicyclic group having 3-7 ring-forming carbon atoms. The term "$C_{3-6}$ alicyclic group" refers to an alicyclic group having 3-6 ring-forming carbon atoms. The alicyclic group may be a monocyclic ring. The definition of alicyclic group also includes an unsaturated non-aromatic alicyclic group. Examples of alicyclic group are, for example, cyclopropyl, cyclobutyl, cyclopentyl, cyclohexyl, cyclohexenyl, cycloheptyl, cyclooctyl, cyclohexadienyl, cyclopentenyl, cycloheptenyl and cyclooctenyl. The alicyclic group may be optionally substituted with one or more suitable substituent(s).

As used herein, the term "$C_{6-12}$ bicyclic alicyclic group" is an alicyclic group containing two rings with 6-12 ring-forming carbon atoms. The bicyclic alicyclic group may be fused or may include a bridged bicyclic alicyclic group system.

As used herein, the term "$C_{8-15}$ membered tricyclic alicyclic group" is an alicyclic group containing three rings with 8-15 ring-forming carbon atoms. The tricyclic alicyclic group may be fused or bridged.

As used herein, the term "n-membered heteroalicyclic group" refers to an alicyclic group having m ring-forming carbon atoms and (n-m) ring-forming heteroatoms, the heteroatoms being selected from O, S and N. For example, the term "4-8-membered heteroalicyclic group" refers to a heteroalicyclic group substituent containing a total of 4 to 8 ring atoms, at least one of which is a heteroatom; the term "4-6-membered heteroalicyclic group" refers to a heteroalicyclic group substituent containing a total of 4 to 6 ring atoms, at least one of which is a heteroatom; and the term "3-10-membered heteroalicyclic group" refers to a heteroalicyclic group substituent containing a total of 3 to 10 ring atoms, at least one of which is a heteroatom. The term "n-membered bicyclic heteroalicyclic group" refers to a bicyclic heteroalicyclic group having m ring-forming carbon atoms and (n-m) ring-forming heteroatoms, the heteroatoms being selected from O, S and N. Examples of heteroalicyclic group includes, but not limited to, azetidinyl, thietidinyl, dihydrofuranyl, dihydrothiophenyl, tetrahydrothienyl, tetrahydrofuranyl, tetrahydrotriazinyl, tetrahydropyrazolyl, tetrahydrooxazinyl, tetrahydropyrimidinyl, octahydrobenzofuranyl, octahydrobenzimidazolyl, octahydrobenzothiazolyl, imidazolidinyl, pyrrolidinyl, piperidinyl, piperazinyl, oxazolidinyl, thiazolidinyl, pyrazolidinyl, thiomorpholinyl, tetrahydropyrimidinyl tetrahydrothiopyranyl, tetrahydrothiazinyl, tetrahydrothiadiazinyl, tetrahydrooxazolyl, morpholinyl, oxetanyl, tetrahydrodiazinyl, oxazinyl, oxathiadiazinyl, quinuclidinyl, benzodipyranyl (chromanyl), isobenzodipyranyl (isochromanyl), dihydrobenzodioxinyl, benzodioxolyl, benzoxazinyl, dihydroindolyl, dihydrobenzofuranyl, tetrahydroquinolinyl, isochromanyl, dihydro-1H-isoindolyl, 2-azabicyclo[2.2.1]heptanoyl, 3-azabicyclo[3.1.0]hexyl, 3-azabicyclo[4.1.0]heptyl, oxacycloheptyl, thiacycloheptyl, azacycloheptyl, and the like. The heteroalicyclic group may be optionally substituted with one or more suitable substituent(s).

In the present application, the term "aryl" refers to a 5 to 16 membered carbon ring aromatic group having at least one conjugated $\pi$-electron ring system. The aryl group may have conjugated or fused rings and may be unsubstituted or substituted as described. Examples of aryl include phenyl, naphthalenyl, anthracenyl, phenanthrenyl, azulenyl, and biphenyl. As used herein, the term "$C_{5-16}$ aryl" refers to an aryl group having an aromatic ring containing 5 to 16 carbon atoms. Similarly, the term "$C_{5-8}$ aryl" refers to an aryl group having an aromatic ring containing 5, 6, 7, or 8 carbon atoms, for example, phenyl.

In the present application, the term "heteroaryl" refers to a 5 to 16 membered aromatic group having at least one

conjugated $\pi$-electron ring system and containing 1 to 4 heteroatoms such as N, O, or S. Heteroaryl groups may have conjugated or fused rings and may be unsubstituted or substituted as described. Examples of heteroaryl include thienyl, furanyl, pyrrolyl, pyrazolyl, imidazoyl, oxazolyl, isoxazolyl, thiazolyl, isothiazolyl, triazolyl, oxadiazolyl, thiadiazolyl, tetrazolyl, pyridinyl, pyridizinyl, pyrimidinyl, pyrazinyl, triazinyl, indolyl, isoindolyl, indolizinyl, benzofuranyl, benzothienyl, indazolyl, benzimidazoyl, benzothiazolyl, purinyl, quinolizinyl, quinolinyl, isoquinolinyl, cinnolinyl, phthalazinyl, quinazolinyl, quinoxalinyl, naphthyridinyl, pteridinyl, carbazolyl, acridinyl, phenazinyl, phenothiazinyl, or phenoxazinyl.

As used herein, the term "n-membered heteroaryl" refers to a heteroaryl group having m aromatic ring-forming carbon atoms and (n-m) aromatic ring-forming heteroatoms, the heteroatoms being selected from O, S and N. For example, 5-7 membered heteroaryl includes but not limited to furanyl, thienyl, pyrrolyl, thiazolyl, pyrazolyl, imidazolyl, pyridinyl, pyranyl, pyridazinyl, pyrimidinyl, pyrazinyl. The heteroaryl may be optionally substituted with one or more suitable substituent(s).

As used herein, the term "$C_{7-11}$ bicycloaryl" refers to a bicycloaryl group having 7-11 carbon atoms, such as naphthalene, indene and the like. The bicycloaryl may be optionally substituted with one or more suitable substituent(s).

As used herein, the term "n-membered bicycloheteroaryl" refers to a bicycloheteroaryl group having m carbon atoms forming an aromatic bicyclic ring and (n-m) heteroatoms forming an aromatic bicyclic ring, and the heteroatoms are selected from O, S and N. For example, 7-11 membered bicycloheteroaryl includes, but not limited to, quinolinyl, isoquinolinyl, indolyl, purinyl, benzothiazolyl. The bicycloheteroaryl may be optionally substituted with one or more suitable substituent(s).

As used herein, the term "11-15 membered tricyclyl" includes but not limited to acridine and the like. The 11-15 membered tricyclyl may be optionally substituted with one or more suitable substituent(s).

As used herein, the term "haloalkyl" refers to an alkyl group having one or more halogen substituent(s) (up to perhaloalkyl, that is, each hydrogen atom of the alkyl is replaced by a halogen atom). For example, the term "$C_{1-6}$ haloalkyl" refers to a $C_{1-6}$ alkyl group with one or more halogen substituent(s) (up to perhaloalkyl, that is, each hydrogen atom of the alkyl group is replaced by a halogen atom). As another example, the term "$C_{1-4}$ haloalkyl" refers to a $C_{1-4}$ alkyl group with one or more halogen substituent(s) (up to perhaloalkyl, that is, each hydrogen atom of the alkyl group is replaced by a halogen atom); the term "$C_{1-3}$ haloalkyl" refers to a $C_{1-3}$ alkyl group with one or more halogen substituent(s) (up to perhaloalkyl, that is, each hydrogen atom of the alkyl group is replaced by a halogen atom); and the term "$C_{1-2}$ haloalkyl" refers to a $C_{1-2}$ alkyl group (i.e. methyl or ethyl) with one or more halogen substituent(s) (up to perhaloalkyl, that is, each hydrogen atom of the alkyl group is replaced by a halogen atom). As another example, the term "$C_1$ haloalkyl" refers to a methyl group with 1, 2, or 3 halogen substituent(s). Examples of haloalkyl groups include: $CF_3$, $C_2F_5$, $CHF_2$, $CH_2F$, $CH_2CF_3$, $CH_2Cl$, and the like.

As used herein, the term "alkoxy" refers to alkyl with a single bond attached to an oxygen atom. The point of attachment of the alkoxy group to a molecule is through the oxygen atom. Alkoxy may be described as alkyl-O-. The term "$C_{1-6}$ alkoxy" refers to a linear or branched alkoxy group containing 1 to 6 carbon atoms. The term "$C_{1-6}$ alkoxy" includes the term "$C_{1-3}$ alkoxy" in its definition. Alkoxy includes, but not limited to, methoxy, ethoxy, propoxy, isopropoxy, butoxy, hexoxy, and the like. The alkoxy group may be optionally substituted with one or more suitable substituent(s).

As used herein, the term "3-14-membered ring" refers to a saturated or unsaturated ring system with 3-14 ring-forming atoms.

Herein, a numerical range relating to the number of substituents, the number of carbon atoms, or the number of ring members represents an enumeration of all integers in the range, and the range is only a simplified representation thereof. For example: "4 to 6-membered ring" means a 4, 5 or 6-membered ring; "5 to 7-membered ring" means a 5, 6 or 7-membered ring; "7 to 11-membered ring" means a 7, 8, 9, 10 or 11-membered ring; "4 to 8-membered ring" means a 4, 5, 6, 7 or 8-membered ring; "3 to 10-membered ring" means a 3, 4, 5, 6, 7, 8, 9 or 10-membered ring; "3 to 14-membered ring" means a 3, 4, 5, 6, 7, 8, 9, 10, 11, 12, 13 or 14-membered ring; "$C_{1-3}$" means 1 ($C_1$), 2 ($C_2$), or 3 ($C_3$) carbon atoms; "$C_{1-4}$" means 1 ($C_1$), 2 ($C_2$), 3 ($C_3$) or 4 ($C_4$) carbon atoms; "$C_{3-6}$" means 3 ($C_3$), 4 ($C_4$), 5 ($C_5$), or 6 ($C_6$) carbon atoms; "$C_{3-8}$" means 3 ($C_3$), 4 ($C_4$), 5 (Cs), 6 ($C_6$), 7 ($C_7$), or 8 ($C_8$) carbon atoms; "$C_{5-7}$" means 5 (Cs), 6 ($C_6$), or 7 ($C_7$) carbon atoms; "$C_{7-11}$" means 7 ($C_7$), 8 ($C_8$), 9 ($C_9$), 10 ($C_{10}$), or 11 ($C_{11}$) carbon atoms. Thus, a numerical range associated with the number of substituents, the number of carbon atoms, or the number of ring members also encompasses any one of its subranges, and each subrange is also considered to be disclosed herein.

In formula (I) as described above, $R_1$ may be any substituent commonly used in organic chemistry, which is not particularly limited.

In some embodiments, $R_1$ is $C_{1-6}$ alkyl. For example, $R_1$ is selected from methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl.

In some embodiments, $R_1$ is $C_{3-7}$ alicyclic group. For example, $R_1$ is selected from cyclopropyl, cyclobutyl, cyclopentyl, cyclohexyl, cyclohexenyl, cycloheptyl, cyclohexadienyl, cyclopentenyl, cycloheptenyl.

In some embodiments, $R_1$ is 3-10 membered heteroalicyclic group. For example, $R_1$ is selected from azetidinyl, thietidinyl, dihydrofuranyl, dihydrothienyl, tetrahydrothienyl, tetrahydrofuranyl, tetrahydrotriazinyl, tetrahydropyrazolyl,

tetrahydrooxazinyl, tetrahydropyrimidinyl, octahydrobenzofuranyl, octahydrobenzimidazolyl, octahydrobenzothiazolyl, imidazolidinyl, pyrrolidinyl, piperidinyl, piperazinyl, oxazolidinyl, thiazolidinyl, pyrazolidinyl, thiomorpholinyl, tetrahydropyranyl, tetrahydrothiopyranyl, tetrahydrothiazinyl, tetrahydrothiadiazinyl, tetrahydrooxazolyl, morpholinyl, oxetanyl, tetrahydrodiazinyl, oxazinyl, oxathiadiazinyl, quinuclidinyl, benzodipyranyl, isobenzodipyranyl, dihydrobenzodioxinyl, benzodioxopentenyl, benzoxazinyl, dihydroindolyl, dihydrobenzofuranyl, tetrahydroquinolinyl, isochromanyl, dihydro-1H-isoindolyl, oxepanyl, thiepanyl, and azepanyl.

In some embodiments, $R_1$ is **H,** halogen, -OH, -$NO_2$, -CN, -$SF_5$, or -SH.

In some embodiments, $R_1$ is selected from -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl.

In some embodiments, $R_1$ is selected from $C_{5-8}$ aryl (e.g., phenyl), 5-10-membered heteroaryl (e.g., furanyl, thienyl, pyrrolyl, thiazolyl, pyrazolyl, imidazolyl, pyridinyl, pyranyl, pyridazinyl, pyrimidinyl, pyrazinyl, etc.), -$C_{1-4}$ alkyl-($C_{5-8}$ aryl), -$C_{1-4}$ alkyl- (5-10-membered heteroaryl).

In some embodiments, $R_1$ is selected from -N($R^{10}$)($R^{11}$), -N($R^{10}$)(C(=O)$R^{11}$), - N($R^{10}$)(C(=O)-O$R^{11}$), -N($R^{12}$)(C(=O)-N($R^{10}$)($R^{11}$)), -C(=O)-N($R^{10}$)($R^{11}$), -C(=O)-$R^{12}$, - C(=O)-O$R^{12}$, -OC(=O)$R^{12}$, -N($R^{10}$)(S(=O)$_2R^{11}$), -S(=O)$_2$-N($R^{10}$)($R^{11}$), -S$R^{12}$, and -O$R^{12}$, wherein $R^{10}$, $R_{11}$, and $R^{12}$ at each occurrence are each independently selected from: **H,** $C_{1-6}$ alkyl, $C_{1-6}$ haloalkyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, -$C_{1-4}$ alkyl-($C_{3-7}$ alicyclic group), -$C_{1-4}$ alkyl- (3-10-membered heteroalicyclic group), -$C_{1-4}$ alkyl-($C_{6-12}$ bicyclic alicyclic group), -$C_{1-4}$ alkyl- (6-12-membered bicyclic heteroalicyclic group), -$C_{1-4}$ alkyl-($C_{8-15}$ tricyclic alicyclic group), -$C_{1-4}$ alkyl-(8-15-membered tricyclic heteroalicyclic group), -$C_{1-4}$ alkyl-($C_{5-8}$ aryl), and -$C_{1-4}$ alkyl-(5-10-membered heteroaryl), wherein each substitute within the group is optionally substituted with 0, 1, 2, 3, or 4 substituents each independently selected from the following groups: halogen, -OH, -$NH_2$, -NH($CH_3$), -N($CH_3$)$_2$, -CN, -$NO_2$, -$SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy, and $C_{1-4}$ haloalkoxy; or $R^{10}$, $R_{11}$, and the atom(s) attached thereto together form a 3-14-membered ring.

In some embodiments, $R_1$ is selected from -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{5-8}$ aryl, 5-10 membered heteroaryl, $C_{7-11}$ bicyclic aryl, 7-11 membered bicyclic heteroaryl, wherein the -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{5-8}$ aryl, 5-10 membered heteroaryl, $C_{7-11}$ bicycloaryl, and 7-11 membered bicyclic heteroaryl are unsustituted or each optionally substituted with 0, 1, 2, 3 or 4 $R^{1a}$; and $R^{1a}$ is independently selected from halogen, -OH, -$NH_2$, -NH($CH_3$), -N($CH_3$)$_2$, -CN, -$NO_2$, -$SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10 membered heteroalicyclic group, $C_{5-8}$ aryl, 5-7 membered heteroaryl, $C_{7-11}$ bicyclic aryl, 7-11 membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy, and $C_{1-4}$ haloalkoxy.

Any exemplary groups enumerated above for $R_1$ are to be understood as being optionally substituted; i.e., where appropriate, any of the groups given above for $R_1$ may be optionally substituted with 0, 1, 2, 3 or 4 $R^{1a}$(s), and $R^{1a}$ is independently selected from halogen, -OH, -$NO_2$, -CN, -$SF_5$, -SH, -S-$C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10-membered heteroalicyclic group, $C_{5-8}$ aryl, 5-7-membered heteroaryl, -N($R^{13}$)($R^{14}$), -N($R^{13}$)(C(=O)$R^{14}$), -N($R^{13}$)(C(=O)-O$R^{14}$), -N($R^{15}$)(C(=O)-N($R^{13}$)($R^{14}$)), -C(=O)-N($R^{13}$)($R^{14}$), -C(=O)-$R^{15}$, - C(=O)-O$R^{15}$, -OC(=O)$R^{15}$, -N($R^{13}$)(S(=O)$_2R^{14}$), -S(=O)$_2$-N($R^{13}$)($R^{14}$), -S$R^{15}$, and -O$R^{15}$, where $R_{13}$, $R^{14}$, and $R^{15}$ at each occurrence are each independently selected from: H, $C_{1-6}$ alkyl, $C_{1-6}$ haloalkyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, -$C_{1-4}$ alkyl- ($C_{3-7}$ alicyclic group), -$C_{1-4}$ alkyl- (3-10-membered heteroalicylic group), -$C_{1-4}$ alkyl-($C_{6-12}$ bicyclic alicyclic group), -$C_{1-4}$ alkyl- (6-12-membered bicyclic heteroalicyclic group), -$C_{1-4}$ alkyl-($C_{8-15}$ tricyclic alicylic group), -$C_{1-4}$ alkyl- (8-15-membered tricyclic heteroalicyclic group), -$C_{1-4}$ alkyl-($C_{5-8}$ aryl), and -$C_{1-4}$ alkyl- (5-10-membered heteroaryl), wherein each substituent within the group is optionally substituted with 0, 1, 2, 3 or 4 substituents each independently selected from the group consisting of: halogen, -OH, -$NH_2$, -NH($CH_3$), - N($CH_3$)$_2$, -CN, -$NO_2$, -$SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10-membered alicyclic group, $C_{5-8}$ aryl, 5-7-membered heteroaryl, $C_{7-11}$ bicyclic aryl, 7-11-membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy, and $C_{1-4}$ haloalkoxy; or $R_{13}$, $R^{14}$, and the atom(s) attached thereto together form a 3-14-membered ring.

In some preferred embodiments, $R_1$ is selected from methyl, ethyl, propyl, isopropyl, and cyclobutyl groups. The methyl, ethyl, propyl, isopropyl or cyclobutyl group may optionally be substituted with 0, 1 or 2 substituents each independently selected from a group consisting of: methyl, ethyl, cyclopentyl, cyclopropyl, -CN, -OH, morpholinyl, piperidinyl, and these substituents may further optionally be substituted with 0, 1 or 2 substituents each independently

selected from a group consisting of: methyl, ethyl, cyclopentyl, cyclopropyl, - CN, and -OH. For example, in some preferred embodiments, $R_1$ is selected from (1-hydroxycyclopropyl)ethyl, 3-hydroxycyclobutyl, 2-cyanoethyl, 2-hydroxyethyl, 2-cyano-1-cyclopentyl ethyl, 1-cyanopropane, 2-morpholinoethyl, ethyl, and (1-methylpiperidin-4-yl)methyl.

In some preferred embodiments, $R_1$ is selected from halogen, such as F.

In some preferred embodiments, $R_1$ is selected from piperazinyl, morpholinyl, pyrrolidinyl, piperidinyl and azetidinyl, the piperazinyl, pyrrolidinyl, piperidinyl and azetidinyl being optionally substituted with 0, 1 or 2 substituents each independently selected from a group consisting of: methyl, ethyl, cyclopentyl, cyclopropyl, -CN, and -OH. In some preferred embodiments, $R_1$ is selected from methyl, ethyl, propyl, isopropyl, and cyclobutyl groups, in which said methyl, ethyl, propyl, isopropyl or cyclobutyl groups may optionally be substituted with 0, 1 or 2 substituents each independently selected from a group consisting of: methyl, ethyl, cyclopentyl, cyclopropyl, -CN, -OH, morpholinyl, and piperidinyl, and these substituents are further optionally substituted with 0, 1 or 2 substituents each independently selected from a group consisting of: methyl, ethyl, cyclopentyl, cyclopropyl, -CN, and -OH.

In some preferred embodiments, $R_1$ is selected from piperazinyl, morpholinyl, pyrrolidinyl, piperidinyl, and azetidinyl. For example, in some preferred embodiments, $R^5$ is selected from 3,5-dimethylpiperazinyl, morpholinyl, 3-hydroxypyrrolidinyl, 4-methylpiperazinyl, 4-ethylpiperazinyl, 4-hydroxypiperidinyl, 1-methylpiperidinyl, 1-ethylpiperidin-4-yl, 1-methylazetidin-3-yl.

In some preferred embodiments, $R_1$ is selected from H, methyl, ethyl, n-propyl, isopropyl, butyl, methoxy, ethoxy, hydroxy, cyclopropyl, cyclobutyl, cyclopentyl, cyclohexyl, morpholinomethyl, hydroxycyclobutyl, hydroxycyclohexyl, cyanoethoxy, cyanomethyl, pyridin-3-yl, 1-methyl-1H-pyrazol-4-yl, 1-methyl-1H-pyrazol-3-yl, 4-methylpiperazin-1-yl, 1-methylpiperidin-4-yl, morpholinyl, pyrrolidin-3-yl, 3-hydroxypyrrolidin-1-yl, and 3-cyanopyrrolidin-1-yl.

In some embodiments, $R_1$ is selected from H, halogen, -OH, $-NO_2$, -CN, $-SF_5$, -SH, - S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{5-8}$ aryl), -$C_{1-4}$ alkyl-(5-10 membered heteroaryl), and -N($R_{10}$)($R_{11}$), wherein the -S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{5-8}$ aryl), and -$C_{1-4}$ alkyl-(5-10 membered heteroaryl) are each optionally substituted with 0, 1, 2, 3 or 4 $R^{1a}$; and $R^{1a}$ is independently selected from halogen, -OH, $-NO_2$, -CN, oxo, $C_{1-4}$ alkyl, $C_{1-4}$ haloalkyl, $C_{1-4}$ alkoxy, $C_{1-4}$ haloalkoxy, $C_{3-7}$ alicyclic group, 3-10 membered heteroalicyclic group, $C_{5-8}$ aryl, 5-7 membered heteroaryl; and $R_{10}$, $R_{11}$, and the atom(s) attached thereto together form a 3-8-membered ring. $R_1$ is selected from H, halogen, -OH, $-NO_2$, -CN, $-SF_5$, -SH, -S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{5-8}$ aryl), -$C_{1-4}$ alkyl-(5-10 membered heteroaryl), and -N($R_{10}$)($R_{11}$), wherein the -S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{5-8}$ aryl), and -$C_{1-4}$ alkyl-(5-10 membered heteroaryl) are each optionally substituted with 0, 1, 2, 3 or 4 $R^{1a}$; and $R^{1a}$ is independently selected from halogen, -OH, $-NO_2$, -CN, oxo, $C_{1-4}$ alkyl, $C_{1-4}$ haloalkyl, $C_{1-4}$ alkoxy, $C_{1-4}$ haloalkoxy, $C_{3-7}$ alicyclic group, 3-10 membered heteroalicyclic group, $C_{5-8}$ aryl, 5-7 membered heteroaryl; and $R_{10}$, $R_{11}$ and $R_{12}$, at each occurrence, are each independently selected from H, $C_{1-6}$ alkyl, $C_{1-6}$ haloalkyl, $C_{3-7}$ alicyclic group, 3-10 membered heteroalicyclic group, wherein each option listed in the group is optionally substituted with 0, 1, 2, 3, or 4 substituents each independently selected from a group consisting of: halogen, -OH, $-NH_2$, oxo, $C_{1-4}$ alkyl, $C_{1-4}$ hydroxyalkyl, $C_{1-4}$ haloalkyl, $C_{1-4}$ alkoxy and $C_{1-4}$ haloalkoxy; or $R_{10}$, $R_{11}$, and the atom(s) attached thereto together form a 3-8-membered ring.

In some preferred embodiments, $R_1$ is selected from H, halogen, -OH, methyl, ethyl, propyl, butyl, pentyl, hexyl, methoxy, ethoxy, propoxy, butoxy, pentoxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl, wherein the methyl, ethyl, propyl, butyl, pentyl, hexyl, methoxy, ethoxy, propoxy, butoxy, pentoxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl are each optionally substituted with 1 or 2 $R^{1a}$(s), and $R^{1a}$ is independently selected from halogen, -OH, $-NO_2$, -CN, oxo, $C_{1-4}$ alkyl, $C_{1-4}$ haloalkyl, and $C_{1-4}$ alkoxy.

In some preferred embodiments, $R_1$ is selected from H, methyl, methylpiperazinyl, pyrrolidinyl, methylpiperazinyl, hydroxycyclobutyl, methylpyrazolyl, hydroxyethyl, hydroxypropyl, methylpiperidinyl, morpholinyl, and hydroxypyrrolidinyl.

It should be understood that any of the above embodiments of $R_1$ may be combined with any of the embodiments of $R_2$, $R_3$, L, n, and ring A as described above and below in any manner.

In embodiments, $R_2$ is H.

In embodiments, $R_3$ is methyl.

It should be understood that any of the aforementioned embodiments of $R_3$ may be combined with any of the embodiments of $R_1$, $R_2$, L, n, and ring A as described above and below in any manner.

In other embodiments, $R_3$ and $R_2$ may be different.

In the formula (I) as described above, L may be (C=O), (O=S=O), ((C=O)-$CH_2$), $CH_2$, or a bond.

In some embodiments, L is (C=O).

In some embodiments, L is (O=S=O).

In some embodiments, L is ((C=O)-$CH_2$).

In some embodiments, L is CH2.

In some embodiments, L is a bond (i.e., $R_1$ and the N atom are directly connected by a covalent bond).

It should be understood that any of the aforementioned embodiments of L may be combined with any of the embodiments of $R_1$, $R_2$, $R_3$, ring A, and n as described above and below in any manner.

In the formula (I) as described above, n is 1.

It should be understood that any of the aforementioned embodiments of n may be combined with any of the embodiments of $R_1$, $R_2$, $R_3$, L, and ring A as described above and below in any manner.

In the formula (I) as described above, the ring A is a nitrogen-containing aromatic ring and satisfies: 1) G1=$NR_4$, G2=$CR_5$; or 2) G1=$CR_4$, G2=$NR_5$; or 3) G1=$CR_4$, G2=O or S; where $R_4$ and $R_5$ are each independently selected from H, halogen, -CN, -OH, -$NO_2$, $C_{1-6}$ alkyl, $C_{1-6}$ haloalkyl, $C_{1-6}$ alkoxy ($R_4$ and $R_5$ may be the same or different); or $R_4$ and $R_5$ together with the atoms to which they are attached form a 3-8 membered ring.

Therefore, the compounds of formula (I) in the present application may have one of the following structures:

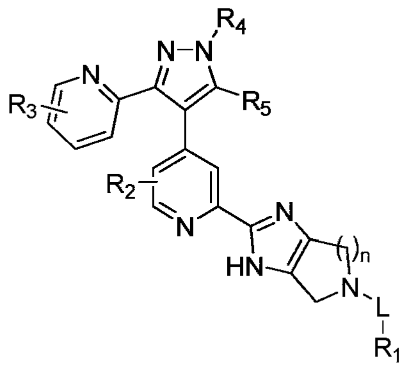 (I-1)

in which, n, L, $R_1$, $R_2$, $R_3$, $R_4$, and $R_5$ are as defined above;

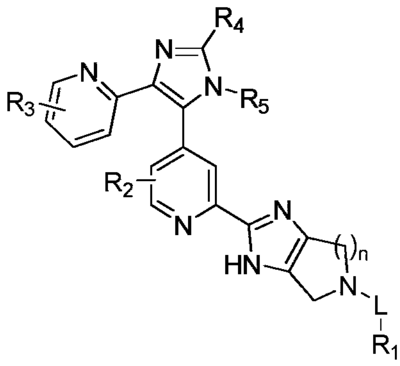 (I-2)

in which, n, L, $R_1$, $R_2$, $R_3$, $R_4$, and $R_5$ are as defined above;

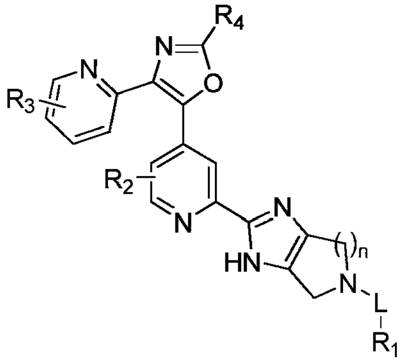

(I-3)

in which, n, L, $R_1$, $R_2$, $R_3$, and $R_4$ are as defined above;

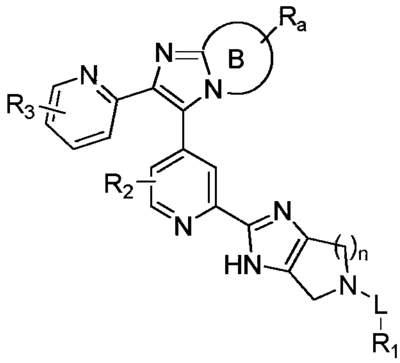

(I-4)

in which, n, L, $R_1$, $R_2$, and $R_3$ are as defined above; ring B is a 3 to 8 membered ring containing 1 N atom, and $R_a$ is selected from H, halogen, -CN, -OH, $-NO_2$, $C_{1-6}$ alkyl, $C_{1-6}$ haloalkyl, and $C_{1-6}$ alkoxy.

The embodiments and preference given above for $R_1$, $R_2$, $R_3$, ring A, L, and n in formula (I) also apply to formulas (I-1), (I-2), (I-3), and (I-4).

In some specific embodiments, the compounds of formula (I) of the present application are selected from the compounds shown in the respective examples.

**II. Preparation of compounds**

The compounds of formula (I) in the present application may be synthesized by those skilled in the art using conventional organic synthesis methods based on the specific structure of the compounds.

In addition, a person skilled in the art can refer to the synthetic routes of specific compounds shown in Examples of the present application and make appropriate adjustments to the raw materials and reaction conditions to obtain synthesis methods of other compounds.

III. Use of the **compounds and compositions containing the compounds**

Experimental results indicate that the compounds of formula (I) in the present application may inhibit the activity of TGF-β, and can therefore be used as a TGF-β inhibitor. Specifically, the compounds of formula (I) in the present application are particularly useful for inhibiting the TGF-β type I receptor (TGFβR1), and may be used as a TGF-β1 inhibitor.

In a second aspect, the present application provides a pharmaceutical composition comprising a compound according to the present application, or an isotopically labeled compound thereof, or an optical isomer thereof, a geometric isomer thereof, a tautomer thereof or a mixture of various isomers, or a pharmaceutically acceptable salt thereof, as described above, and one or more pharmaceutically acceptable carriers, adjuvants, or excipients.

The pharmaceutical compositions according to the present application may be prepared in a manner well known in the pharmaceutical field and may be administered by a variety of routes, depending on whether topical or systemic treatment is desired and depending on the site to be treated. Administration may be topical (including ophthalmic and to mucous membranes, including intranasal, vaginal, and rectal delivery), pulmonary (e.g., by inhalation or by blowing in a

powder or aerosol, including through aerosol dispensers; intratracheal, intranasal, epidermal, and transdermal), ocular, transoral, or parenteral. Methods for ocular delivery may include topical administration (eye drops), subconjunctival, periocular or intravitreal injection or introduction via a balloon catheter or ophthalmic insert surgically placed in the conjunctival sac. Parenteral administration includes intravenous, intra-arterial, subcutaneous, intraperitoneal, or intramuscular injection or infusion; or intracranial (e.g., intrathecal or intracerebroventricular) administration. Parenteral administration may be in the form of a single push dose, or may be, for example, via a continuous perfusion pump. Pharmaceutical compositions and formulations for topical administration may include transdermal patches, ointments, lotions, creams, gels, drops, suppositories, sprays, liquids, and powders.

If a solid carrier is used, the dosage may be tableted, or placed in a hard gelatin capsule in powder or pellet form, or in the form of a troche or lozenge. The solid carrier may contain conventional excipients such as binding agents, fillers, tableting lubricants, disintegrants, wetting agents and the like. The tablet may, if desired, be film coated by conventional techniques. If a liquid carrier is employed, the dosage may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The medicament are prepared by conventional techniques appropriate to the desired preparation containing appropriate amounts of the active ingredient, that is, the compound according to the invention.

Medicament dosage suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

The medicament may also contain excipients such as preserving, wetting, emulsifying, and dispensing agents. Prevention of the action of microorganisms may be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form may be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules may be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which may be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylatedisostearyl alcohols, polyoxyethylene sorbitol and sorbitan esters, microcrystalline cellulose, meta-aluminum hydroxide, bentonite, agar-agar and tragacanth, or mixtures of these substances, and the like.

Dosage forms for topical administration of a compound of the invention include paste, powders, sprays, and inhalants. The active component is admixed under sterile conditions with a physiologically acceptable carrier and any preservatives, buffers, or propellants as may be required.

The amount of the compound according to the present application in the pharmaceutical composition and dosage form may be appropriately determined by those skilled in the art as needed. For example, the compound according to the present application may be present in the pharmaceutical composition or dosage form in a therapeutically effective amount.

In a third aspect, the present application provides use of the compounds of formula (I) according to the present application, or an isotopically labeled compound thereof, or an optical isomer thereof, a geometric isomer thereof, a tautomer thereof or a mixture of various isomers, or a pharmaceutically acceptable salt thereof, as described above, or the pharmaceutical composition as described above, in the preparation of drugs for the treatment of diseases or conditions associated with TGF-β, especially TGF-β1.

In a fourth aspect, the present application further provides a method of treating diseases or conditions associated with TGF-β, especially TGF-β1, said method comprising administering to a patient in need thereof a therapeutically effective amount of a compound of formula (I) according to the present application, or an isotopically labeled compound thereof, or an optical isomer thereof, a geometric isomer thereof, a tautomer thereof or a mixture of various isomers, or a pharmaceutically acceptable salt thereof, as described above, or the pharmaceutical composition as described above. Said patient is preferably a mammal, more preferably a human patient. The route of administration may be oral, topical (including, but not limited to, topical application, spraying, and the like), parenteral (including subcutaneous, intramuscular, cortical and intravenous) administration, bronchial administration, or nasal administration, etc. The administered amount is a therapeutically effective amount, which can be determined by a skilled person in the art based on actual needs.

In the present application, "diseases or conditions related to TGF-β (especially TGF-β1)" may include cancer, viral infections, chronic glomerulonephritis, acute glomerulonephritis, diabetic nephropathy, osteoporosis, arthritis, wound healing, scarring, ulcers, corneal trauma, heart valve stenosis, congestive cardiac necrosis, neurological damage, Alzheimer's disease, peritoneal or subcutaneous adhesions, atherosclerosis, skin fibrosis and skin aging due to fat loss, skeletal or chondrocyte disorders, hypophosphatemia disorders, and organ fibrosis diseases, particularly hepatocellular carcinoma, breast cancer, bladder cancer, colorectal cancer, melanoma, mesothelioma, lung cancer, prostate cancer, pancreatic cancer, testicular cancer, thyroid cancer, squamous cell carcinoma, glioblastoma, neuroblastoma, cervical cancer, rhabdomyosarcoma, renal fibrosis, liver fibrosis, pulmonary fibrosis, skin scarring, skin fibrosis and skin aging due to fat loss, etc.

In some embodiments, the disease or condition related to TGF-β is cancer. The compound of the present application may be used, for example, to inhibit the proliferation and metastasis of cancer cells, among others.

Exemplary cancers include bladder cancer, breast cancer, cervical cancer, colorectal cancer, small bowel cancer, colon cancer, rectal cancer, anal cancer, endometrial cancer, head and neck cancer (e.g., cancers of larynx, laryngopharynx, nasopharynx, oropharynx, lips, and mouth), kidney cancer, liver cancer (e.g., hepatocellular carcinoma, bile duct cell carcinoma), lung cancer (e.g., adenocarcinoma, small cell lung cancer, and non-small cell lung cancer, small cell cancer and non-small cell cancer, bronchial cancer, bronchial adenoma, pleural pneumoblastoma), ovarian cancer, prostate cancer, testicular cancer, uterine cancer, esophageal cancer, gallbladder cancer, pancreatic cancer (e.g., exocrine pancreatic cancer), thyroid cancer, parathyroid cancer, skin cancer (e.g., squamous cell carcinoma, Kaposi's sarcoma, Merkel cell skin cancer), and brain cancer (e.g., stellate cell tumor, neural tube embryonal cell tumor, ventricular meningioma, neuroectodermal tumor, pineal gland tumor).

Additional exemplary cancers include hematopoietic malignancies such as leukemia or lymphoma, multiple myeloma, chronic lymphocytic lymphoma, adult T-cell leukemia, B-cell lymphoma, cutaneous T-cell lymphoma, acute myelogenous leukemia, Hodgkin or non-Hodgkin lymphoma, myeloproliferative neoplasms (e.g., true erythroblastosis, primary thrombocythemia, and primary myelofibrosis ), Waldenstrom's macroglobulinemia, hairy cell lymphoma, chronic myelogenous lymphoma, acute lymphoblastic lymphoma, AIDS-related lymphoma, and Burkitt's lymphoma.

Additional exemplary cancers include eye tumors, glioblastoma, melanoma, rhabdomyosarcoma, lymphosarcoma, and osteosarcoma.

In some preferred embodiments, said diseases or conditions associated with TGF-β are selected from hepatocellular carcinoma, breast cancer, bladder cancer, colorectal cancer, melanoma, mesothelioma, lung cancer, prostate cancer, membrane adenocarcinoma, testicular cancer, thyroid cancer, squamous cell carcinoma, glioblastoma, neuroblastoma, uterine cancer, and rhabdomyosarcoma.

In other embodiments, the diseases or conditions related to TGF-β are selected from skeletal disorders and chondrocyte disorders, which include but are not limited to achondroplasia, chondrodysplasia, dwarfism, lethal chondrodysplasia (TD) (clinical forms TD I and TD II), Apert syndrome, Crouzon syndrome, Jackson-Weiss syndrome, Beare-Stevenson cutis gyrata syndrome, Pfeiffer syndrome, and craniosynostosis syndromes.

In other embodiments, the diseases or conditions related to TGF-β are hypophosphatemia disorders, which include, for example, X-linked hypophosphatemic rickets, autosomal recessive hypophosphatemic rickets, autosomal dominant hypophosphatemic rickets, and tumor-induced osteomalacia.

In other embodiments, the diseases or conditions related to TGF-β are selected from fibrosis diseases. Exemplary fibrosis diseases include liver cirrhosis, glomerulonephritis, pulmonary fibrosis, systemic sclerosis, rheumatoid arthritis, and wound healing.

In some preferred embodiments, the diseases or conditions related to TGF-β are skin diseases, particularly skin scarring, skin fibrosis, and skin aging due to fat loss.

The present application is further described and illustrated below in connection with specific examples.

## Examples

The following examples set forth herein are for illustrative purposes only, to exemplify aspects of the invention and the manner in which they are to be carried out, and are not intended to limit in any way the scope of protection as claimed.

Unless otherwise stated, all raw materials and reagents were obtained from commercial sources. The instruments and equipment used in the synthesis experiments and product analysis are all conventional instruments and equipment normally used in organic synthesis. All unspecified reaction conditions and test conditions are conventional reaction conditions and test conditions commonly used in the field.

**Example** 1: **1-(2-(4-(2-(6-Methylpyridin-2-yl)-6,7-dihydro-5H-pyrrolo[1,2-a]imidazol-3-yl)pyridin-2-yl)-4,6-dihydro-pyrrolo[3,4-d]imidazol-5(1H)-yl)ethan-1-one**

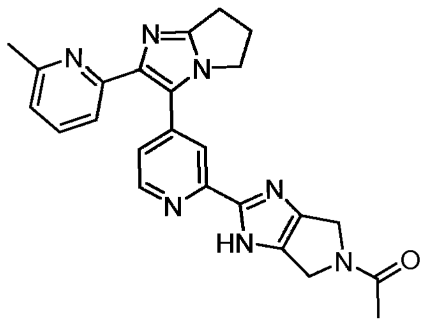

1

**Synthetic route for Compound 1:**

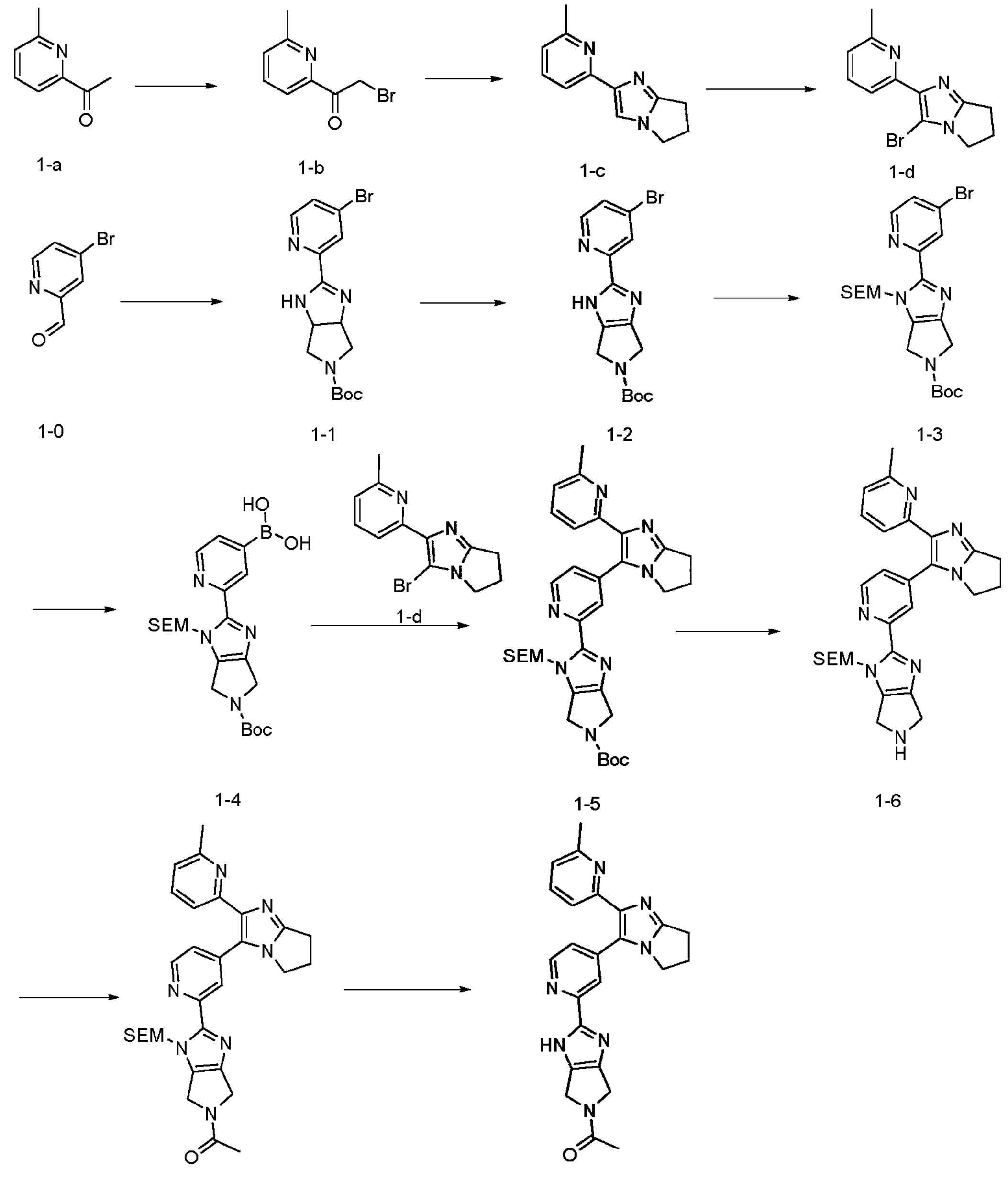


1-7 1

**Synthesis method:**

**Synthesis of Intermediate 1-b: 2-Bromo-1-(6-methylpyridin-2-yl)ethan-1-one**

The starting material 1-a (2.0 g, 14.8 mmol) was dissolved in 40 ml of acetonitrile, to which p-toluenesulfonic acid (3.8 g, 22.2 mmol) was added, and the resulting mixture was heated to 80°C. NBS (2.6 g, 14.8 mmol) was added in portions over 2 hours, and the reaction was continued for an additional 3 hours at 80°C. The reaction was monitored by TLC, and after the consumption of the starting material was complete, after which saturated sodium bicarbonate solution was added to the reaction mixture to quench the reaction. The product was extracted with ethyl acetate (EA) twice, and the combined organic phases were washed with saturated brine, dried over anhydrous sodium sulfate, and purified by silica gel column chromatography to yield Intermediate 1-b, 1.8 g, with a yield of 57.2%. LC-MS m/z (ESI) $[M+H]^+$ calculated for $C_8H_9BrNO$: 214.1; measured: 214.1.

**Synthesis of Intermediate 1-c: 2-(6-Methylpyridin-2-yl)-6,7-dihydro-5H-pyrrolo[1,2-a]imidazole**

Intermediate 1-b (800.0 mg, 3.7 mmol) was dissolved in 40 ml of toluene, to which sodium bicarbonate (942.4 mg,

11.2 mmol) and 2-aminopyrrolidine hydrochloride (896.4 mg, 7.5 mmol) were added, and the resulting mixture was heated to 80°C overnight. The reaction was monitored by TLC, and after the consumption of the starting material was complete, water was added to quench the reaction. The product was extracted with DCM twice, and the combined organic phases were washed with saturated brine, dried over anhydrous sodium sulfate, and purified by silica gel column chromatography to yield 313.8 mg of Intermediate 1-c with a yield of 41.9% yield. LC-MS m/z (ESI) $[M+H]^+$ calculated for C12H14N3: 200.1; measured: 200.2.

**Synthesis of Intermediate 1-d: 3-Bromo-2-(6-methylpyridin-2-yl)-6,7-dihydro-5H-pyrrolo[1,2-a]imidazole**

Intermediate 1-c (313.8 mg, 1.6 mmol) was dissolved in 20 ml of DCM, to which NBS (279.5 mg, 1.6 mmol) was added in portions at room temperature for 5 minutes. The reaction was monitored by TLC, and after the consumption of the starting material was complete, water was added to quench the reaction, and the product was extracted with DCM twice. The combined organic phases were washed with saturated brine, dried over anhydrous sodium sulfate, and purified by silica gel column chromatography to yield 289.7 mg of Intermediate 1-d with a yield of 66.4%. LC-MS m/z (ESI) $[M+H]^+$ calculated for $C_{12}H_{13}N_3Br$: 278.0; measured: 278.0.

**Synthesis of Intermediate 1-1: tert-butyl 2-(4-Bromopyridin-2-yl)-3a,4,6,6a-tetrahydro-pyrrolo[3,4-d]imidazol-5(1H)-carboxylate**

2-Formyl-4-bromopyridine (0.8 g, 4.3 mmol) was dissolved in 30 ml of tert-butanol, to which tert-butyl 3,4-diaminopyrrolidine-1-carboxylate (1.1 g, 5.6 mmol), iodine (1.6 g, 6.5 mmol), and potassium carbonate (1.2 g, 8.6 mmol) were added, and the resulting mixture was then heated to 70°C for 3 hours. After the reaction was complete, it was cooled to room temperature, and the reaction was quenched with a 5% sodium thiosulfate aqueous solution. Extraction was performed with EA, the organic phase was washed with saturated brine, dried over anhydrous sodium sulfate, concentrated, and purified with a silica gel column chromatography to yield Intermediate 1-1, 1.4 g, with a yield of 88.9%. LC-MS m/z (ESI) $[M+H]^+$ calculated for $C_{15}H_{20}BrN_4O_2$: 367.1; measured: 367.1.

**Synthesis of Intermediate 1-2: tert-butyl 2-(4-bromopyridin-2-yl)-4,6-dihydropyrrolo[3,4-d]imidazole-5(1H)-carboxylate**

Intermediate 1-1 (1.4 g, 3.8 mmol) was dissolved in 20 ml of DMSO, to which IBX (2.1 g, 7.6 mmol) was added. The resulting mixture was heated to 50°C overnight. After the reaction was complete, it was cooled to room temperature and quenched with saturated sodium bicarbonate solution. Extraction was carried out with EA, and the organic phase was washed with saturated brine, dried over anhydrous sodium sulfate, concentrated, and purified with a silica gel column chromatography to yield Intermediate 1-2, 1.2 g, with a yield of 84.7%. LC-MS m/z (ESI) $[M+H]^+$ calculated for $C_{15}H_{18}BrN_4O_2$: 365.1; measured: 365.1.

**Synthesis of Intermediate 1-3: tert-butyl 2-(4-bromopyridin-2-yl)-1-((2-(trimethylsilyl)ethoxy)methyl)-4,6-dihydropyrrolo[3,4-d]imidazole-5(1H)-carboxylate**

Intermediate 1-2 (1.2 g, 3.2 mmol) was dissolved in 20 ml of THF and cooled to 0°C, to which NaH (60%) (0.2 g, 4.9 mmol) was added, and the resulting mixture was stirred for 0.5 hours. And then SEMCl (0.8 g, 4.9 mmol) was added. After the addition was complete, the mixture was warmed to room termperature with stirring until the reaction was complete. The reaction was quenched with water, and extracted with EA, and the organic phase was washed with saturated brine, dried over anhydrous sodium sulfate, concentrated, and purified with a silica gel column chromatography to yield Intermediate 1-3, 1.2 g, with a yield of 75.2%. LC-MS m/z (ESI) $[M+H]^+$ calculated for $C_{21}H_{32}BrN_4O_3Si$ : 495.1; measured: 495.1.

**Synthesis of Intermediate 1-4: (2-(5-(tert-butoxycarbonyl)-1-((2-(trimethylsilyl)ethoxy)methyl)-1,4,5,6-tetrahydropyrrolo[3,4-d]imidazol-2-yl)pyridin-4-yl)boronic acid.**

Intermediate 1-3 (200.0 mg, 0.4 mmol) was dissolved in 10 ml of dioxane, to which bis(pinacolato)diboron (207.5 mg, 0.8 mmol) and $Pd(dppf)Cl_2$ (29.7 mg, 0.04 mmol) were added. The system was purged with nitrogen gas, potassium acetate (120.5 mg, 1.2 mmol) was added, and the nitrogen gas was replaced again. The reaction mixture was then heated to 80°C and it was allowed to react overnight. The reaction mixture was cooled to room temperature to obtain the crude product of Intermediate 1-4, which was used directly in the subsequent step.

**Synthesis of Intermediate 1-5: tert-butyl 2-(4-(2-(6-methylpyridin-2-yl)-6,7-dihydro-5H-pyrrolo[1,2-a]imidazol-3-yl)pyridin-2-yl)-1-((2-trimethylsilyl)ethoxy) methyl)-4,6-dihydropyrrolo[3,4-d]imidazole-5(1H)-carboxylate.**

To the reaction veseel containing Intermediate 1-4, 2 ml of water and potassium carbonate (111.6 mg, 0.8 mmol) were added, followed by the addition of Intermediate 1-d (114.1 mg, 0.8 mmol) dissolved in 10 ml of dioxane and Pd(dppf)$Cl_2$ (29.7 mg, 0.04 mmol). The system was purged with nitrogen gas, and the reaction mixture was heated to 100°C and it was allowed to react overnight. The reaction mixture was then cooled to room temperature, concentrated, and purified by silica gel column chromatography to obtain Intermediate 1-5, 86.5 mg, with a yield of 35.2%. LC-MS m/z (ESI) $[M+H]^+$ calculated for $C_{33}H_{44}N_7O_3Si$: 614.3; measured: 614.3.

**Synthesis of Intermediate 1-6: 2-(4-(2-(6-methylpyridin-2-yl)-6,7-dihydro-5H-pyrrolo[1,2-a]imidazol-3-yl)pyridin-2-yl)-1-((2-(trimethylsilyl)ethoxy)methyl)-1,4,5,6-tetrahydropyrrolo[3,4-d]imidazole.**

Intermediate 1-5 (60.0 mg, 0.1 mmol) was dissolved in 10 ml of DCM, to which zinc bromide (44.1 mg, 0.2 mmol) was added. The system was purged with nitrogen gas, and it was allowed to react at room temperature overnight. The solvent was then removed by concentration, and the residue was dissolved in 20 ml of THF. The solution was washed once each with saturated sodium bicarbonate solution and saturated sodium chloride solution, concentrated, to obtain the crude product of Intermediate 1-6, 46.4 mg, with a yield of 92.8%. LC-MS m/z (ESI) $[M+H]^+$ calculated for $C_{28}H_{36}N_7OSi$: 514.3; measured: 514.3.

**Synthesis of Intermediate 1-7: 1-(2-(4-(2-(6-methylpyridin-2-yl)-6,7-dihydro-5H-pyrrolo[1,2-a]imidazol-3-yl)pyridin-2-yl)-1-(2-(trimethylsilyl)ethoxy) methyl)-4,6-dihydropyrrolo[3,4-d]imidazol-5(1H)-yl)ethan-1-one**

Intermediate 1-6 (46.4 mg, 0.1 mmol) was dissolved in 10 ml of DCM, to which triethylamine (18.4 mg, 0.2 mmol) was added at room temperature, followed by the addition of acetyl chloride (14.2 mg, 0.2 mmol), and the resulting mixture was stirred at room temperature for 0.5 hours. After the reaction was complete, it was quenched with water, extracted with DCM, and the organic phase was washed with saturated brine, dried over anhydrous sodium sulfate, concentrated, and purified by preparative plate to yield Intermediate 1-7, 27.4 mg, with a yield of 54.8%. LC-MS m/z (ESI) $[M+H]^+$ calculated for $C_{30}H_{38}N_7O_2Si$: 556.3; measured: 556.3.

**Synthesis of Compound 1: 1-(2-(4-(2-(6-methylpyridin-2-yl)-6,7-dihydro-SH-pyrrolo[1,2-a]imidazol-3-yl)pyridin-2-yl)-4,6-dihydropyrrolo[3,4-d]imidazol-5(1H)-yl)ethan-1-one**

Intermediate 1-7 (27.4 mg, 0.1 mmol) was dissolved in 4 ml of methanol, to which 2 ml of concentrated hydrochloric acid was added, and the resulting mixture was reacted at 50°C for 3 hours. The reaction mixture was concentrated, dissolved in 5 ml of methanol, to which 0.5 ml of aqueous ammonia was added, and the resulting mixture was concentrated and then purified with a reverse-phase silica gel column to yield the final product, 6.2 mg, with a yield of 29.6%. LC-MS m/z (ESI) $[M+H]^+$ calculated for $C_{24}H_{24}N_7O$: 426.2; measured: 426.2.

**Example 2: 2-(4-(2-(6-methylpyridin-2-yl)-6,7-dihydro-5H-pyrrolo[1,2-a]imidazol-3-yl)pyridin-2-yl)-1,4,5,6-tetrahydropyrrolo[3,4-d]imidazole**

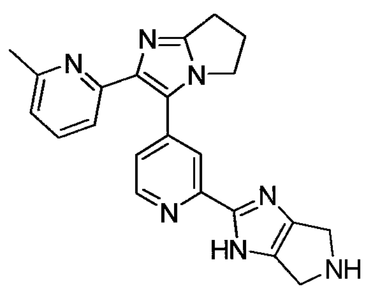

2

**The synthetic route for Compound 2:**

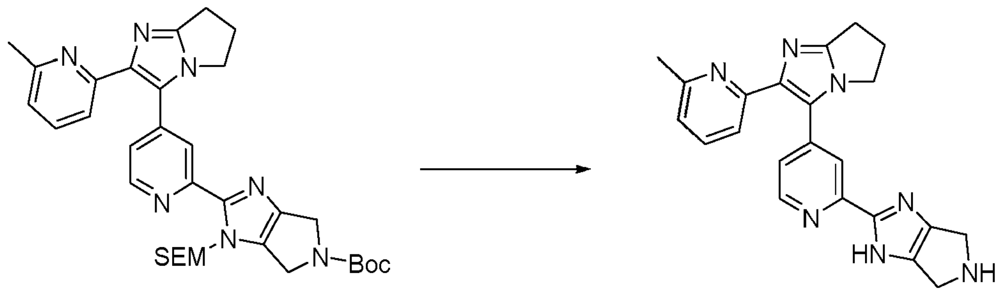

1-5

2

**Synthesis method:**

**Synthesis of compound 2: 2-(4-(2-(6-methylpyridin-2-yl)-6,7-dihydro-5H-pyrrolo[1,2-a]imidazol-3-yl)pyridin-2-yl)-1,4,5,6-tetrahydropyrrolo[3,4-d]imidazole**

Intermediate 1-5 (36.5 mg, 0.1 mmol) was dissolved in 4 ml of methanol, to which 2 ml of concentrated hydrochloric acid was added. It was allowed to react at 50°C for 3 hours. The reaction mixture was concentrated, and the resulting resiue was dissolved in 5 ml of methanol, to which 0.5 ml of aqueous ammonia was added. The resulting mixture was concentrated and purified using a reverse-phase silica gel column to yield the final product, 4.1 mg, with a yield of 17.9%. LC-MS m/z (ESI) $[M+H]^+$ calculated for $C_{22}H_{22}N_7$: 384.2; measured: 384.2.

**<u>Example 3: 5-Methyl-2-(4-(2-(6-methylpyridin-2-yl)-6,7-dihydro-5H-pyrrolo[1,2-a]imidazol-3-yl)pyridin-2-yl)-1,4,5,6-tetrahydropyrrolo[3,4-d]imidazole</u>**

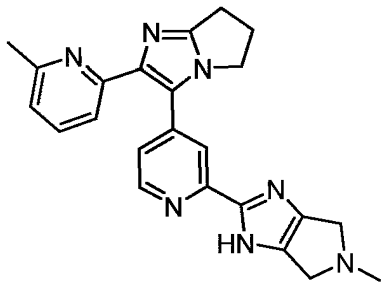

3

**Synthetic route for Compound 3:**

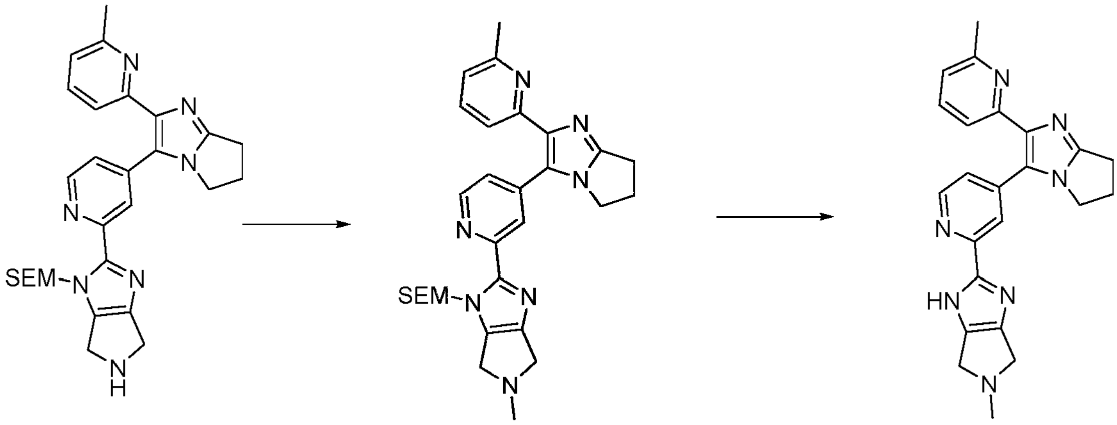

1-6

3

Synthesis method:

**Synthes of Compound 3: 5-methyl-2-(4-(2-(6-methylpyridin-2-yl)-6,7-dihydro-5H-pyrrolo[1,2-a]imidazol-3-yl) pyridin-2-yl)-1,4,5,6-tetrahydropyrrolo[3,4-d]imidazole**

Intermediate 1-6 (50.0 mg, 0.1 mmol) and 30% formaldehyde aqueous solution (11.7 mg, 0.4 mmol) were dissolved in 5 ml of DCM. The resulting mixture was stirred at room temperature for 0.5 hours, then cooled to 0°C, to which sodium triacetoxyborohydride (82.8 mg, 0.4 mmol) was added. It was allowed to react at room temperature for 2 hours, then the reaction was quenched with water, extracted with DCM, and the organic layers were combined, washed with saturated brine, and concentrated. The concentrate was dissolved in 4 ml of methanol, to which 2 ml of concentrated hydrochloric acid was added, and it was allowed to react at 50°C for 2 hours. The reaction mixture was concentrated, dissolved in 5 ml of methanol, to which 0.5 ml of aqueous ammonia was added. The mixture was concentrated again, and the product was purified using a preparative plate to yield the final product, 3.2 mg, with a yield of 8.3%.

$^1H$ NMR (400 MHz, $CD_3OD$) δ8.61 (d, J= 4.0 Hz, 1H), 8.14 (s, 1H),7.74-7.70 (m, 1H), 7.54 (d, *J* = 8.0 Hz, 1H),7.43-7.41 (m, 1H), 7.19 (d, *J* = 8.0 Hz, 1H), 4.59 (s, 4H), 4.27-4.23 (m, 2H), 3.21 (s, 3H), 3.07-3.03 (m, 2H), 2.79-2.72 (m, 2H), 2.36 (s, 3H).

**Example 4: (4-Methylpiperazin-1-yl)(2-(4-(2-(6-methylpyridin-2-yl)-6,7-dihvdro-5H-pyrrolo[1,2-a]imidazol-3-yl) pyridin-2-yl)-4,6-dihydropyrrolo[3,4-dlimidazol-5(1H)-vl)ketone (MDI-980)**

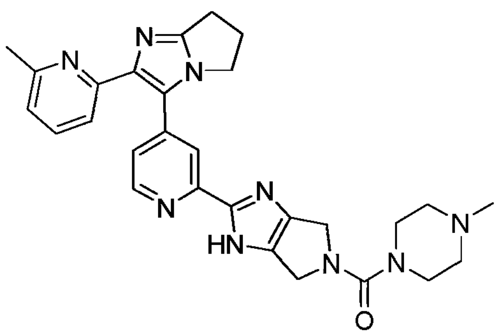

**4**

**Synthetic route for Compound 4:**

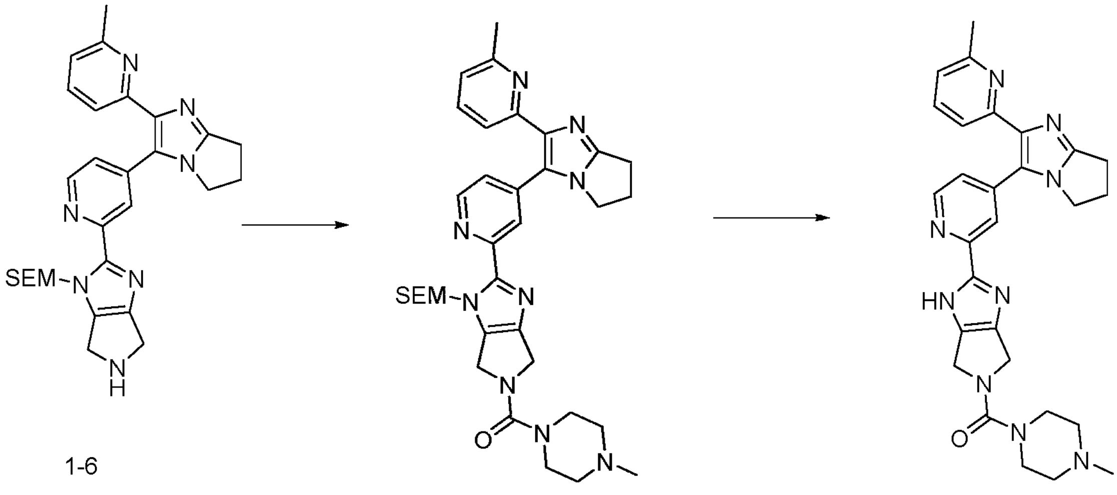

1-6

4

**Synthesis method:**

**Synthesis** of **Compound** 4: **(4-methylpiperazin-1-yl)(2-(4-(2-(6-methylpyridin-2-yl)-6,7-dihydro-5H-pyrrolo[1,2-a] imidazol-3-yl)pyridin-2-yl)-4,6-dihydropyrrolo [3,4-d]imidazol-5(1H)-yl)ketone**

Intermediate 1-6 (40.0 mg, 0.1 mmol) was dissolved in 10 ml of DCM. Triphosgene (23.1 mg, 0.1 mmol) was added to the system, and the system was cooled to 0°C. Triethylamine (80.8 mg, 0.8 mmol) was then added dropwise, and it was allowed to react 0.5 hours. N-Methylpiperidine (8.0 mg, 0.1 mmol) was added to the system, and it was warmed to room temperature for reaction. After the reaction was complete, water was added to quench the reaction, and the reaction mixture was extracted with DCM. The organic layers were combined, washed with saturated brine, and concentrated. The concentrate was dissolved in 4 ml of methanol, to which 2 ml of concentrated hydrochloric acid was added, and it was allowed to react at 50°C for 2 hours. The reaction mixture was concentrated, and the concentrate was dissolved in 5 ml of methanol, to which 0.5 ml of aqueous ammonia was added. The mixture was concentrated again, and purified using a preparative plate to yield the final product, 2.2 mg, with a yield of 5.5%. LC-MS m/z (ESI) $[M+H]^+$ calculated for $C_{28}H_{32}N_9O$: 510.3; measured: 510.3.

**Example 6: (2-(4-(4-(6-methylpyridin-2-yl)-1H-imidazol-5-yl)pyridin-2-yl)-4,6-dihydropyrrolo[3,4-d]imidazol-5(1H)-yl)(pyrrolidin-1-yl)keone**

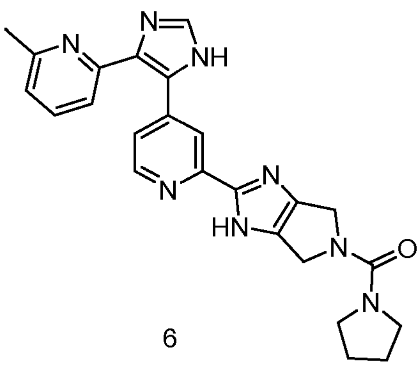

6

**Synthetic route for Compound 6:**

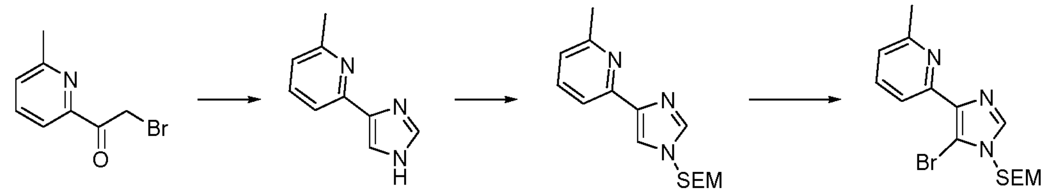

1-b 6-1 6-2 6-3

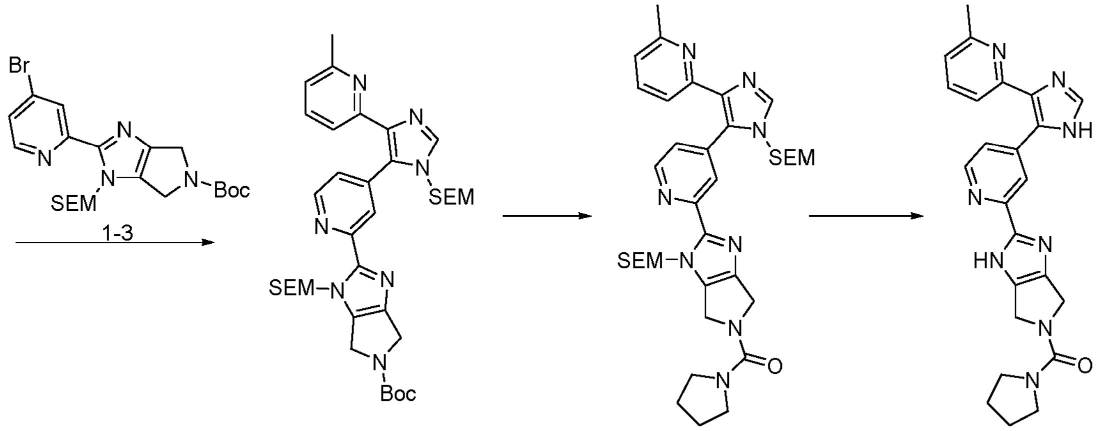


6-4 6-5 6

**Synthesis method:**

**Synthesis of Intermediate 6-1: 2-(1H-Imidazol-4-yl)-6-methylpyridine**

Intermediate 1-b (0.8 g, 3.7 mmol) was dissolved in 30 ml of ethylene glycol, to which formamide acetate (2.0 g, 18.7 mmol) was added. The resulting mixture was protected with nitrogen and it was allowed to react at 130°C for 2 hours. The reaction mixture was cooled to room temperature, to which water was added, and the pH was adjusted to 10. The mixture was extracted with ethyl acetate (EA). The organic phase was washed with saturated brine, dried over anhydrous sodium sulfate, and concentrated to yield Intermediate 6-1, 400.0 mg. LC-MS m/z (ESI) $[M+H]^+$ calculated for $C_9H_{10}N_3$: 160.1; measured: 160.1.

**Synthesis of Intermediate 6-2: 2-Methyl-6-(1-((2-(trimethylsilyl) ethoxy) methyl)-1H-imidazol-4-yl)pyridine**

Intermediate 6-1 (400.0 mg, 2.5 mmol) was dissolved in 10 ml of THF, to which sodium hydride (120.0 mg, 3.0 mmol, 60%) was added. It was allowed to react at 0°C for 0.5 hours, then to which SEMCl (628.4 mg, 3.8 mmol) was added, and it was allowed to react at room temperature for 2 hours. The reaction was quenched with saturated ammonium chloride solution, extracted with EA. The organic phase was washed with saturated brine, dried over anhydrous sodium sulfate, concentrated, and purified by column chromatography to yield Intermediate 6-2, 140.0 mg, with a yield of 19.3%.

**Synthesis of Intermediate 6-3: 2-(5-bromo-1-((2-(trimethylsilyl)ethoxy) methyl)-1H-imidazol-4-yl)-6-methylpyridine**

Intermediate 6-2 (140.0 mg, 0.5 mmol) was dissolved in 10 ml of DCM, to which N-bromosuccinimide (NBS) (77.5 mg, 0.4 mmol) was added in portions. It was allowed to react at room temperature for 10 minutes. The reaction was quenched with saturated sodium bicarbonate solution, and extracted with DCM. The organic phase was washed with saturated brine, dried over anhydrous sodium sulfate, concentrated, and purified by column chromatography to yield Intermediate 6-3, 90.0 mg, with a yield of 50.5%. LC-MS m/z (ESI) $[M+H]^+$ calculated for $C_{15}H_{23}BrN_3OSi$: 368.1; measured: 368.1.

**Synthesis of Intermediate 6-4: tert-butyl 2-(4-(4-(6-Methylpyridin-2-yl)-1-((2-(trimethylsilyl)ethoxy)methyl)-1H-imidazol-5-yl)pyridin-2-yl)-1-((2-(trimethylsilyl) ethoxy) methyl)-4,6-dihydropyrrolo[3,4-d]imidazol-5(1H)-carboxylate**

Intermediate 1-3 (300.0 mg, 0.6 mmol) was dissolved in 10 ml of 1,4-dioxane, to which potassium acetate (178.0

mg, 1.8 mmol) was added, followed by the addition of bis(pinacolato)diboron (184.0 mg, 0.7 mmol). The mixture was protected with nitrogen, to which Pd(dppf)$Cl_2$ (45.0 mg, 0.1 mmol) was added, and it was allowed to react at 85°C overnight. Intermediate 6-3 (90.0 mg, 0.2 mmol), potassium acetate (178.0 mg, 1.8 mmol), 1 ml of $H_2O$, and Pd(dppf)$Cl_2$ (45.0 mg, 0.1 mmol) were then added, and it was allowed to react at 100°C for 6 hours. The mixture was filtered, concentrated, and purified to yield Intermediate 6-4, 50.0 mg, with a yield of 11.5%.

**Synthesis of Intermediate 6-5: (2-(4-(4-(6-Methylpyridin-2-yl)-1-((2-(trimethylsilyl)ethoxy)methyl)-1H-imidazol-5-yl)pyridin-2-yl)-1-((2-(trimethylsilyl) ethoxy) methyl)-4,6-dihydropyrrolo[3,4-d]imidazol-5(1H)-yl)(pyrrolidin-1-l)ketone**

Intermediate 6-4 (50.0 mg, 0.1 mmol) was dissolved in 10 ml of DCM. Under nitrogen protection, $ZnBr_2$ (32.0 mg, 0.1 mmol) was added, and it was allow to react at room temperature overnight. Triethylamine (1 ml) was added, and it was allowed to react at room temperature for 0.5 hours. Pyrrolidin-1-carbonyl chloride (10.0 mg, 0.1 mmol) was then added, and it was allowed to react at room temperature for 2 hours. Water was added, the mixture was extracted with DCM, and the organic phase was washed with saturated brine, dried over anhydrous sodium sulfate, concentrated, and purified to yield Intermediate 6-5, 20.0 mg, with a yield of 42.9%. LC-MS m/z (ESI) $[M+H]^+$ calculated for $C_{36}H_{53}N_8O_3Si_2$: 701.4; measured: 701.4.

**Synthesis of Compound 6: (2-(4-(4-(6-Methylpyridin-2-yl)-1H-imidazol-5-yl)pyridin-2-yl)-4,6-dihydropyrrolo[3,4-d]imidazol-5(1H)-yl)(pyrrolidin-1-yl)ketone**

Intermediate 6-5 (20.0 mg, 0.03 mmol) was dissolved in 2 ml of methanol, to which 1 ml of hydrochloric acid was added, and it was allowed to react at 50°C for 3 hours. The mixture was concentrated, adjusted to basic pH with aqueous ammonia, concentrated, and purified to obtain the product, 4.7 mg, with a yield of 37.4%. LC-MS m/z (ESI) $[M+H]^+$ calculated for $C_{24}H_{25}N_8O$: 441.2; measured: 441.2.

**Synthesis of Compound 7: (4-Methylpiperazin-1-yl)(2-(4-(3-(6-methylpyridin-2-yl)-1H-pyrazol-4-yl)pyridin-2-yl)-4,6-dihydropyrrolo[3,4-d]imidazol-5(1H)-yl)ketone**

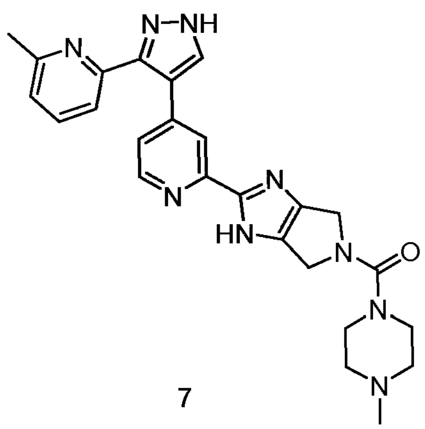

7

Synthetic route for Compound 7:

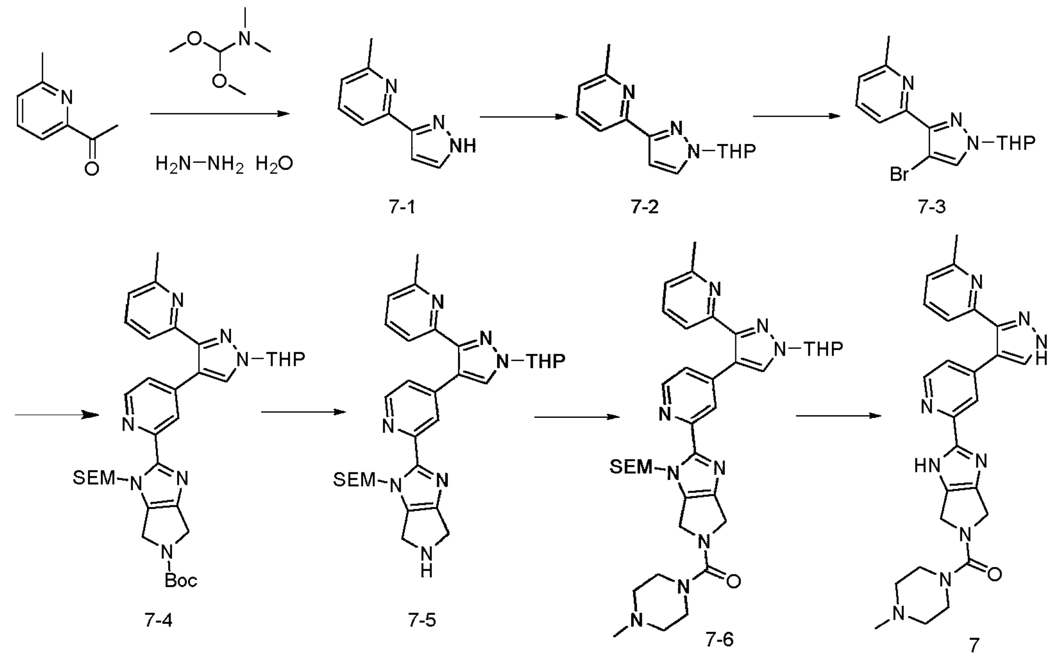


7-1 7-2 7-3 7-4 7-5 7-6 7

**Synthesis method:**

**Synthesis of Intermediate 7-1: 2-Methyl-6-(1H-pyrazol-3-yl)pyridine**

1-(6-Methylpyridin-2-yl)ethan-1-one (1.0 g, 7.4 mmol) was dissolved in 10 ml of DMF-DMA and stirred at 110°C for 20 hours. The reaction solution was cooled to room temperature and concentrated to a yellow solid. The solid was dissolved in 8 ml of ethanol, to which 4 ml of hydrazine hydrate was added, and it was allowed to react at 90°C for 30 minutes. Water was added to the reaction solution, and the resulting was extracted with EA twice, and the organic layers were combined, washed with saturated brine, dried over anhydrous sodium sulfate, and concentrated to yield crude Intermediate 7-1, 1.1 g, with a yield of 90.2%.

**Synthesis of Intermediate 7-2: 2-Methyl-6-(1-(tetrahydro-2H-pyran-2-yl)-1H-pyrazol-3-yl)pyridine**

Intermediate 7-1 (1.1 g, 6.7 mmol) was dissolved in 10 ml of DCM, to which p-toluenesulfonic acid (1.3 g, 6.7 mmol) was added, and DHP (0.7 ml, 8.0 mmol) was added dropwise at room temperature. It was allowed to react at room temperature for 2 hours. Water was added to the reaction solution, and the resulting mixture was extracted with DCM twice, and the organic layers were combined, washed with saturated brine, dried over anhydrous sodium sulfate, concentrated, and purified by silica gel column chromatography to yield Intermediate 7-2, 1.6 g, with a yield of 95.5%.

$^1H$ NMR (400 MHz, $CDCl_3$) $\delta$ 7.79 (d, J = 7.8 Hz, 1H), 7.70-7.67 (m, 1H), 7.63-7.59 (m, 1H), 7.08 (d, J = 7.5 Hz, 1H), 6.96 (d, J = 2.5 Hz, 1H), 5.51-5.48 (m, 1H), 4.15-4.11 (m, 1H), 3.77-3.72 (m, 1H), 2.62 (s, 3H), 2.16-2.11 (m, 3H), 1.77-1.71 (m, 3H).

**Synthesis of Intermediate 7-3: 2-(4-Bromo-1-(tetrahydro-2H-pyran-2-yl)-1H-pyrazol-3-yl)-6-methylpyridine**

Intermediate 7-2 (250.0 mg, 1.0 mmol) was dissolved in 10 ml of DCM, to which NBS (274.3 mg, 1.5 mmol) was added, and the mixture was stirred at room temperature for 15 minutes until the reaction was complete. Water was added to the reaction solution, the resulting mixture was extracted with DCM twice, and the organic layers were combined, washed with saturated brine, dried over anhydrous sodium sulfate, concentrated, and purified by silica gel column chromatography to yield Intermediate 7-3, 223.2 mg, with a yield of 67.4%.

$^1H$ NMR (400 MHz, $CDCl_3$) $\delta$ 7.77-7.74 (m, 2H), 7.66 (t, J = 7.7 Hz, 1H), 7.15 (d, J = 7.8 Hz, 1H), 5.51-5.48 (m, 1H), 4.12-4.08 (m, 1H), 3.77-3.69 (m, 1H), 2.67 (s, 3H), 2.17-2.00 (m, 3H), 1.74-1.64 (m, 3H).

**Synthesis of Intermediate 7-4: tert-butyl 2-(4-(3-(6-Methylpyridin-2-yl)-1-(tetrahydro-2H-pyran-2-yl)-1H-pyrazol-4-yl)pyridin-2-yl)-1-(2-(trimethylsilyl)ethoxy) methyl)-4,6-dihydropyrrolo[3,4-d]imidazole-5(1H)-carboxylate**

Tert-butyl 2-(4-bromopyridin-2-yl)-1-(2-(trimethylsilyl)ethoxy)methyl)-4,6-dihydropyrrolo[3,4-d]imidazole-5(1H)-carboxylate (300.0 mg, 0.6 mmol) and bis(pinacolato)diboron (184.5 mg, 0.7 mmol) were dissolved in 8 ml of dioxane, to which potassium acetate (118.9 mg, 1.2 mmol) and Pd(dppf)$Cl_2$ (45.0 mg, 0.1 mmol) were added. The mixture was purged with nitrogen, andit was allowed to react at 90°C for 16 hours. The reaction solution was cooled to room temperature, to the reaction solution, Intermediate 7-3 (195.1 mg, 0.6 mmol), potassium carbonate (251.1 mg, 1.8 mmol), additional Pd(dppf)$Cl_2$ (45.0 mg, 0.1 mmol), and 2 ml of water were added. The mixture was purged with nitrogen and it was allowed to react at 100°C for 24 hours. The reaction solution was filtered, and the filtrate was diluted with water and extracted with EA twice. The combined organic layers were washed with saturated brine, dried over anhydrous sodium sulfate, concentrated, and purified by silica gel column chromatography to yield Intermediate 7-4, 203.6 mg, with a yield of 50.8%.

$^1$H NMR (400 MHz, $CDCl_3$) δ 8.38 (t, J = 5.7 Hz, 1H), 8.32-8.27 (m, 1H), 8.03 (d, J = 5.2 Hz, 1H), 7.58 (t, J = 7.7 Hz, 1H), 7.46-7.43 (m, 1H), 7.23-7.20 (m, 1H), 7.11 (d, J = 7.7 Hz, 1H), 6.07-6.02 (m, 2H), 5.51-5.47 (m, 1H), 4.61-4.44 (m, 4H), 4.13-4.10 (m, 1H), 3.73 (t, J = 10.8 Hz, 1H), 3.62-3.54 (m, 2H), 2.53 (s, 3H), 2.22-2.07 (m, 3H), 1.72-1.64 (m, 3H), 1.53 (s, 9H), 0.95-0.85 (m, 2H), -0.06 (s, 9H).

**Synthesis of Intermediate 7-5: 2-(4-(3-(6-Methylpyridin-2-yl)-1-(tetrahydro-2H-pyran-2-yl)-1H-pyrazol-4-yl)pyridin-2-yl)-1-(2-(trimethylsilyl)ethoxy) methyl)-1,4,5,6-tetrahydropyrrolo[3,4-d]imidazole**

Intermediate 7-4 (65.0 mg, 0.1 mmol) was dissolved in 10 ml of DCM, to which $ZnBr_2$ (110.9 mg, 0.5 mmol) was added, and the mixture was stirred at room temperature until the reaction was complete. The reaction was quenched with aqueous ammonia, extracted with DCM, and the organic layer was washed with saturated brine, dried over anhydrous sodium sulfate, concentrated, to yield Intermediate 7-5, 55.1 mg, with a yield of 99.7%.

**Synthesis of Intermediate 7-6: (4-Methylpiperazin-1-yl)(2-(4-(3-(6-methylpyridin-2-yl)-1-(tetrahydro-2H-pyran-2-yl)-1H-pyrazol-4-yl)pyridin-2-yl)-1-((2-(trimethylsilyl)ethoxy)methyl)-4,6-dihydropyrrolo[3,4-d]imidazole-5(1H)-yl)ketone**

Intermediate 7-5 (55.0 mg, 0.1 mmol) and triphosgene (29.2 mg, 0.1 mmol) were dissolved in 10 ml of DCM. The solution was cooled to 0°C, to which triethylamine (99.5 mg, 0.1 mmol) was added. The reaction was allowed to react for 5 minutes. To the reaction system, N-methylpiperazine (19.7 mg, 0.2 mmol) was added and the mixture was then allowed to warm to room temperature and stir until the reaction was complete. The reaction was quenched with water, extracted with DCM, and the combined organic layers were washed with saturated brine, concentrated and purified by silica gel column chromatography to yield Intermediate 7-6, 35.2 mg, with a yield of 51.9%.

**Synthesis of Compound 7: (4-Methylpiperazin-1-yl)(2-(4-(3-(6-methylpyridin-2-yl)-1H-pyrazol-4-yl)pyridin-2-yl)-4,6-dihydropyrrolo[3,4-d]imidazole-5(1H)-yl)ketone**

Intermediate 7-6 (35.2 mg, 0.1 mmol) was dissolved in 4 ml of methanol, to which 2 ml of concentrated hydrochloric acid was added. It was allowed to react at 45°C for 6 hours. The reaction mixture was then concentrated. The concentrate was dissolved in 5 ml of methanol and adjusted to basic pH with 0.5 ml of aqueous ammonia. The mixture was concentrated and purified to yield the final product, 5.0 mg, with a yield of 20.8%. LC-MS m/z (ESI) $[M+H]^+$ for $C_{25}H_{28}N_9O$ calculated: 470.2; measured: 470.2.

**Examples 9 to 11**

The following examples were prepared according to the synthetic route of Example 1 using appropriate starting materials.

| Examples | Structural Formula and Name of Compounds | $^{1}$HNMR(400 MHz) | Theoretical LC-MS $(M+1)^+$ | Measured LC-MS $(M+1)^+$ |
|---|---|---|---|---|
| 9 | 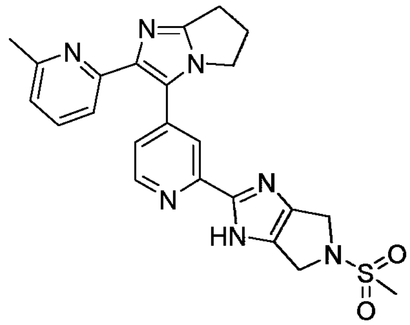 9 2-(4-(2-(6-Methylpyridin-2-yl)-6,7-dihydro-5H-pyrrolo[1,2-a]imidazol-3-yl)pyridin-2-yl)-5-(methylsulfonyl)-1,4,5,6-tetrahydropyrrolo[3,4-d]imidazole | $(CD_3OD)$ δ 8.56 (d, *J* = 8.0 Hz, 1H), 8.11 (s, 1H), 7.69 - 7.73 (m, 1H), 7.54 (d, *J* = 8.0 Hz, 1H), 7.36 (d, *J* = 4.0 Hz, 1H),7.16 (d, *J= 8.0* Hz, 1H), 4.55 (s, 4H), 4.23-4.27 (m, 2H),3.01 - 3.05(m, 2H), 3.00 (s, 3H), 2.82 -2.85 (m, 2H), 2.35 (s, 3H). | | |
| 10 | 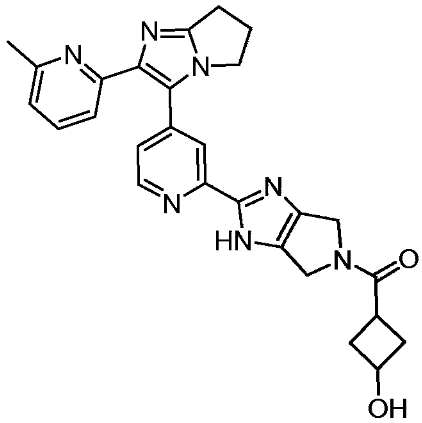 10 (3-Hydroxycyclobutyl)(2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)ketone | | 482.2 | 482.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 11 | 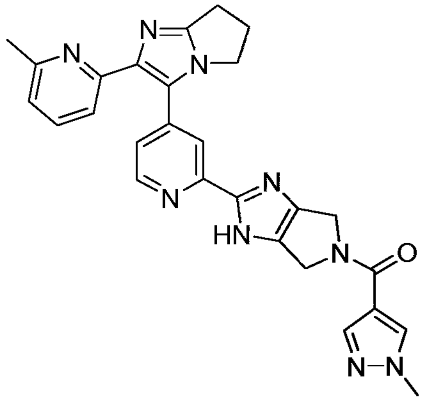 11 (1-Methyl-1H-pyrazol-4-yl)(2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)ketone | | 492.2 | 492.2 |

**Examples 12 to 13**

The following examples were prepared according to the synthetic route of Example 3 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 12 | 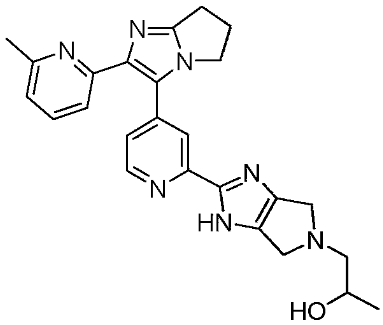 12 1-(2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)propan-2-ol | | 442.2 | 442.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 13 | 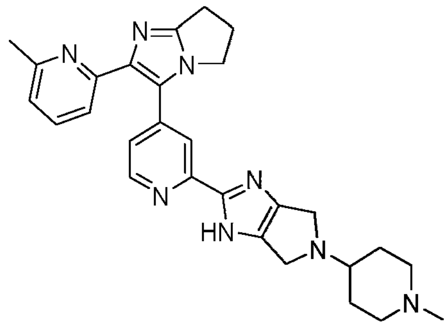 13 5-(1-Methylpiperidin-4-yl)-2-(4-(2-(6-methylpyridin-2-yl)-6,7-dihydro-5H-pyrrolo[1,2-a]imidazol-3-yl)pyridin-2-yl)-1,4,5,6-tetrahydropyrrolo[3,4-d]imidazole | | 481.3 | 481.2 |

## Examples 14 to 15

The following examples were prepared according to the synthetic route of Example 4 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 14 | 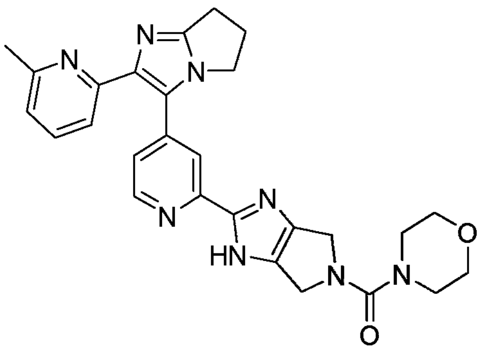 14 (2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)(morpholin-4-yl)ketone | | 497.2 | 497.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 15 | 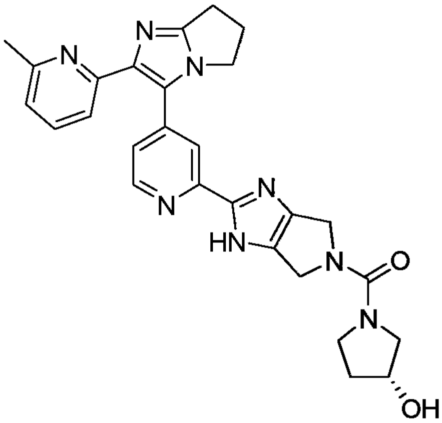 15 (R)-(3-Hydroxypyrrolidin-1-yl)(2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)ketone | | 497.2 | 497.2 |

**Examples 22 to 23**

The following examples were prepared according to the synthetic route of Example 7 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 22 | 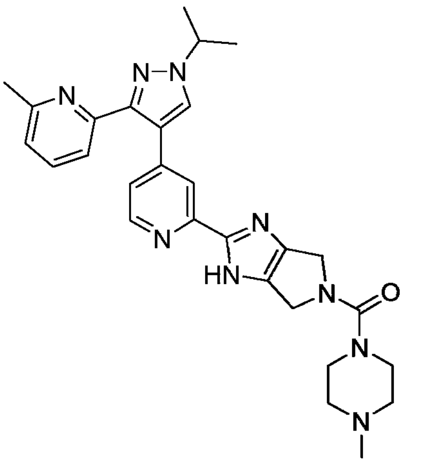 22 (2-(4-(1-Isopropyl-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 512.3 | 512.3 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 23 | 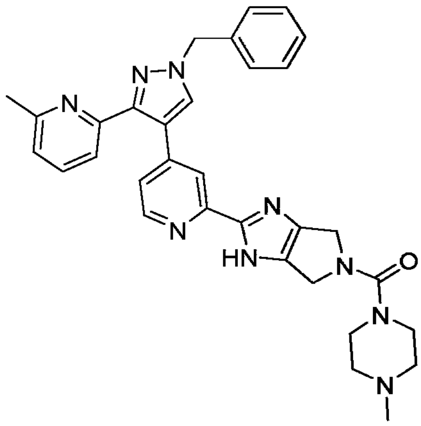 23 (2-(4-(1-Benzyl-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 560.3 | 560.3 |

**Examples 25 to 26**

The following examples were prepared according to the synthetic route of Example 3 using appropriate starting materials.

| | | | | |
|---|---|---|---|---|
| 25 | 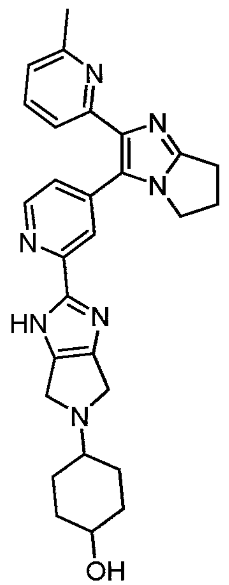 25 4-(2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)cyclohexan-1-ol | | 482.3 | 482.3 |

(continued)

| | | | | |
|---|---|---|---|---|
| 26 | 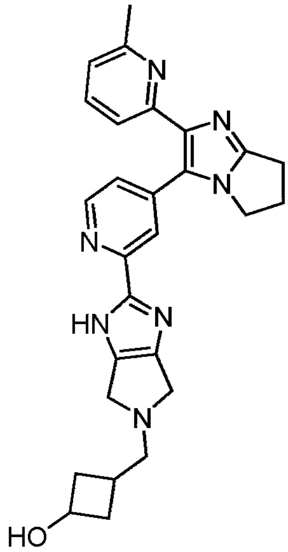 **26** 3-((2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)methyl)cyclobutanol | | 468.2 | 468.2 |

**Examples 27 to 34**

The following examples were prepared according to the synthetic route of Example 4 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 27 | 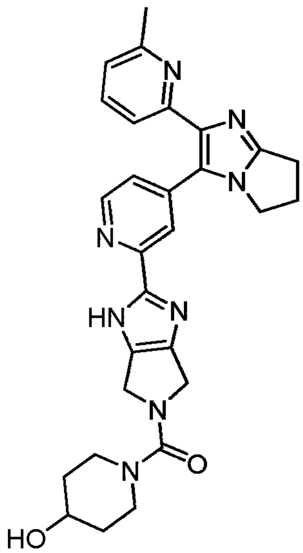 **27** (4-Hydroxypiperidin-1-yl)(2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl) ketone | | 511.3 | 511.3 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 28 | 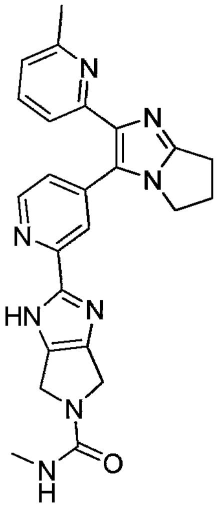 28 N-Methyl-2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-carboxamide | | 441.2 | 441.2 |
| 29 | 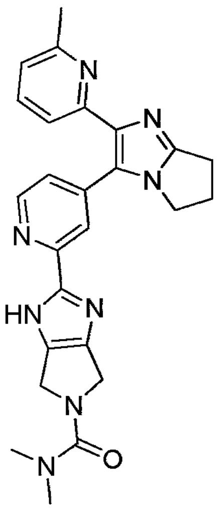 29 N,N-Dimethyl-2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-carboxamide | | 455.2 | 455.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 30 | 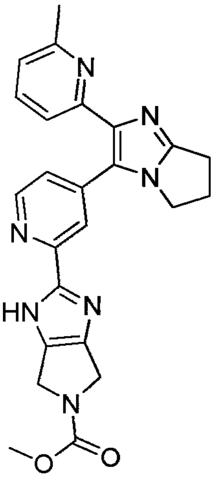 **30** Methyl 2-(4-(2-(6-Methylpyridin-2-yl)-6,7-dihydro-5H-pyrrolo[1,2-a]imidazol-3-yl)pyridin-2-yl)-4,6-dihydro-pyrrolo[3,4-d]imidazol-5(1H)-carboxylate | | 442.2 | 442.2 |
| 31 | 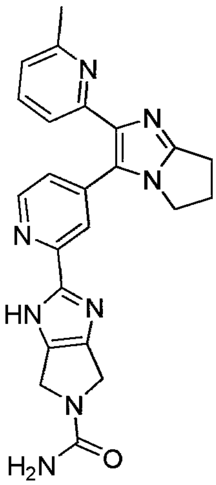 **31** 2-(4-(2-(6-Methylpyridin-2-yl)-6,7-dihydro-5H-pyrrolo [1,2-a]imidazol-3-yl)pyridin-2-yl)-4,6-dihydropyrrolo [3,4-d]imidazol-5(1H)-carboxamide | | 427.2 | 427.2 |

(continued)

| Examples | Structural Formula and Name of Compounds | 1HNMR(400 MHz) | Theoretical LC-MS $(M+1)^+$ | Measured LC-MS $(M+1)^+$ |
|---|---|---|---|---|
| 32 | 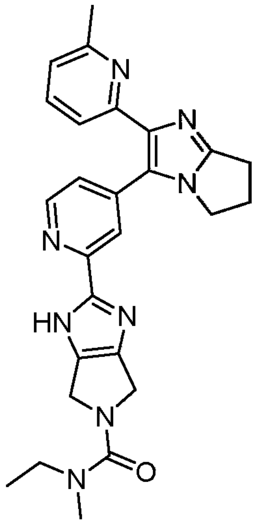 32 N-Ethyl-N-methyl-2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-carboxamide | | 469.2 | 469.2 |
| 33 | 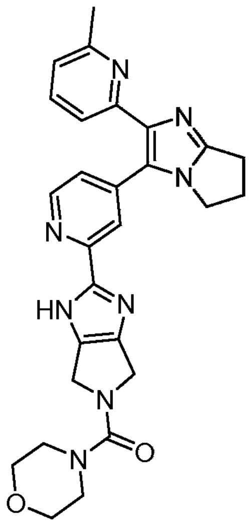 33 (2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)(morpholinyl)ketone | | 497.2 | 497.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 34 | 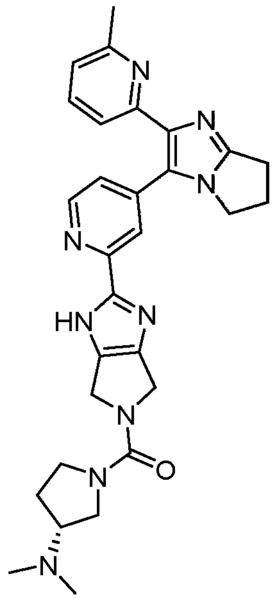 34 (R)-(3-(Dimethylamino)pyrrolidin-1-yl)(2-(4-(2-(6-methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)ketone | | 524.3 | 524.3 |

**Examples 37 to 60**

The following examples were prepared according to the synthetic route of Example 7 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 37 | 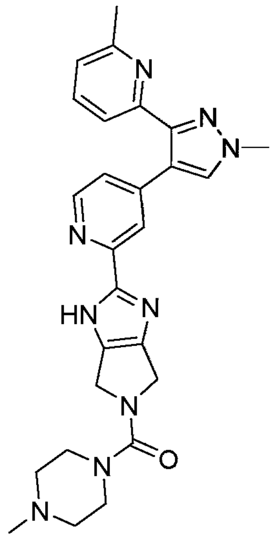 37 (2-(4-(1-Methyl-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 484.3 | 484.3 |
| 38 | 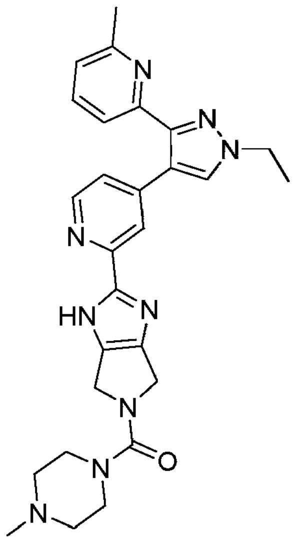 38 (2-(4-(1-Ethyl-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 498.3 | 498.3 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 39 | 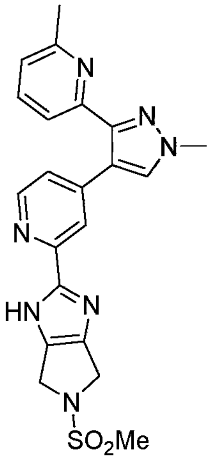 39 2-[4-(1-Methyl-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 436.2 | 436.2 |
| 40 | yl)ketone 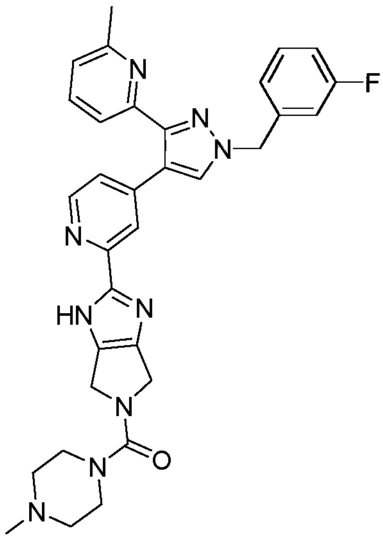 40 (2-(4-(1-(3-Fluorobenzyl)-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 578.3 | 578.4 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 41 | 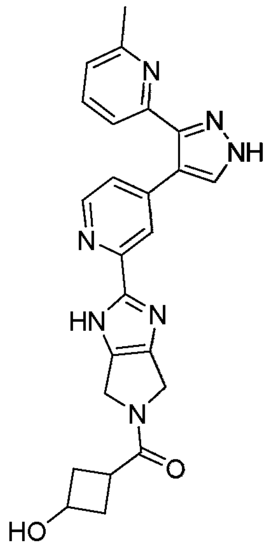<br>41<br>(3-Hydroxycyclobutyl)(2-(4-(3-(6-methylpyridin-2-yl)-1H-pyrazol-4-yl)pyridin-2-yl)-4,6-dihydropyrrolo [3,4-d]imidazol-5(1H)-yl)ketone | | 442.2 | 442.2 |
| 42 | 5(1H)-yl)ketone<br>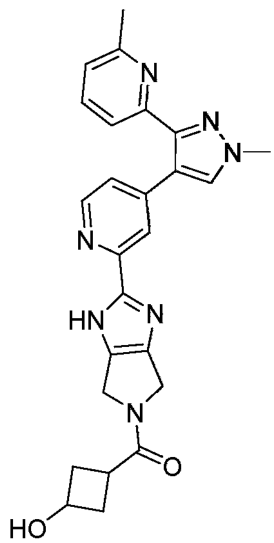<br>42<br>(3-Hydroxycyclobutyl)(2-(4-(1-methyl-3-(6-methylpyridin-2-yl)-1H-pyrazol-4-yl)pyridin-2-yl)-4,6-dihydropyrrolo[3,4-d]imidazol-5(1H)-yl)ketone | | 456.2 | 456.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 43 | 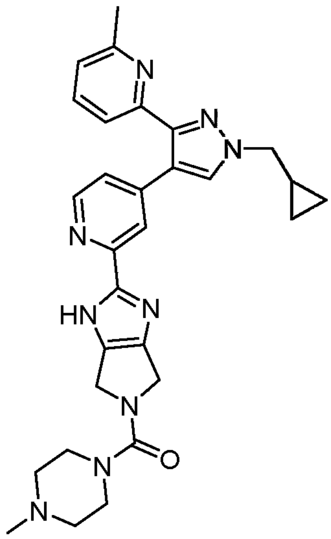 43 (2-(4-(1-Cyclopropylmethyl-3-(6-methylpyridin-2-yl)-1H-pyrazol-4-yl)pyridin-2-yl)-4,6-dihydropyrrolo[3,4-d]imidazol-5(1H)-yl)(4-methylpiperazin-1-yl)ketone | | 524.3 | 524.3 |
| 44 | 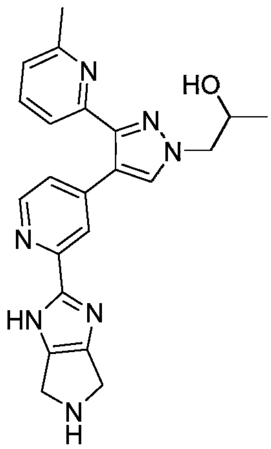 44 3-(3-(6-Methylpyridin-2-yl)-4-(2-(1,4,5,6-tetrahydropyrrolo[3,4-d]imidazol-2-yl)pyridin-4-yl)-1H-pyrazol-1-yl)propan-2-ol | | 402.2 | 402.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 45 | 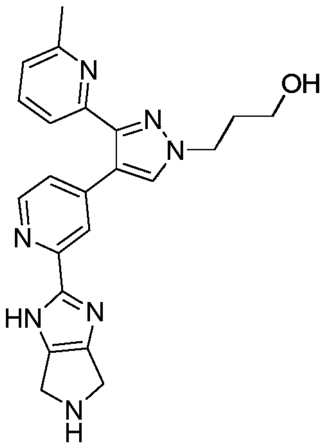 45 3-(3-(6-Methylpyridin-2-yl)-4-(2-(1,4,5,6-tetrahydro-pyrrolo[3,4-d]imidazol-2-yl)pyridin-4-yl)-1H-pyrazol-1-yl)propan-2-ol | | 402.2 | 402.2 |
| 46 | 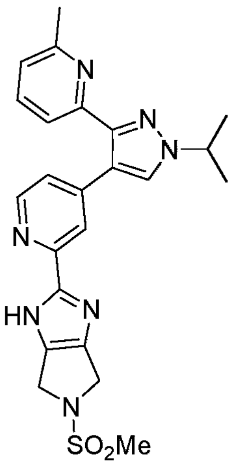 46 2-[4-(1-Isopropyl-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 464.2 | 464.3 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 47 | 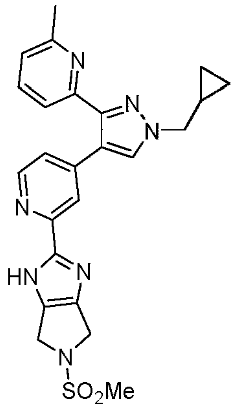 47 2-[4-(1-(Cyclopropylmethyl)-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 476.2 | 476.2 |
| 48 | 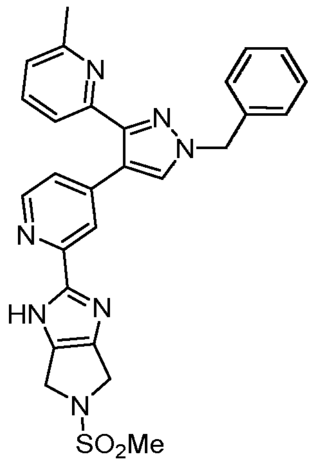 48 2-[4-(1-Benzyl-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 512.2 | 512.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 49 | 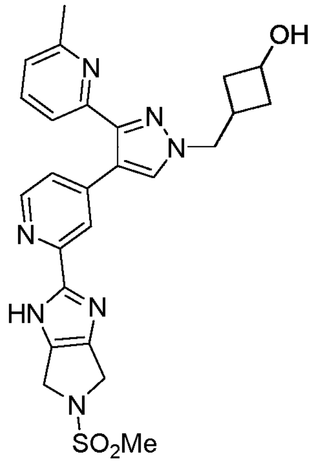 49 3-((3-(6-Methylpyridin-2-yl)-4-(2-(5-(methylsulfonyl)-1,4,5,6-tetrahydropyrrolo[3,4-d]imidazol-2-yl) pyridin-4-yl)-1H-pyrazol-1-yl)methyl)cyclobutanol | | 506.2 | 506.2 |
| 50 | 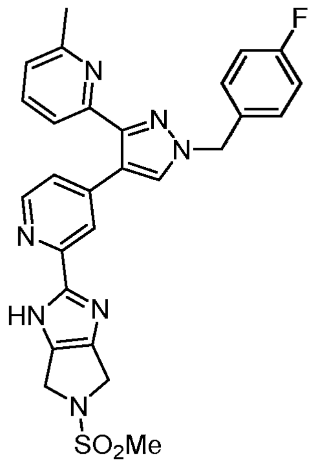 50 2-[4-(1-(4-Fluorobenzyl)-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 530.2 | 530.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 51 | 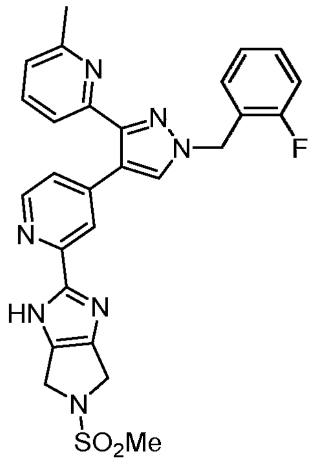 51 2-[4-(1-(2-Fluorobenzyl)-3-(6-methylpyridin-2-yl)-1H-pyrazol-4-yl)pyridin-2-yl]-5-(methylsulfonyl)-1,4,5,6-tetrahydropyrrolo[3,4-d]imidazole | | 530.2 | 530.2 |
| 52 | 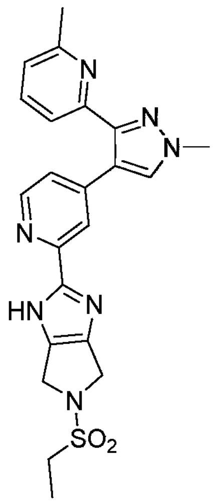 52 5-(Ethylsulfonyl)-2-[4-(1-methyl-3-(6-methylpyridin-2-yl)-1H-pyrazol-4-yl)pyridin-2-yl]-1,4,5,6-tetrahydropyrrolo[3,4-d]imidazole | | 450.2 | 450.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 53 | 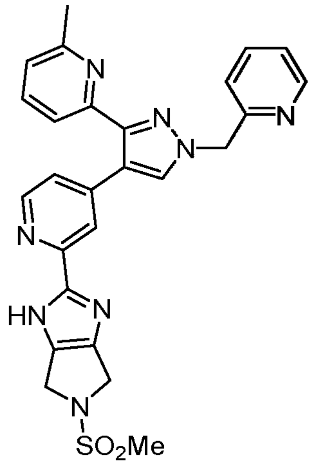 53 2-[4-(3-(6-Methylpyridin-2-yl)-1-(pyridin-2-ylmethyl)-1H-pyrazol-4-yl)pyridin-2-yl]-5-(methylsulfonyl)-1,4,5,6-tetrahydropyrrolo[3,4-d]imidazole | | 513.2 | 513.3 |
| 54 | 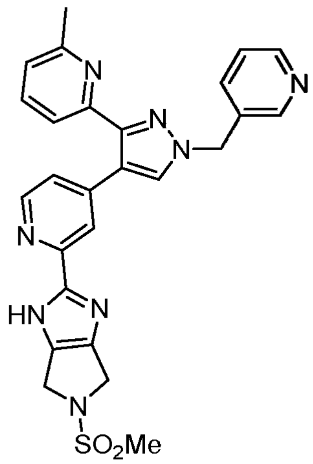 54 2-[4-(3-(6-Methylpyridin-2-yl)-1-(pyridin-3-ylmethyl)-1H-pyrazol-4-yl)pyridin-2-yl]-5-(methylsulfonyl)-1,4,5,6-tetrahydropyrrolo[3,4-d]imidazole | | 513.2 | 513.2 |

(continued)

| Examples | Structural Formula and Name of Compounds | $^{1}$HNMR(400 MHz) | Theoretical LC-MS $(M+1)^+$ | Measured LC-MS $(M+1)^+$ |
|---|---|---|---|---|
| 55 | 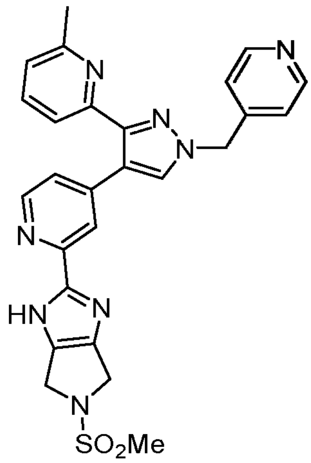 55 2-[4-(3-(6-Methylpyridin-2-yl)-1-(pyridin-4-ylmethyl)-1H-pyrazol-4-yl)pyridin-2-yl]-5-(methylsulfonyl)-1,4,5,6-tetrahydropyrrolo[3,4-d]imidazole | | 513.2 | 513.2 |
| 56 | 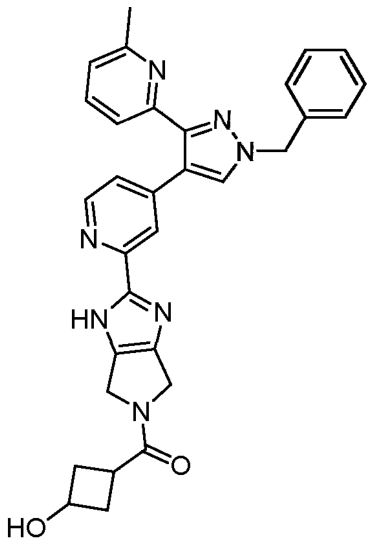 56 (2-(4-(1-Benzyl-3-(6-methyl pyridin-2-yl)-1H-pyrazol-4-yl)pyridin-2-yl)-4,6-dihydropyrrolo[3,4-d]imidazol-5(1H)-yl)(3-hydroxycyclobutyl) ketone | | 532.2 | 532.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 57 | 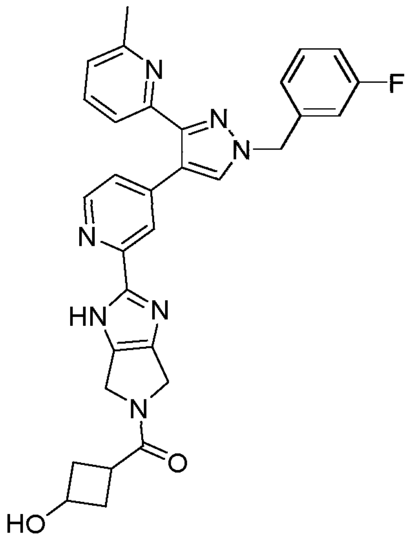 57 (2-(4-(1-(3-Fluorobenzyl)-3-(6-methylpyridin-2-yl)-1H-pyrazol-4-yl)pyridin-2-yl)-4,6-dihydropyrrolo [3,4-d]imidazol-5(1H)-yl)(3-hydroxycyclobutyl) ketone | | 550.2 | 550.2 |
| 58 | 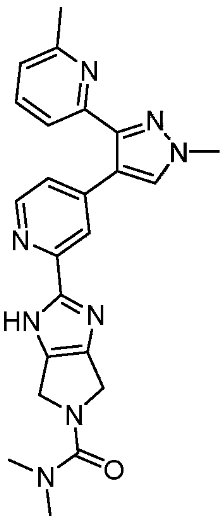 58 N,N-Dimethyl-2-(4-(1-methyl-3-(6-methylpyridin-2-yl)-1H-pyrazol-4-yl)pyridin-2-yl)-4,6-dihydropyrrolo [3,4-d]imidazol-5(1H)-carboxamide | | 429.2 | 429.0 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 59 | 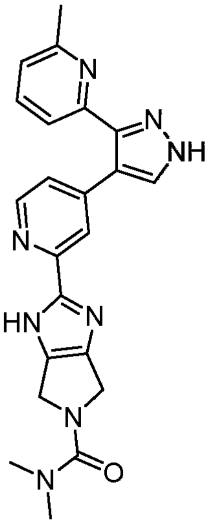 59 N,N-Dimethyl-2-(4-(3-(6-methylpyridin-2-yl)-1H-pyrazol-4-yl)pyridin-2-yl)-4,6-dihydro pyrrolo[3,4-d]imidazol-5(1H)-carboxamide | | 415.2 | 415.2 |
| 60 | 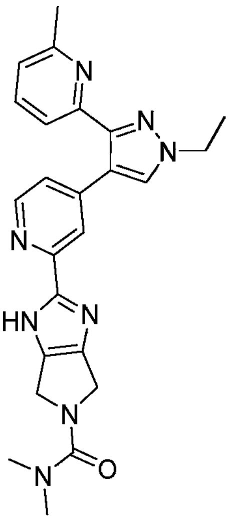 60 2-(4-(1-Ethyl-3-(6-methylpyridin-2-yl)-1H-pyrazol-4-yl)pyridin-2-yl)-N,N-dimethyl-4,6-dihydropyrrolo[3,4-d]imidazol-5(1H)-carboxamide | | 443.2 | 443.2 |

**Example 61: (4-Methylpiperazin-1-yl)(2-(4-(2-(6-methylpyridin-2-yl)-5,6-dihydro-8H-imidazo[2,1-c][1,4]oxazin-3-yl)pyridin-2-yl)-4,6-dihydropyrrolo[3,4-d]imidazol-5(1H)-yl)ketone**

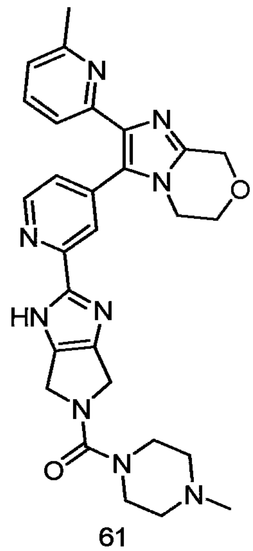

61

**Synthetic route for compound 61:**

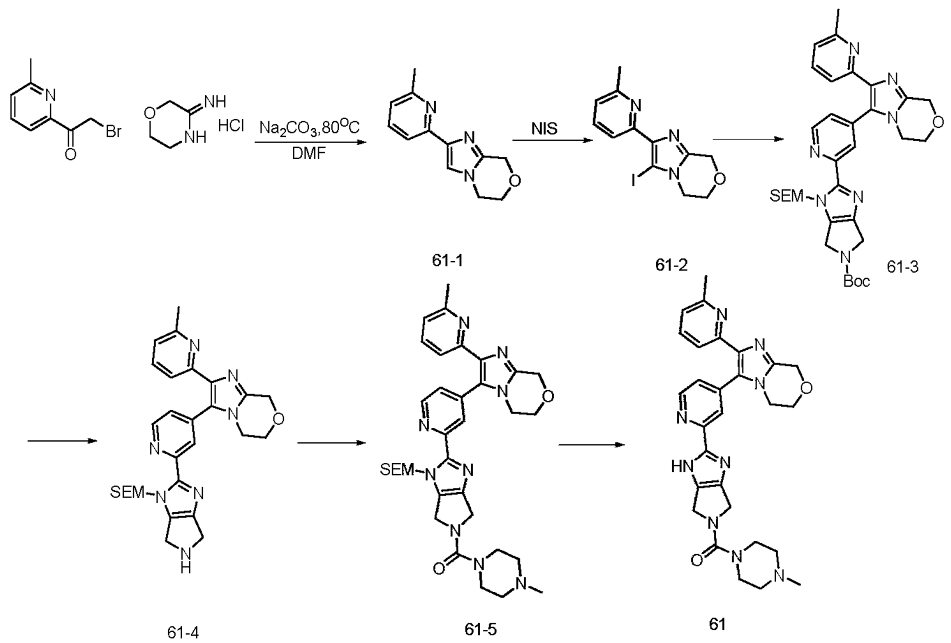


61-1 61-2 61-3

61-4 61-5 61

**Synthesis method:**

**Synthesis of intermediate 61-1: 2-(6-Methylpyridin-2-yl)-5,6-dihydro-8H-imidazo[2,1-c][1,4]oxazine**

2-Bromo-1-(6-methylpyridin-2-yl)ethan-1-one (500.0 mg, 2.3 mmol), morpholine-3-imine hydrochloride (318.0 mg, 2.3 mmol), and sodium carbonate (1.2 g, 11.7 mmol) were dissolved in 10 ml DMF. It was allowed to heat to 80°C and react for overnight. After the reaction was complete, water was added to quench the reaction. EA extraction was performed twice. The organic phases were combined, washed with saturated brine, concentrated, and purified by silica gel column chromatography to obtain intermediate 61-1, 282.0 mg, with a yield of 56.1%. LC-MS m/z (ESI) $[M+H]^+$ calculated for $C_{12}H_{14}N_3O$: 216.1; measured: 216.1.

$^1H$ NMR (400 MHz, $CDCl_3$) δ7.70 (d, J = 8.0 Hz, 1H), 7.62-7.58 (m, 2H), 7.03 (d, J = 8.0 Hz, 1H), 4.94 (s, 2H), 4.11 (s, 4H), 2.58 (s, 3H).

**Synthesis of intermediate 61-2: 3-lodo-2-(6-methylpyridin-2-yl)-5,6-dihydro-8H-imidazo[2,1-c] [1,4]oxazine**

Intermediate 61-1 (20.0 mg, 0.1 mmol) and NIS (48.2 mg, 0.3 mmol) were dissolved in 1 ml DMF and it was allowed to heat to 60°C and react for 4-hours. After the reaction was complete, water was added to quench the reaction, and EA extraction was performed twice. The organic phases were combined, washed with saturated brine, concentrated, and purified to obtain intermediate 61-2, 23.0 mg, with a yield of 72.6%. LC-MS m/z (ESI) $[M+H]^+$ calculated for $C_{12}H_{13}IN_3O$: 342.0; measured: 342.0.

$^1H$ NMR (400 MHz, $CDCl_3$) δ7.76 (d, J = 8.0 Hz, 1H), 7.63 (t, J = 8.0 Hz, 1H), 7.09 (d, J = 8.0 Hz, 1H), 4.92 (s, 2H), 4.14 (t, J = 4.0 Hz, 2H), 3.96 (t, J = 8.0 Hz, 1H), 2.64 (s, 3H).

**Synthesis of intermediate 61-3: Tert-butyl 2-(4-(2-(6-Methylpyridin-2-yl)-5,6-dihydro-8H-imidazo[2,1-c][1,4]oxazin-3-yl)pyridin-2-yl)-1-((2-(trimethylsilyl)ethoxy) methyl)-4,6-dihydropyrrolo[3,4-d]imidazol-5(1H)-carboxylate**

Tert-butyl 2-(4-Bromopyridin-2-yl)-1-((2-(trimethylsilyl)ethoxy)methyl)-4,6-dihydropyrrolo[3,4-d]imidazol-5(1H)-carboxylate (76.3 mg, 0.2 mmol) was dissolved in 10 ml, to which potassium acetate (20.1 mg, 0.2 mmol), bis(pinacolato)diboron (58.6 mg, 0.2 mmol), and $Pd(dppf)Cl_2$ (7.5 mg, 0.01 mmol) were added. Under nitrogen atmosphere, it was allowed to react at 100°C overnight. The reaction mixture was cooled to room temperature, intermediate 61-2 (35.0 mg, 0.1 mmol), potassium carbonate (28.4 mg, 0.2 mmol), 2 ml of $H_2O$, and Pd-127 (7.8 mg, 0.01 mmol) were added. Under nitrogen atomosphere, it was allowed to react at 100°C for 5 hours. The reaction mixture was cooled to room temperature, water was added, and the mixture was extracted with EA. The organic phase was washed with saturated brine, dried over anhydrous sodium sulfate, concentrated, and purified to obtain intermediate 61-3, 42.0 mg, with a yield of 65.0%. LC-MS m/z (ESI) $[M+H]^+$ calculated for $C_{33}H_{44}N_7O_4Si$: 630.3; measured: 630.3.

**Synthesis of intermediate 61-4: 2-(6-Methylpyridin-2-yl)-3-(2-(1-((2-(trimethylsilyl)ethoxy)methyl)-1,4,5,6-tetrahydropyrrolo[3,4-d]imidazol-2-yl)pyridin-4-yl)-5,6-dihydro-8H-imidazo[2,1-c][1,4]oxazine**

Intermediate 61-3 (42.0 mg, 0.1 mmol) was dissolved in 10 ml of DCM, to which zinc bromide (60.3 mg, 0.3 mmol) was added. It was allowed to react at room temperature overnight. Saturated sodium bicarbonate solution was added and stirred for 10 minutes. Then the mixture was extracted with DCM, and the organic phase was washed once with saturated brine, dried over anhydrous sodium sulfate, concentrated, to obtain the crude product intermediate 61-4, 35.0 mg, with a yield of 99.1%.

**Synthesis of intermediate 61-5: (4-Methylpiperazin-1-yl)(2-(4-(2-(6-methylpyridin-2-yl)-5,6-dihydro-8H-imidazo [2,1-c] [1,4] oxazin-3-yl)pyridin-2-yl)-1-((2-(trimethylsilyl)ethoxy)methyl)-4,6-dihydropyrrolo[3,4-d]imidazol-5(1H)-yl)ketone**

Intermediate 61-4 (35.0 mg, 0.1 mmol) was dissolved in 10 ml of DCM and cooled to 0°C. Trichloromethyl carbonate (24.5 mg, 0.1 mmol) was added, and it was allowed to react for 5 minutes. Triethylamine (66.9 mg, 0.7 mmol) was added dropwise, and it was allowed to react for 0.5 hours. N-Methylpiperidine (7.9 mg, 0.1 mmol) was then added, and the reaction mixture was allowed to warm to room temperature. After the reaction was complete, water was added to quench the reaction, and the reaction mixture was extracted with DCM. The organic layers were combined, washed with saturated brine, concentrated, and purified to obtain intermediate 61-5, 31.0 mg, with a yield of 71.5%.

**Synthesis of compound 61: (4-Methylpiperazin-1-yl)(2-(4-(2-(6-methylpyridin-2-yl)-5,6-dihydro-8H-imidazo[2,1-c][1,4]oxazin-3-yl)pyridin-2-yl)-4,6-dihydropyrrolo [3,4-d]imidazol-5(1H)-yl)ketone**

Intermediate 61-5 (31.0 mg, 0.05 mmol) was dissolved in 4 ml of methanol, to which 2 ml of concentrated hydrochloric acid was added. It was allowed to react 50°C for 2 hours. The reaction mixture was concentrated and the concentrate was dissolved in 5 ml of methanol and adjusted to basic with 0.5 ml of aqueous ammonia. The mixture was concentrated, and purified by preparative plate to yield 4.6 mg, with a yield of 18.5%. LC-MS m/z (ESI) $[M+H]_+$ calculated for $C_{28}H_{32}N_9O_2$: 526.3; measured: 526.3.

**Example 62: (4-Methylpiperazin-1-yl)(2-(4-(2-(6-methylpyridin-2-yl)-5,6-dihydro-4H-pyrrolo[1,2-b]pyrazol-3-yl) pyridin-2-yl)-4,6-dihydropyrrolo[3,4-d]imidazol-5(1H)-yl)ketone**

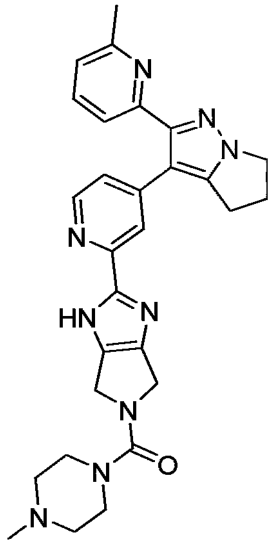

62

**Synthetic route of compound 62:**

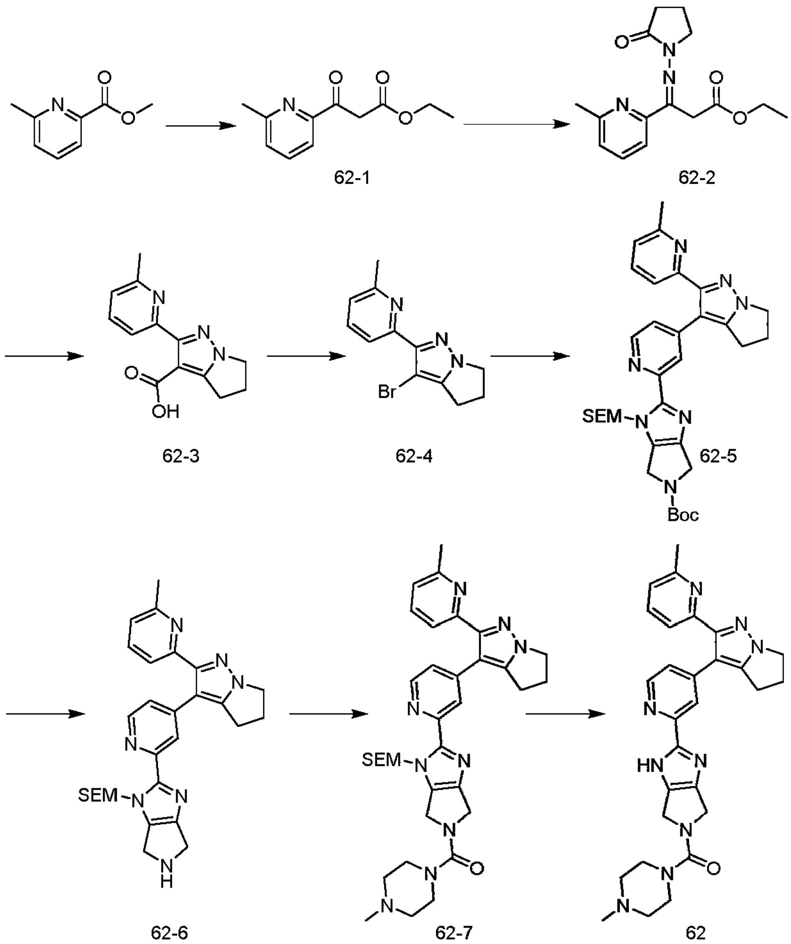

62-1 62-2

62-3 62-4 62-5

62-6 62-7 62

**Synthesis method:**

**Synthesis of intermediate 62-1: ethyl 3-(6-Methylpyridin-2-yl)-3-oxopropanate**

Ethyl acetate (874.3 mg, 9.9 mmol) was dissolved in 10 ml of toluene, to which sodium ethoxide (450.0 mg, 6.6 mmol) was added, and the mixture was stirred at room temperature for 1 hour. Methyl 6-methylpyridine-2-carboxylate (500.0 mg, 3.3 mmol) was then added, and the mixture was stirred at 95°C for 16 hours. After the reaction was complete, the reaction solution was cooled to room temperature and the pH was adjusted to 7 with acetic acid. Water was added to the reaction solution, and the mixture was extracted with ethyl acetate twice. The organic phases were combined, washed with saturated brine, dried over anhydrous sodium sulfate, and concentrated to obtain the crude intermediate 62-1, totaling 530.3 mg with a yield of 77.3%. LC-MS m/z (ESI) $[M+H]^+$ calculated for $C_{11}H_{13}NO_3$: 208.1; measured: 208.2.

**Synthesis of intermediate 62-2: ethyl 3-(6-Methylpyridin-2-yl)-3-((2-oxopyrrolidin-1-yl)imino)propanate**

Intermediate 62-1 (430.0 mg, 2.1 mmol) was dissolved in 10 ml of pyridine, to which 2-iminopyrrolidine hydrochloride (275.2 mg, 2.3 mmol) was added. It was allowed to react at room temperature for 16 hours until the reaction was complete. The reaction solution was concentrated to remove pyridine, water was added to the concentrate, and the resulting mixture was extracted with ethyl acetate twice. The organic phases were combined, washed with saturated brine, dried over anhydrous sodium sulfate, and concentrated to obtain the crude intermediate 62-2, totaling 580.1 mg with a yield of 96.6%.

**Synthesis of intermediate 62-3: 2-(6-Methylpyridin-2-yl)-5,6-dihydro-4H-pyrrolo[1,2-b]pyrazole-3-carboxylic acid**

Intermediate 62-2 (580.0 mg, 2.0 mmol) was dissolved in 10 ml of toluene, to which sodium ethoxide (272.7 mg, 4.0 mmol) was added. The mixture was stirred at 100°C for 15 hours. The reaction solution was cooled to room temperature, water was added to the reaction solution, the resulting mixture was stirred for 20 minutes and the pH was adjusted to 4 with concentrated hydrochloric acid. The mixture was extracted with dichloromethane: isopropanol (10:1) twice. The organic phases were combined, washed with saturated brine, dried over anhydrous sodium sulfate, and concentrated to obtain the crude intermediate 62-3, totaling 340.2 mg with a yield of 69.4%. LC-MS m/z (ESI) $[M+H]^+$ calculated for $C_{13}H_{13}N_3O_2$: 244.1; measured: 244.1.

**Synthesis of intermediate 62-4: 3-Bromo-2-(6-methylpyridin-2-yl)-5,6-dihydro-4H-pyrrolo[1,2-b]pyrazole**

Intermediate 62-3 (340.0 mg, 1.4 mmol) was dissolved in 10 ml of DMF, to which NBS (272.1 mg, 1.5 mmol) was added. The mixture was stirred at room temperature for 16 hours. After the reaction was complete, water was added to the reaction solution, and the resulting mixture was extracted with ethyl acetate twice. The organic phases were combined, washed with saturated brine, dried over anhydrous sodium sulfate, concentrated and purified by silica gel column chromatography to obtain intermediate 62-4, totaling 350.1 mg with a yield of 90.0%. LC-MS m/z (ESI) $[M+H]^+$ calculated for $C_{12}H_{12}BrN_3$: 278.0; measured: 278.0.

**Synthesis of intermediate 62-5: tert-butyl 2-(4-(2-(6-Methylpyridin-2-yl)-5,6-dihydro-4H-pyrrolo[1,2-b]pyrazol-3-yl)pyridin-2-yl)-1-((2-(trimethylsilyl)ethoxy) methyl)-4,6-dihydroxypyrido[3,4-d]imidazole-5(1H)-carboxylate**

Tert-butyl 2-(4-bromopyridin-2-yl)-1-((2-(trimethylsilyl)ethoxy)methyl)-4,6-dihydroxypyrido[3,4-d]imidazole-5(1H)-carboxylate (106.9 mg, 0.2 mmol) and bis(pinacolato)diboron (109.6 mg, 0.4 mmol) were dissolved in 8 ml of dioxane, to which potassium acetate (42.3 mg, 0.4 mmol) and Pd(dppf)$Cl_2$ (16.0 mg, 0.02 mmol) were added. The mixture was purged with nitrogen, and it was allowed to react at 90°C for 16 hours. The reaction solution was cooled to room temperature, to which intermediate 62-4 (60.0 mg, 0.2 mmol) dissolved in 4 ml of dioxane and potassium carbonate (89.2 mg, 0.6 mmol) were added along with additional Pd(dppf)$Cl_2$ (16.0 mg, 0.02 mmol), 3 ml of water was added. The mixture was purged with nitrogen, and it was allowed to react at 100°C for 20 hours. The reaction solution was filtered, water was added to the filtrate, and the resulting mixture was extracted with ethyl acetate twice. The organic phases were combined, washed with saturated brine, dried over anhydrous sodium sulfate, concentrated, and purified by silica gel column chromatography to obtain intermediate 62-5, totaling 70.6 mg with a yield of 52.9%. LC-MS m/z (ESI) $[M+H]^+$ calculated for $C_{33}H_{43}N_7O_3Si$: 614.3; measured: 614.5.

**Synthesis of intermediate 62-6: 2-(4-(2-(6-Methylpyridin-2-yl)-5,6-dihydro-4H-pyrrolo[1,2-b]pyrazol-3-yl)pyridin-2-yl)-1-((2-(trimethylsilyl)ethoxy) methyl)-1,4,5,6-tetrahydropyrrolo [3,4-d]imidazole**

Intermediate 62-5 (26.0 mg, 0.04 mmol) was dissolved in 5 ml of dichloromethane, to which zinc bromide (38.0

mg, 0.17 mmol) was added. The mixture was stirred at room temperature for 16 hours. The reaction solution was quenched with ammonia water, and extracted with dichloromethane, and the organic phase was washed with saturated brine, dried over anhydrous sodium sulfate, and concentrated, to obtain the crude intermediate 62-6, totaling 16.3 mg with a yield of 73.5%.

**Synthesis of intermediate 62-7: (4-Methylpiperazin-1-yl)(2-(4-(2-(6-methylpyridin-2-yl)-5,6-dihydro-4H-pyrrolo [1,2-b]pyrazol-3-yl)pyridin-2-yl)-1-((2-(trimethylsilyl)ethoxy)methyl)-4,6-dihydropyrrolo [3,4-d]imidazole-5(1H)-yl)ketone**

N-Methylpiperazine (6.2 mg, 0.06 mmol) was dissolved in 5 ml of dichloromethane and cooled to 0°C, to which triphosgene (18.4 mg, 0.06 mmol) was added, and then triethylamine (62.9 mg, 0.6 mmol) was slowly added. It was allowed to react for 5 minutes. The solution of intermediate 62-6 (16.3 mg, 0.03 mmol) in dichloromethane was added to the reaction mixture, and the temperature was allowed to rise to room temperature with stirring. After the reaction was complete, water was added to quench the reaction, and the mixture was extracted with dichloromethane. The organic phases were combined, washed with saturated brine, concentrated, and purified by silica gel column chromatography to obtain intermediate 62-7, totaling 15.2 mg with a yield of 37.7%.

**Synthesis of compound 62: (4-Methylpiperazin-1-yl)(2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazole-5(1H)-yl)ketone**

Intermediate 62-7 (15.2 mg, 0.02 mmol) was dissolved in 4 ml of methanol, to which 2 ml of concentrated hydrochloric acid was added. It was allowed to react at 45°C for 1 hour. The reaction mixture was concentrated, and the concentrate was dissolved in 5 ml of methanol, adjusted to basic pH with 0.5 ml of ammonia water, concentrated, and purified by silica gel plate to obtain the final product, totaling 5.3 mg with a yield of 44.4%. LC-MS m/z (ESI) $[M+H]^+$ calculated for $C_{28}H_{31}N_9O$: 510.3; measured: 510.3.

<u>**Example 63: 2-(4-(2-(6-Methylpyridin-2-yl)-6,7-dihydro-5H-pyrrolo[1,2-a]imidazol-3-yl)pyridin-2-yl)-5-(methylsulfonyl)-4,5,6,7-tetrahydro-3H-imidazo[4,5-c]pyridine**</u>

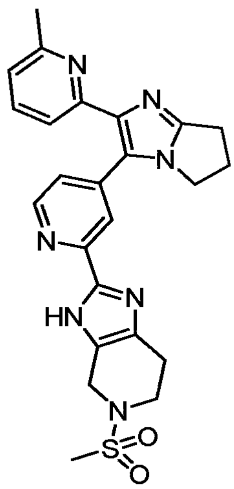

63

**Synthetic route for compound 63:**

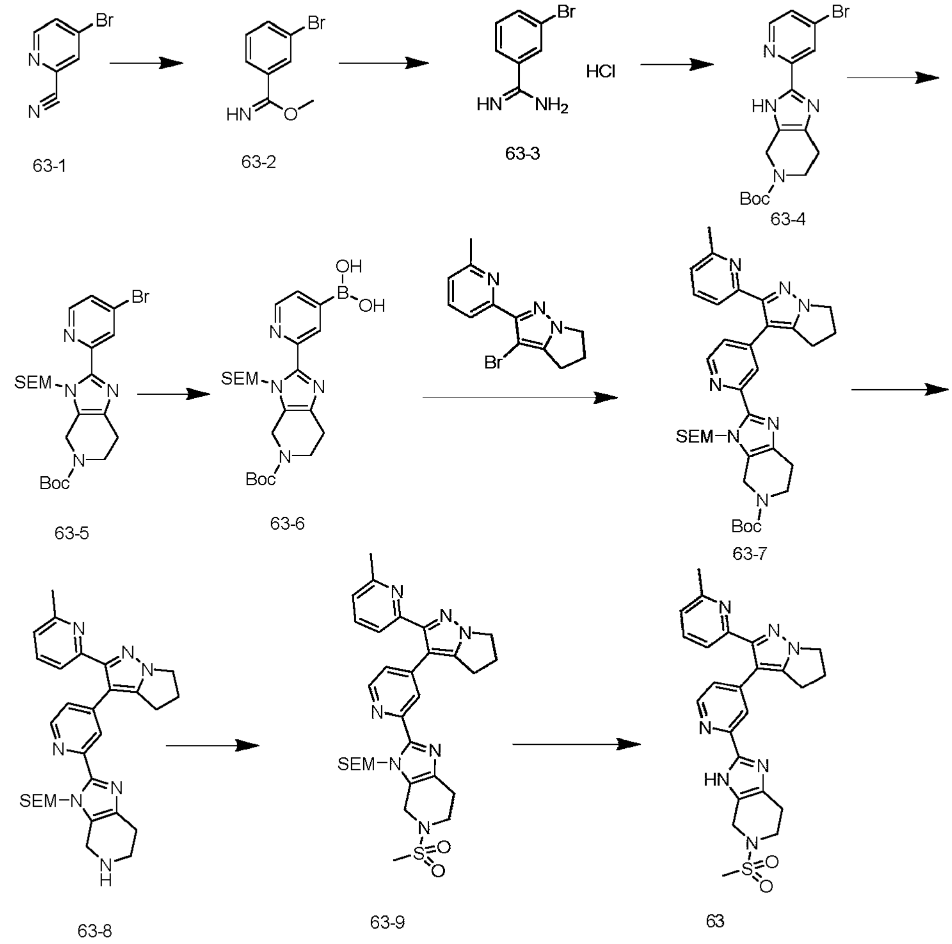

63-1 63-2 63-3 63-4 63-5 63-6 63-7 63-8 63-9 63

**Synthesis method:**

**Synthesis of Intermediate 63-2: 3-Bromo-biphenyl-4-yl methyl ether**

Intermediate 63-1 (5.0 g, 27.5 mmol) was dissolved in 50 ml of methanol, to which sodium methoxide (3.0 g, 55.4 mmol) was added. It was allowed to react at room temperature for 16 hours. The reaction solution was concentrated to remove the methanol, water was added to the concentrate, and the resulting mixture was extracted with ethyl acetate twice. The organic phases were combined, washed with saturated brine, dried over anhydrous sodium sulfate, and concentrated to yield the crude intermediate 63-2 (4.8 g) with a yield of 81.4%.

**Synthesis of Intermediate 63-3, 3-Bromo-1H-benzo[d]imidazole hydrochloride**

Intermediate 63-2 (4.8 g, 22.3 mmol) was dissolved in 40 ml of ethanol, to which $NH_4Cl$ (3.6 g, 67.2 mmol) was added, and it was allowed to react at 80°C for 2 hours. Upon completion of the reaction, the mixture was concentrated to obtain intermediate 63-3 (3.2 g) with a yield of 57.1%.

**Synthesis of intermediate 63-4: tert-butyl 2-(4-bromopyridin-2-yl)-3,4,6,7-tetrahydro-5H-imidazolo[4,5-c]pyridine-5-carboxylate:**

Intermediate 63-3 (3.2 g, 13.6 mmol) was dissolved in 40 ml of toluene, to which N-Boc-3-bromo-4-oxopiperidine (7.5 g, 27.2 mmol) and sodium bicarbonate (3.4 g, 40.8 mmol) were added. The reaction mixture was heated to 80°C and it was allowed to react overnight. After cooling, the reaction was monitored by TLC, and upon complete consumption of the starting material, water was added to quench the reaction. The mixture was extracted with ethyl acetate (EA) twice, and the combined organic phases were washed with saturated brine, dried over anhydrous sodium sulfate, and purified by silica gel column chromatography to yield intermediate 63-4, 2.9 g, with a yield of 56.6%. LC-MS m/z (ESI) $[M+H]^+$ calculated for $C_{16}H_{20}BrN_4O_2$: 379.1; and measured: 379.0.

**Synthesis of intermediate 63-5: tert-butyl 2-(4-bromopyridin-2-yl)-3-((2-(trimethylsilyl)ethoxy)methyl)-3,4,6,7-tetrahydro-5H-imidazolo[4,5-c]pyridine-5-**carboxylate

Intermediate 63-4 (2.9 g, 7.6 mmol) was dissolved in 30 ml of THF and cooled to 0°C, to which sodium hydride (60%) (0.6 g, 15.2 mmol) was added. The mixture was stirred for 0.5 hours and then SEMCl (1.9 g, 11.4 mmol) was added. After the addition, the reaction mixture was allowed to warm to room temperature with stirring until the reaction was complete. The reaction was then quenched with water, and extracted with EA, and the organic phase was washed with saturated brine, dried over anhydrous sodium sulfate, concentrated, and purified by silica gel column chromatography to obtain intermediate 63-5, 2.7 g, with a yield of 68.8%. LC-MS m/z (ESI) $[M+H]^+$ calculated for $C_{22}H_{34}BrN_4O_3Si$: 509.2 and measured: 509.1.

**Synthesis of intermediate 63-6: (2-(5-(tert-butoxycarbonyl)-3-((2-(trimethylsilyl)ethoxy)methyl)-4,5,6,7-tetrahydro-3H-imidazolo[4,5-c]pyridin-2-yl)pyridin-4-yl)boronic acid**

Intermediate 63-5 (2.7 g, 5.2 mmol) was dissolved in 30 ml of DME, to which bis(pinacolato)diboron (2.6 g, 10.4 mmol) and nickel dibromide dimethoxyethane (393.3 mg, 0.5 mmol) were added. The system was purged with nitrogen, potassium acetate (1.6 g, 15.6 mmol) was added, and the system was purged again with nitrogen. The reaction mixture was then heated to 90°C and it was allowed to react overnight. After it was cooled to room temperature, the reaction mixture was concentrated and purified by silica gel column chromatography to yield intermediate 63-6 (1.2 g) with a yield of 48.1%. LC-MS m/z (ESI) $[M+H]^+$ calculated for $C_{22}H_{36}BN_4O_5Si$: 475.3; and measured: 475.3.

**Synthesis of intermediate 63-7: tert-butyl 2-(4-(2-(6-methylpyridin-2-yl)-6,7-dihydro-5H-pyrrolo[1,2-a]imidazol-3-yl)pyridin-2-yl)-3-((2-(trimethylsilyl)ethoxy) methyl)-3,4,6,7-tetrahydro-5H-imidazolo[4,5-c]pyridine-5-carboxylate**

3-Bromo-2-(6-methylpyridin-2-yl)-6,7-dihydro-5H-pyrrolo[1,2-a]imidazole (100.0 mg, 0.4 mmol) was dissolved in a mixture of 10 ml DME and 1 ml water, to which intermediate 63-6 (256.5 mg, 0.5 mmol), sodium carbonate (76.2 mg, 0.7 mmol), and nickel dibromide dimethoxyethane (27.2 mg, 0.04 mmol) were added. The system was purged with nitrogen and it was allowed to react at 80°C overnight. After the reaction, water was added, the mixture was extracted, washed with saturated brine, dried over anhydrous sodium sulfate, concentrated, and purified by silica gel column chromatography to obtain intermediate 63-7 (89.4 mg) with a yield of 39.6%. LC-MS m/z (ESI) $[M+H]^+$ calculated for $C_{34}H_{46}N_7O_3Si$: 628.3 and measured: 628.3.

**Synthesis of intermediate 63-8, 2-(4-(2-(6-methylpyridin-2-yl)-6,7-dihydro-5H-pyrrolo[1,2-a]imidazol-3-yl)pyridin-2-yl)-3-((2-(trimethylsilyl)ethoxy) methyl)-4,5,6,7-tetrahydro-3H-imidazolo[4,5-c]pyridine**

Intermediate 63-7 (89.4 mg, 0.2 mmol) was dissolved in 10 ml of DCM, to which zinc bromide (128.3 mg, 0.6 mmol) was added. The system was purged with nitrogen and it was allowed to react at room temperature overnight. After the reaction, 0.5 ml of aqueous ammonia was added to the reaction mixture, followed by the addition of water. The mixture was then extracted with DCM twice, and the organic phases were combined, washed once each with saturated sodium bicarbonate solution and saturated brine, concentrated, to obtain the crude product intermediate 63-8 (75.0 mg) with a yield of 100%.

**Synthesis of intermediate 63-9: (2-(4-(2-(6-methylpyridin-2-yl)-6,7-dihydro-5H-pyrrolo[1,2-a]imidazol-3-yl)pyridin-2-yl)-5-(methylsulfonyl)-3-((2-(trimethylsilyl)ethoxy)methyl)-4,5,6,7-tetrahydro-3H-imidazolo[4,5-c]pyridine**

Intermediate 63-8 (32.0 mg, 0.1 mmol) was dissolved in 5 ml of DCM, to which triethylamine (20.2 mg, 0.2 mmol) and methanesulfonyl chloride (8.3 mg, 0.1 mmol) were added. It was allowed to react at room temperature for 1 hour. Water was then added to the reaction solution, and the resulting mixture was extracted with DCM, washed with saturated brine, dried over anhydrous sodium sulfate, and concentrated to obtain the crude product intermediate 63-9, 33.0 mg, with a yield of 89.9%.

**Synthesis of compound 63: 2-(4-(2-(6-methylpyridin-2-yl)-6,7-dihydro-5H-pyrrolo[1,2-a]imidazol-3-yl)pyridin-2-yl)-5-(methylsulfonyl)-4,5,6,7-tetrahydro-3H-imidazolo[4,5-c]pyridine**

Intermediate 63-9 (33.0 mg, 0.1 mmol) was dissolved in 2 ml of methanol, to which 1 ml of concentrated hydrochloric acid was added. It was allowed to react at 50°C for 2 hours. The reaction mixture was concentrated, and the concentrate was dissolved in 5 ml of methanol, to which 0.5 ml of aqueous ammonia was added, and the resulting mixture

was concentrated and purified to obtain the final product, compound 63, in 5.2 mg with a yield of 20.1%. LC-MS m/z (ESI) $[M+H]^+$ calculated for $C_{24}H_{26}N_7O_2S$: 476.2 and measured: 476.2.

**Example 64**

The following example was prepared according to the synthetic route of Example 1 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 64 | 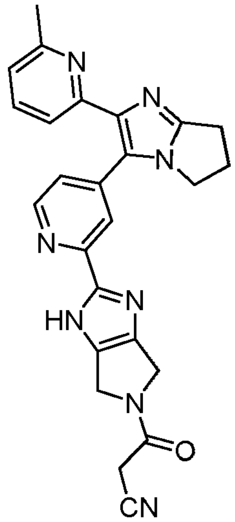 64 3-(2-(4-(2-(6-Methylpyridin-2-yl)-6,7-dihydro-5H-pyrrolo[1,2-a]imidazol-3-yl)pyridin-2-yl)-4,6-dihydropyrrolo[3,4-d]imidazol-5(1H)-yl)-3-oxopropanenitrile | | 451.2 | 451.2 |

**Examples 65-74**

The following examples were prepared according to the synthetic route of Example 1 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 65 | 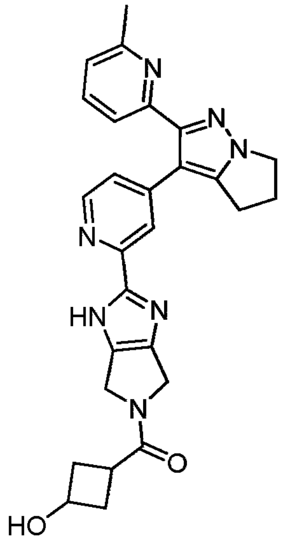 65 (3-Hydroxycyclobutyl)(2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazol-5(1H)-yl) ketone | | 482.2 | 482.3 |
| 66 | 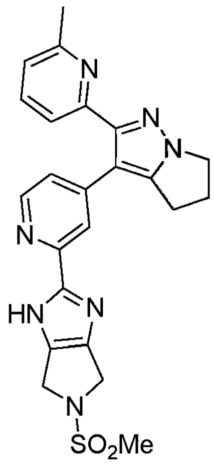 66 2-(4-(2-(6-Methylpyridin-2-yl)-5,6-dihydro-4H-pyrrolo [1,2-b]pyrazol-3-yl)pyridin-2-yl)-5-(methylsulfonyl)-1,4,5,6-tetrahydropyrrolo[3,4-d]imidazole | | 462.2 | 462.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 67 | 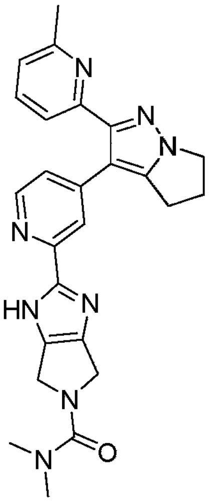 67 N,N-Dimethyl-2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazole-5(1H)-carboxamide | | 455.2 | 455.2 |
| 68 | 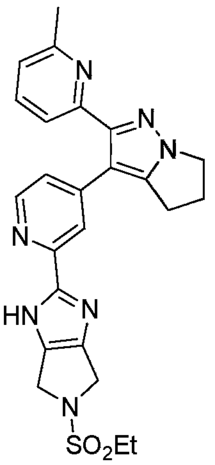 68 5-(Ethylsulfonyl)-2-(4-(2-(6-methylpyridin-2-yl)-5,6-dihydro-4H-pyrrolo[1,2-b]pyrazol-3-yl)pyridin-2-yl)-1,4,5,6-tetrahydropyrrolo[3,4-d]imidazole | | 476.2 | 476.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 69 | 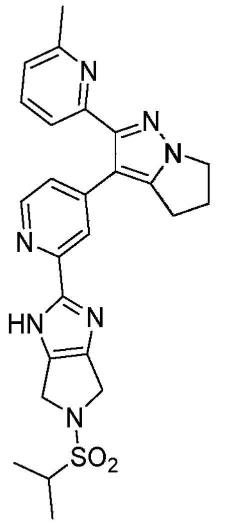 69 5-(Isopropylsulfonyl)-2-(4-(2-(6-methylpyridin-2-yl)-5,6-dihydro-4H-pyrrolo[1,2-b]pyrazol-3-yl)pyridin-2-yl)-1,4,5,6-tetrahydropyrrolo[3,4-d]imidazole | | 490.2 | 490.2 |
| 70 | 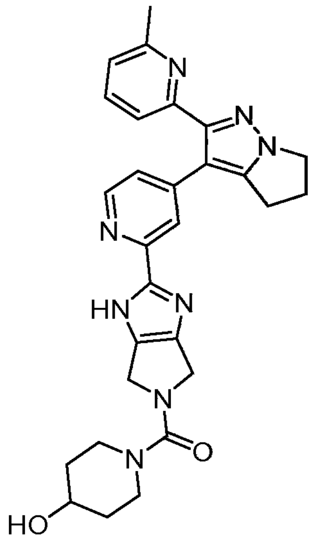 70 (4-Hydroxypiperidin-1-yl)(2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazol-5(1H)-yl) ketone | | 511.3 | 511.3 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 71 | 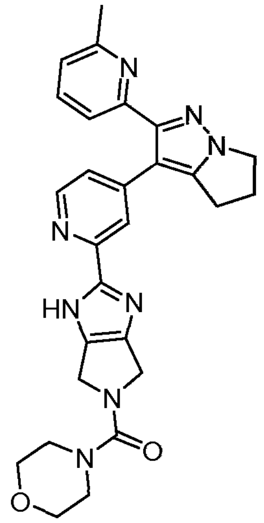 71 (2-(4-(2-(6-Methylpyridin-2-yl)-5,6-dihydro-4H-pyrrolo[1,2-b]pyrazol-3-yl)pyridin-2-yl)-4,6-dihydro-pyrrolo [3,4-d]imidazol-5(1H)-yl)(morpholinyl)ketone | | 497.2 | 497.3 |
| 72 | 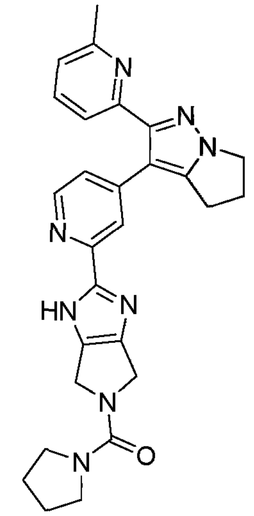 72 (2-(4-(2-(6-Methylpyridin-2-yl)-5,6-dihydro-4H-pyrrolo[1,2-b]pyrazol-3-yl)pyridin-2-yl)-4,6-dihydropyrrolo [3,4-d]imidazol-5(1H)-yl)(pyrrolidin-1-yl)ketone | | 481.2 | 481.2 |

(continued)

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 73 | 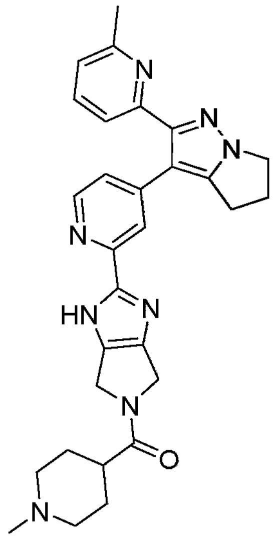 73 (1-Methylpiperidin-4-yl)(2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazol-5(1H)-yl) ketone | | 509.3 | 509.3 |
| 74 | 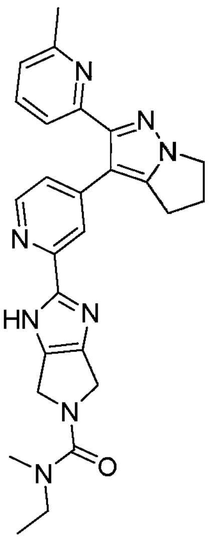 74 N-Ethyl-N-methyl-2-(4-(2-(6-methylpyridin-2-yl)-5,6-dihydro-4H-pyrrolo[1,2-b]pyrazol-3-yl)pyridin-2-yl)-4,6-dihydropyrrolo[3,4-d]imidazole-5(1H)-carboxamide | | 469.2 | 469.2 |

**Examples 75-76**

The following examples were prepared according to the synthetic route of Example 63 using appropriate starting materials.

| Examples | Structural Formula and Name of Compounds | $^1$HNMR(400 MHz) | Theoretical LC-MS $(M+1)^+$ | Measured LC-MS $(M+1)^+$ |
|---|---|---|---|---|
| 75 | 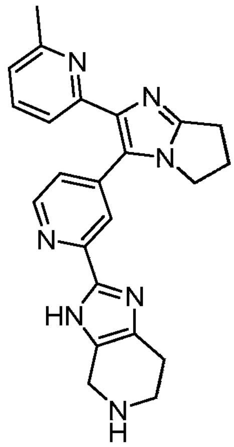 75 2-(4-(2-(6-Methylpyridin-2-yl)-6,7-dihydro-5H-pyrrolo [1,2-a]imidazol-3-yl)pyridin-2-yl)-4,5,6,7-tetrahydro-3H-imidazolo[4,5-c]pyridine | | 398.2 | 398.2 |
| 76 | 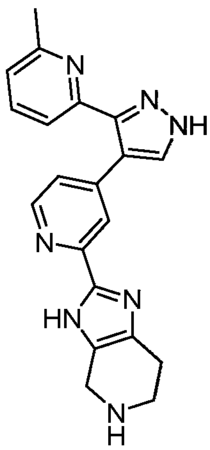 76 2-(4-(3-(6-Methylpyridin-2-yl)-1H-pyrazol-4-yl)pyridin-2-yl)-4,5,6,7-tetrahydro-3H-imidazolo[4,5-c]pyridine | | 358.2 | 358.2 |

**Example 77: Determination of Activity of compounds**

**1. Materials and Reagents**

| Materials and reagents | Manufacturer | Cat. No. |
|---|---|---|
| Hepes | Santa Cruz | SC-29097A |
| Brij35 | Millipore | 1018940100 |
| EGTA | Sigma | E3889 |
| ADP-Glo Kinase Assay | Promega | V9101 |
| TGFβR1 | ThermoFisher | 1795372T |
| MgCl2 | Sigma | M1028 |

(continued)

| Materials and reagents | Manufacturer | Cat. No. |
|---|---|---|
| ATP | Promega | V915B |
| DTT | Solarbio | D8220 |
| DMSO | Sigma | D4540 |
| TGFβR1 tide | GenScript | C4256GD280-1 |
| LY364947 (Positive control) | MCE | HY-13226 |
| **Instruments and Equipment** | **Manufacturer** | **Cat. No. or Model No.** |
| 384-well plate, white, low volume, round-bottom | Greiner | 784075 |
| 96-well polypropylene plate | Nunc | 249944 |
| Microplate low-speed centrifuge | Xiang Yi | TDZ5-WS |
| High-throughput Screening (HTS) Multifunctional Microplate Reader | Biotek | Synergy 4 |

**2. Experimental method**

2.1 Preparation of 1X kinase reaction buffer:

| Name | Stock Concentration | Volume | Final Concentration |
|---|---|---|---|
| Hepes | 1 M (20X) | 12500μL | 50 mM |
| $MgCl_2$ | 1 M (100X) | 2500μL | 10 mM |
| Brij 35 | 30% | 25μL | 0.01% |
| EGTA | 1M (1000X) | 250μL | 1mM |
| DTT | 1M (500X) | 500μL | 2 mM |
| ddH2O | | 235000μL | |

2.2 Rection conditions of Kinase

| | Stock solution | Working concentration |
|---|---|---|
| TGFβR1 | 310ng/ul | 0.5ng/ul |
| ATP | 10mM | 5uM |
| TGFβR1 tide | 10ug/ul | 0.1ug/ul |

Activity Assay Procedure:

The principle of the activity assay was that TGFβR1 phosphorylates the substrate TGFβR1tide, consuming ATP in the process. This experiment employed the ADP-Glo method to detect kinase activity and determine the IC50 value, which was used to evaluate the inhibitory ability of the test compounds against human TGFβR1. In the experiment, DSM was used as a negative control, and LY364947 was used as a positive control. The specific experimental steps were as follows. Compounds were diluted 4-fold in DMSO in a dilution plate, with the final starting concentration of the compounds being 10μM, across 10 concentration gradient points. The compounds were then further diluted 50-fold into the kinase reaction buffer and incubated on a shaker for 20 minutes. Kinase was formulated with an enzyme reaction buffer. 2 μl kinase was added to each well of the reaction plate. 1 μl of the compound diluted in buffer was added to each well, and then the plate was sealed with a sealing membrane and centrifuged at 1000g for 30 seconds, leaving it at room temperature for 10 minutes. Solutions of TGFβR1tide and ATP were prepared with the kinase reaction buffer, and 2 μL of the TGFβR1tide/ATP solution was added to the reaction plate. The plate was sealed with a sealing film and centrifuged

at 1000g for 30 seconds. It was allowed to react at room temperature for 60 minutes. Subsequently, 4 μL of ADP-Glo was transferred to a 384-well reaction plate and centrifuged at 1000 rpm/min for 1 minute, followed by incubation at 25°C for 40 minutes. Then, 8 μL of Detection solution was transferred to the 384-well reaction plate and centrifuged at 1000 rpm/min for 1 minute, after which it was incubated at 25°C for 40 minutes. The RLU (Relative Luminescence Unit) signal was read using a BMG microplate reader, which was used to characterize the degree of kinase activity.

3. Data analysis

3.1 The inhibition rate was calculated as follows: inhibition rate of compound (% inh) = 100% - (compound - positive control) / (negative control - positive control) * 100%.

3.2 Calculation of IC50 and plotting of inhibition curves for the compounds.

The IC50 (half inhibition concentration) of a compound was obtained using the following non-linear fitting equation, wherein data analysis was performed using Graphpad 7.0 software.

$$Y=Bottom + (Top-Bottom)/(1+10^{((LogIC50-X)*Hill\ Slope)})$$

X: log value of compound concentration; and Y: inhibition rate (% inhibition)

3.3 Report on the second inspection.

3.3.1 After an experimenter completed the report writing, another experimenter conducted a second inspection to ensure the accuracy of data analysis.

3.3.2 Data were exported from BMG and analyzed manually.

3.3.2.1 The ratios were converted to inhibition rates and IC50 was calculated by Prism GraphPad 7.0 from the inhibition rates.

3.3.2.2 IC50 was calculated again by the ratios to verify accuracy of the results.

3.4 Quality control

Z-factor > 0.5; and S/B >2
Positive control IC50 was within 3 times of the average value

4. Activity Results

| **Compounds** | TGFβR1 $IC_{50}$ (nM) |
|---|---|
| LY364947 | 20.9 |
| **1** | 21 |
| **2** | 23 |
| **3** | 7.4 |
| **4** | 3.8 |
| **6** | 22 |
| **7** | 1.1 |
| **9** | 5.4 |
| **10** | 2.2 |
| **11** | 11.6 |

(continued)

| **Compounds** | TGFβR1 $IC_{50}$ (nM) |
|---|---|
| **12** | 4.3 |
| **13** | 11.9 |
| **14** | 7.9 |
| **15** | 16 |
| **22** | 7.5 |
| **23** | 0.9 |
| **25** | 25 |
| **26** | 4.1 |
| **27** | 4.3 |
| **28** | 19 |
| **29** | 0.18 |
| **30** | 5.7 |
| **32** | 2.3 |
| **33** | 1.7 |
| **34** | 1.9 |
| **37** | 6.5 |
| **38** | 4.4 |
| **39** | 2.0 |
| **40** | 1.6 |
| **42** | 27 |
| **43** | 2.7 |
| **44** | 6.2 |
| **45** | 19 |
| **46** | 1.7 |
| **47** | 3.4 |
| **48** | 1.6 |
| **49** | 3.0 |
| **50** | 2.2 |
| **51** | 5.6 |
| **52** | 2.1 |
| **53** | 2.5 |
| **54** | 1.4 |
| **55** | 5.0 |
| **56** | 23 |
| **57** | 9.9 |
| **58** | 1.8 |
| **59** | 9.2 |
| **60** | 1.8 |
| **61** | 6.9 |

(continued)

| Compounds | TGFβR1 $IC_{50}$ (nM) |
|---|---|
| **62** | 0.9 |
| **63** | 1.6 |
| **64** | 7.3 |
| **65** | 0.70 |
| **66** | 4.0 |
| **67** | 2.0 |
| **68** | 8.1 |
| **69** | 1.3 |
| **70** | 2.3 |
| **71** | 0.67 |
| **72** | 2.0 |
| **73** | 2.8 |
| **74** | 1.7 |
| **75** | 5.6 |
| **76** | 13 |

It was shown by the above results that the compounds of the present application exhibited equivalent or superior inhibition of TGFβR1 compared to the positive control LY364947, and therefore can be used for the treatment of diseases or conditions related to TGF-β, particularly those associated with TGF-β1.

## Claims

**1.** A compound of Formula (I)

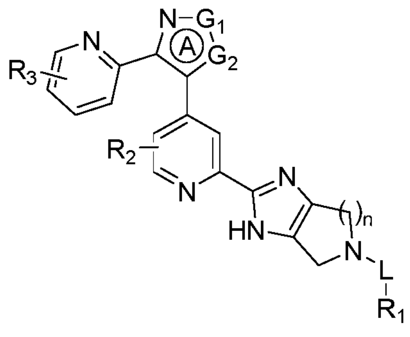
(I)

or an isotopically labeled compound thereof, or an optical isomer thereof, a geometric isomer thereof, a tautomer thereof or a mixture of the optical isomer, geometric isomer, and tautomer, or a pharmaceutically acceptable salt thereof,
wherein

n is 1;
L is (C=O), (O=S=O), ((C=O)- $CH_2$), $CH_2$ or a bond; and
$R_1$ is selected from H, halogen, -OH, $-NO_2$, -CN, $-SF_5$, -SH, -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicyclic aryl, 7-11 membered bicyclic heteroaryl, -$C_{1-4}$ alkyl-($C_{3-7}$ alicyclic group), -$C_{1-4}$ alkyl-(3-10 membered heteroalicyclic

group), $-C_{1-4}$ alkyl-($C_{6-12}$ bicyclic alicyclic group), $-C_{1-4}$ alkyl-(6-12 membered bicyclic heteroalicyclic group), $-C_{1-4}$ alkyl-($C_{8-15}$ tricyclic alicyclic group), $-C_{1-4}$ alkyl-(8-15 membered tricyclic heteroalicyclic group), $-C_{1-4}$ alkyl-($C_{5-8}$ aryl), $-C_{1-4}$ alkyl-(5-10 membered heteroaryl), $-N(R_{10})(R_{11})$, $-N(R_{10})(C(=O)R_{11})$, $-N(R_{10})(C(=O)-OR_{11})$, $-N(R_{12})(C(=O)-N(R_{10})(R_{11}))$, $-C(=O)-N(R_{10})(R_{11})$, $-C(=O)-R_{12}$, $-C(=O)-OR_{12}$, $-OC(=O)R_{12}$, $-N(R_{10})(S(=O)_2R_{11})$, $-S(=O)_2-N(R_{10})(R_{11})$, $-SR_{12}$, and $-OR_{12}$, wherein the $-S-C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicycloaryl, 7-11 membered bicyclic heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{6-12}$ bicyclic alicyclic group), $-C_{1-4}$ alkyl-(6-12 membered bicyclic heteroalicyclic group), $-C_{1-4}$ alkyl-($C_{8-15}$ tricyclic alicyclic group), $-C_{1-4}$ alkyl-(8-15 membered tricyclic heteroalicyclic group), $-C_{1-4}$ alkyl-($C_{5-8}$ aryl) and $-C_{1-4}$ alkyl-(5-10 membered heteroaryl) are each optionally substituted with 0, 1, 2, 3 or 4 $R^{1a}$; and $R^{1a}$ is independently selected from halogen, -OH, $-NO_2$, -CN, $-SF_5$, -SH, $-S-C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10 membered heteroalicyclic group, $C_{5-8}$ aryl, 5-7 membered heteroaryl, $-N(R_{13})(R_{14})$, $-N(R_{13})(C(=O)R_{14})$, $-N(R_{13})(C(=O)-OR_{14})$, $-N(R_{15})(C(=O)-N(R_{13})(R_{14}))$, $-C(=O)-N(R_{13})(R_{14})$, $-C(=O)-R_{15}$, $-C(=O)-OR_{15}$, $-OC(=O)R_{15}$, $-N(R_{13})(S(=O)_2R_{14})$, $-S(=O)_2-N(R_{13})(R_{14})$, $-SR_{15}$, and $-OR_{15}$; and

$R_{10}$, $R_{11}$, $R_{12}$, $R_{13}$, $R_{14}$ and $R_{15}$, at each occurrence, are each independently selected from H, $C_{1-6}$ alkyl, $C_{1-6}$ haloalkyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{6-12}$ bicyclic alicyclic group), $-C_{1-4}$ alkyl-(6-12 membered bicyclic heteroalicyclic group), $-C_{1-4}$ alkyl-($C_{8-15}$ tricyclic alicyclic group), $-C_{1-4}$ alkyl-(8-15 membered tricyclic heteroalicyclic group), $-C_{1-4}$ alkyl-($C_{5-8}$ aryl), and $-C_{1-4}$ alkyl-(5-10 membered heteroaryl), wherein each option listed in the group is optionally substituted with 0, 1, 2, 3, or 4 substituents each independently selected from a group consisting of: halogen, -OH, $-NH_2$, $-NH(CH_3)$, $-N(CH_3)_2$, -CN, $-NO_2$, $-SF_5$, -SH, $-S-C_{1-4}$ alkyl, oxo, $C_{1-4}$ alkyl, $C_{2-6}$ alkenyl, $C_{2-6}$ alkynyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C(=O)-N(C_{1-4}$ alkyl$)_2$, $C_{1-4}$ haloalkyl, $C_{1-4}$ alkoxy and $C_{1-4}$ haloalkoxy; or $R_{10}$, $R_{11}$, and the atom(s) attached thereto together form a 3-14-membered ring; or $R_{13}$, $R_{14}$, and the atom(s) attached thereto together form a 3-14-membered ring;

1, 2, or 3 $R_2$(s) are present in formula (I), and each $R_2$ is H;

1, 2, or 3 $R_3$(s) are present in formula (I), and each $R_3$ is methyl;

and

the ring A is an aromatic ring containing nitrogen and satisfies: 1) G1 = $NR_4$, G2 = $CR_5$; or 2) G1 = $CR_4$, G2 = $NR_5$; or 3) G1 = $CR_4$, G2 = O or S; wherein $R_4$ and $R_5$ are each independently selected from H, halogen, -CN, -OH, $-NO_2$, $C_{1-6}$ alkyl, $C_{1-6}$ haloalkyl, $C_{1-6}$ alkoxy; or $R_4$ and $R_5$ together with the atom(s) to which they are attached form a 3-8 membered ring.

**2.** The compound as claimed in claim 1, or an isotopically labeled compound thereof, or an optical isomer thereof, a geometric isomer thereof, a tautomer thereof or a mixture of the optical isomer, geometric isomer, and tautomer, or a pharmaceutically acceptable salt thereof, wherein the compound of formula (I) has one of the following structures:

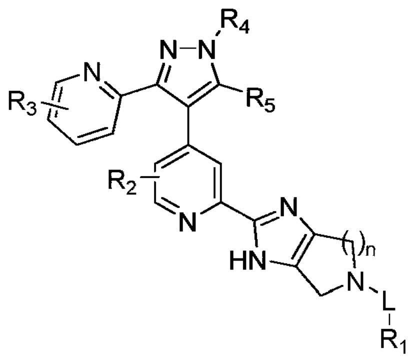
(I-1)

in which, n, L, $R_1$, $R_2$, $R_3$, $R_4$, and $R_5$ are as defined in claim 1;

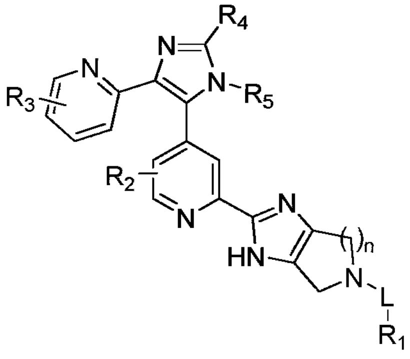

(I-2)

in which, n, L, $R_1$, $R_2$, $R_3$, $R_4$, and $R_5$ are as defined in claim 1;

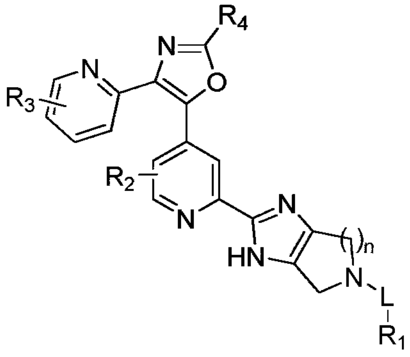

(I-3)

in which, n, L, $R_1$, $R_2$, $R_3$, and $R_4$ are as defined in claim 1;

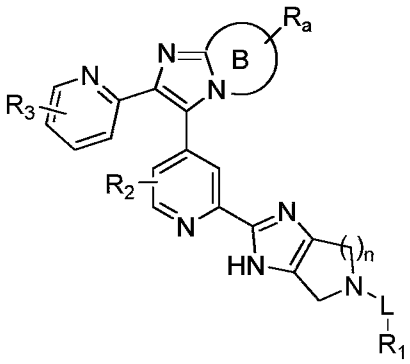

(I-4)

in which, n, L, $R_1$, $R_2$, and $R_3$ are as defined in claim 1; ring B is a 3 to 8 membered ring containing 1 N atom, and $R_a$ is selected from H, halogen, -CN, -OH, $-NO_2$, $C_{1-6}$ alkyl, $C_{1-6}$ haloalkyl, and $C_{1-6}$ alkoxy.

**3.** The compound as claimed in any one of claims 1 to 2, or an isotopically labeled compound thereof, or an optical isomer thereof, a geometric isomer thereof, a tautomer thereof or a mixture of the optical isomer, geometric isomer, and tautomer, or a pharmaceutically acceptable salt thereof, wherein $R_1$ is selected from H, halogen, -OH, $-NO_2$, -CN, $-SF_5$, -SH, $-S-C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{5-8}$ aryl), - $C_{1-4}$ alkyl-(5-10 membered heteroaryl), and $-N(R_{10})(R_{11})$, wherein the $-S-C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{5-8}$ aryl), and $-C_{1-4}$ alkyl-(5-10 membered heteroaryl) are each optionally substituted with 0, 1, 2, 3, or 4 $R^{1a}$ groups, and $R^{1a}$ is independently selected from halogen, -OH, $-NO_2$, -CN, oxo, $C_{1-4}$ alkyl, $C_{1-4}$ haloalkyl, $C_{1-4}$ alkoxy, $C_{1-4}$ haloalkoxy, $C_{3-7}$ alicyclic group, 3-10 membered heteroalicyclic group, $C_{5-8}$ aryl, 5-7 membered heteroaryl; and

$R_{10}$, $R_{11}$, and $R_{12}$ are each independently selected from: H, $C_{1-6}$ alkyl, $C_{1-6}$ haloalkyl, $C_{3-7}$ alicyclic group, and 3-10 membered heteroalicyclic group, wherein each option in the group is optionally substituted with 0, 1, 2, 3, or 4 substituents independently selected from the group consisting of halogen, -OH, $-NH_2$, oxo, $C_{1-4}$ alkyl, $C_{1-4}$ hydroxyalkyl, $C_{1-4}$ haloalkyl, $C_{1-4}$ alkoxy, and $C_{1-4}$ haloalkoxy; or $R_{10}$, $R_{11}$, and the atoms to which they are attached together form a 3-8 membered ring.

**4.** The compound as claimed in any one of claims 1 to 3, or an isotopically labeled compound thereof, or an optical isomer thereof, a geometric isomer thereof, a tautomer thereof or a mixture of the optical isomer, geometric isomer, and tautomer, or a pharmaceutically acceptable salt thereof, wherein $R_1$ is selected from H, halogen, -OH, methyl, ethyl, propyl, butyl, pentyl, hexyl, methoxy, ethoxy, propoxy, butoxy, pentoxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl, wherein the methyl, ethyl, propyl, butyl, pentyl, hexyl, methoxy, ethoxy, propoxy, butoxy, pentoxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl are each optionally substituted with 1 or 2 $R^{1a}$, and $R^{1a}$ is independently selected from halogen, - OH, $-NO_2$, -CN, oxo, $C_{1-4}$ alkyl, $C_{1-a}$ haloalkyl, and $C_{1-4}$ alkoxy; preferably $R_1$ is selected from H, methyl, methylpiperazinyl, pyrrolidinyl, methylpiperazinyl, hydroxycyclobutyl, methylpyrazolyl, hydroxyethyl, hydroxypropyl, methylpiperidinyl, morpholinyl, and hydroxypyrrolidinyl.

**5.** The compound as claimed in any one of claims 1 to 4, or an isotopically labeled compound thereof, or an optical isomer thereof, a geometric isomer thereof, a tautomer thereof or a mixture of the optical isomer, geometric isomer, and tautomer, or a pharmaceutically acceptable salt thereof, wherein the compound is selected from compounds 1-4, 6-7, 9-15, 22-23, 25-34, and 37-76:

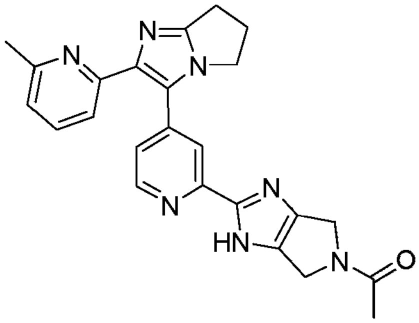

1 ,

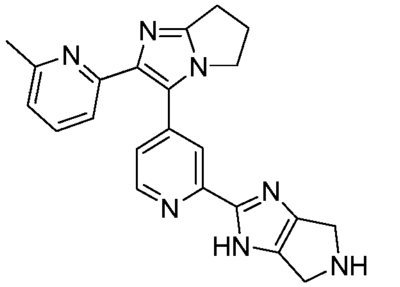

2 ,

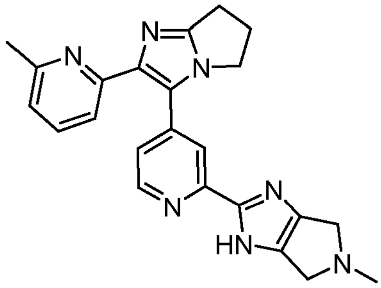

3 ,

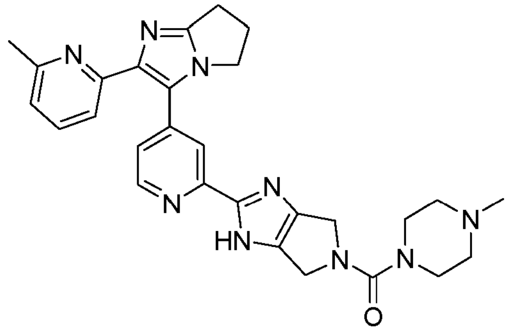

4 ,

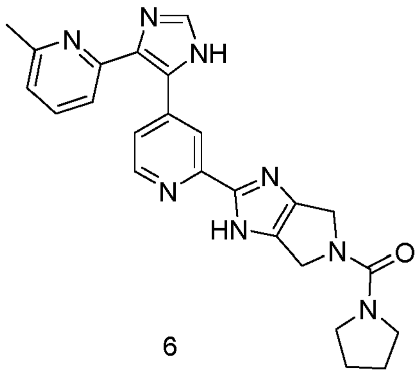

6 ,

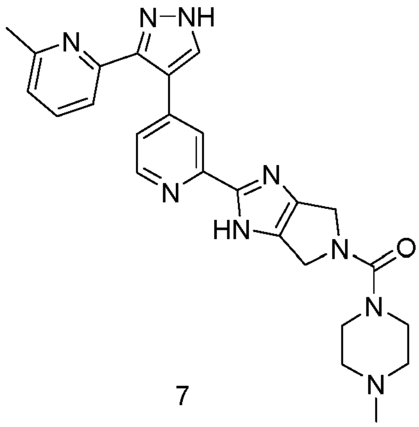

7 ,

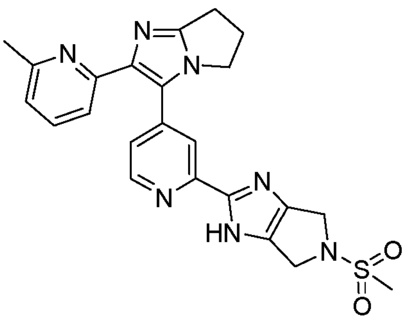

9,

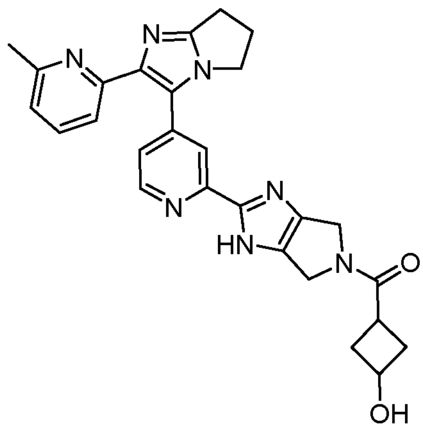

10,

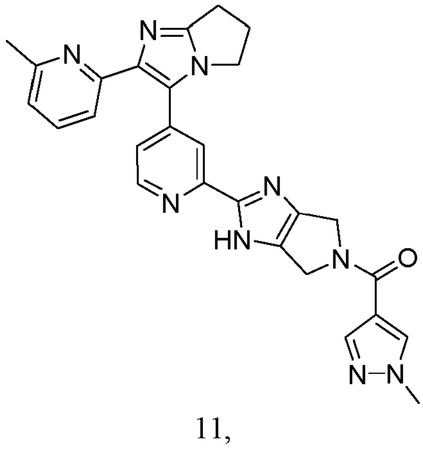

11,

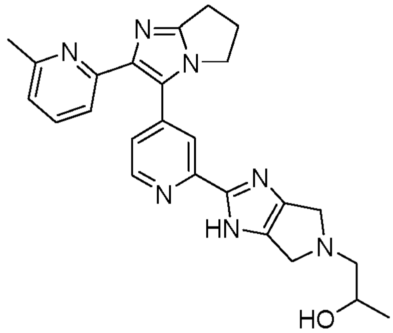

12,

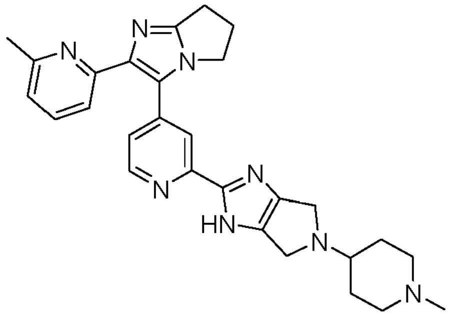

13,

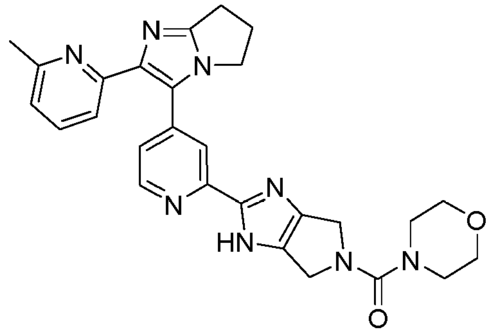

14,

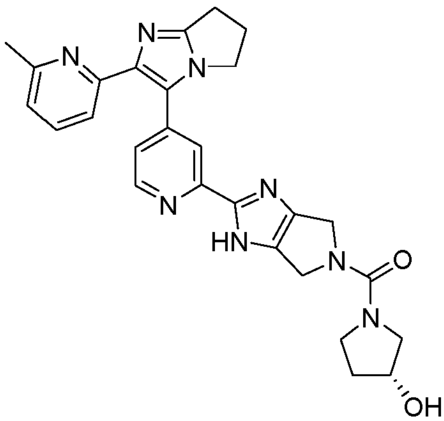

15,

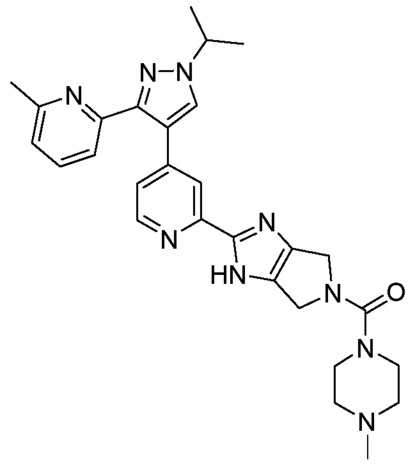

22,

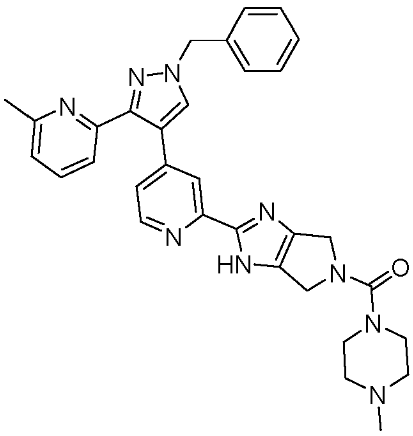

23,

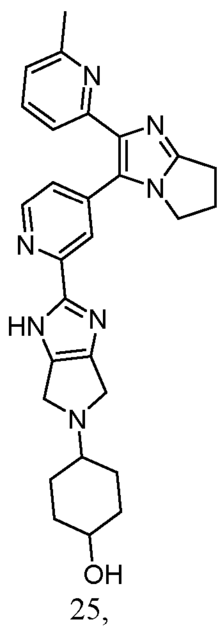

25,

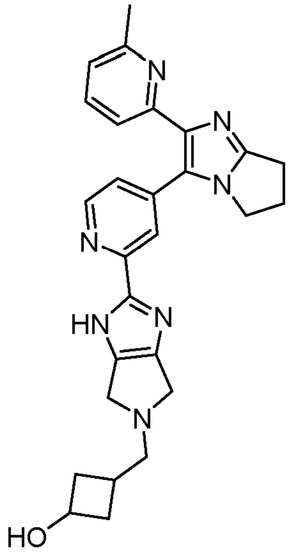
26,

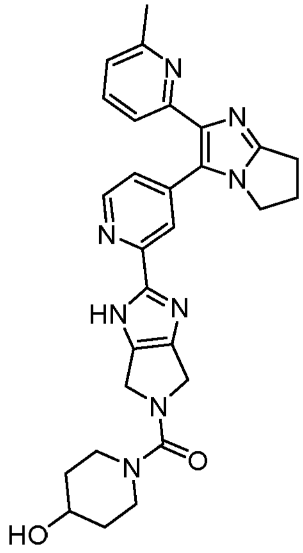
27,

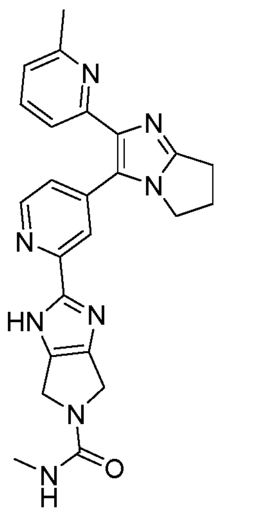
28,

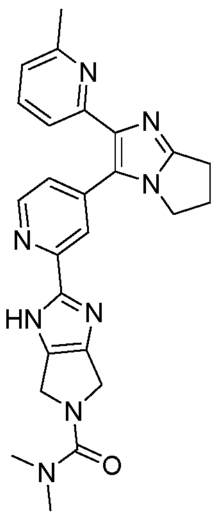
29,

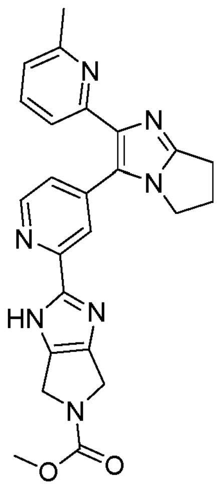
30,

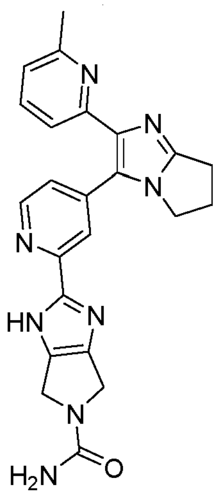
31,

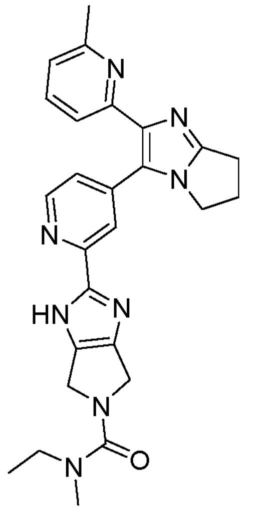

32,

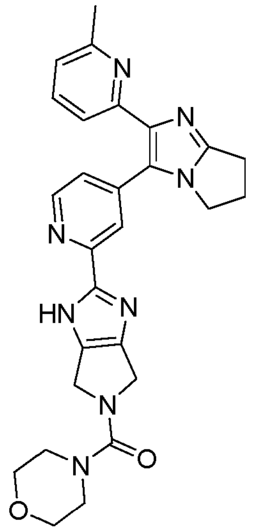

33,

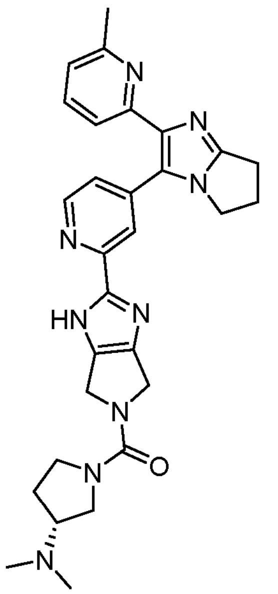

34,

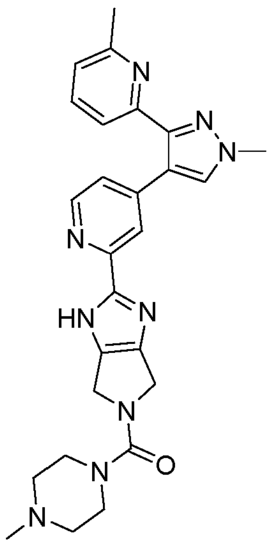

37,

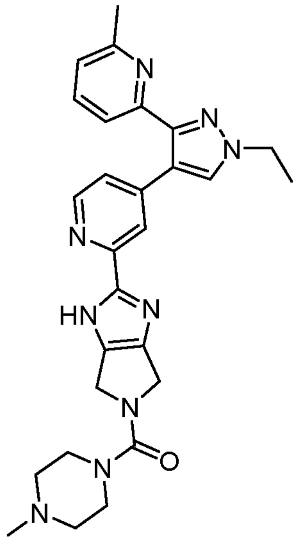

38,

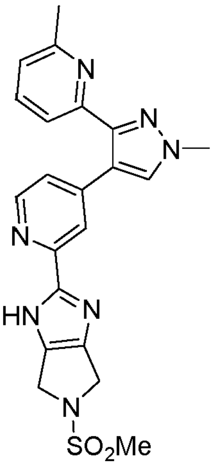

39,

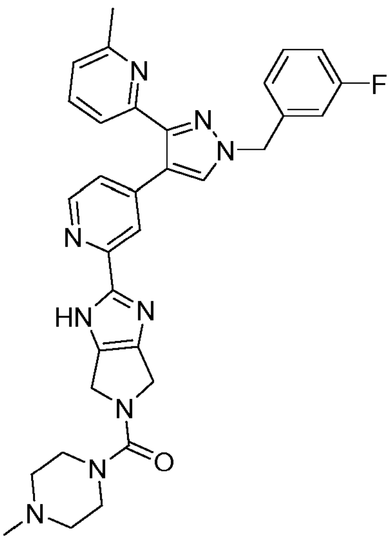

40,

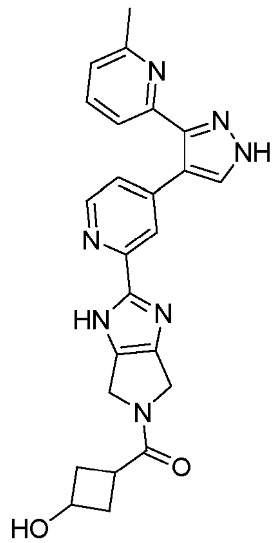

41,

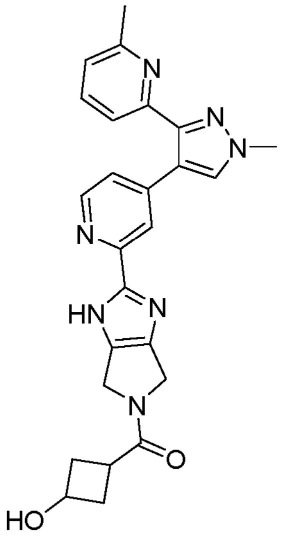

42,

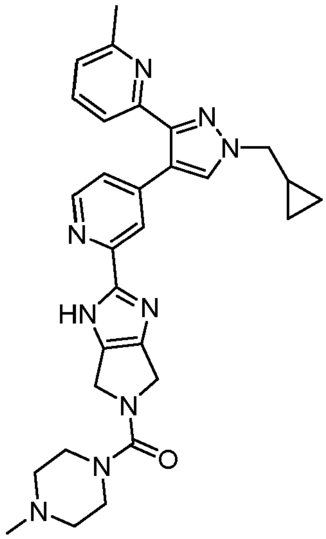

43,

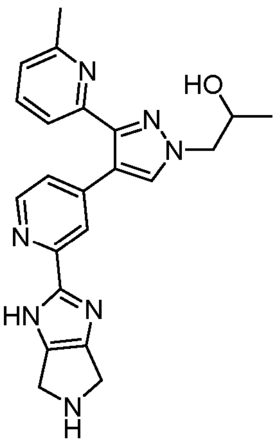

44,

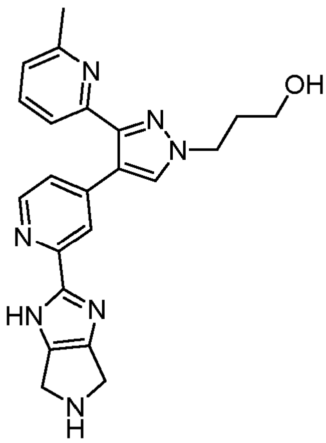

45,

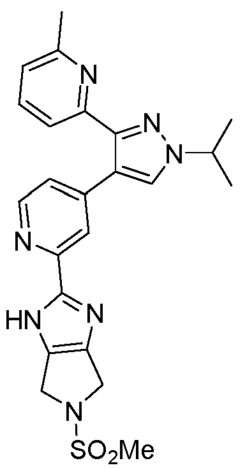

46,

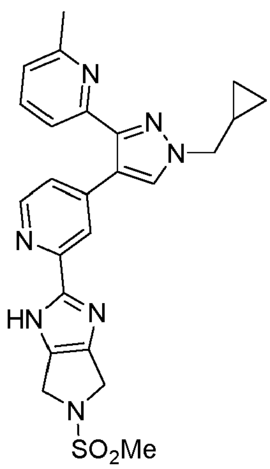

47,

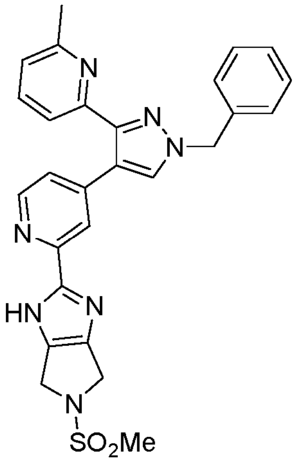

48,

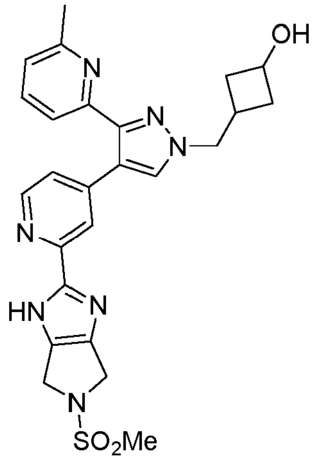

49,

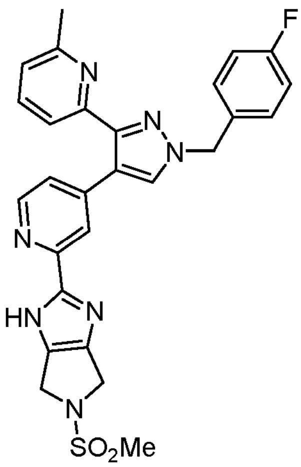

50,

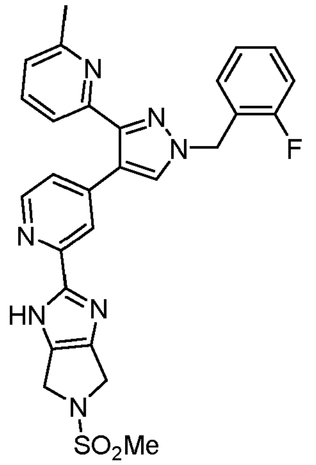

51,

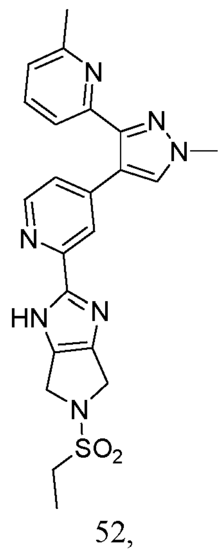

52,

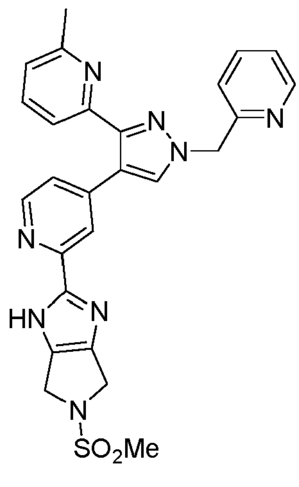

53,

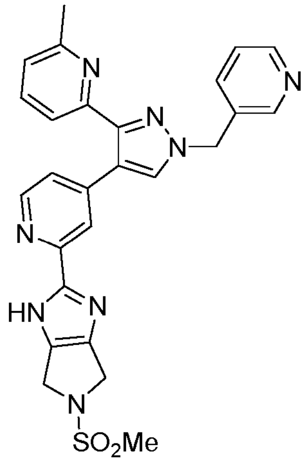

54,

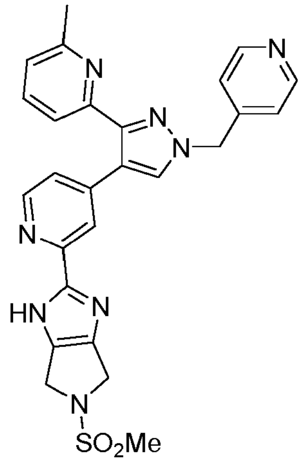

55,

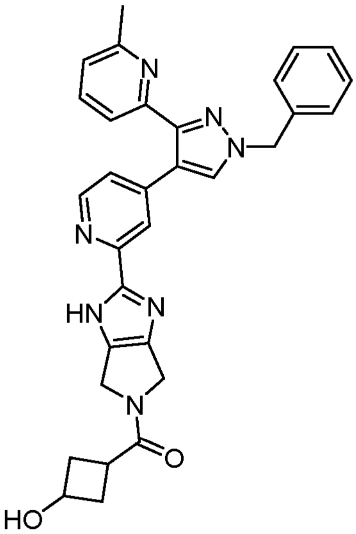

56,

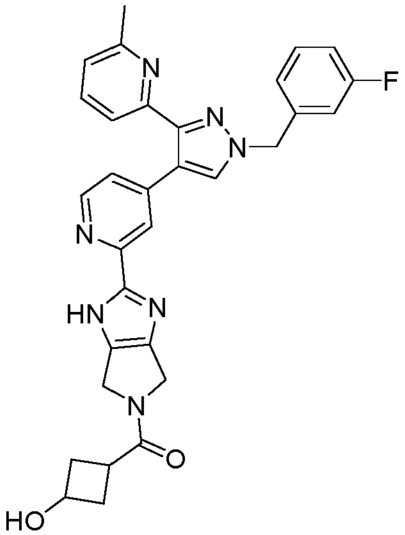

57

,

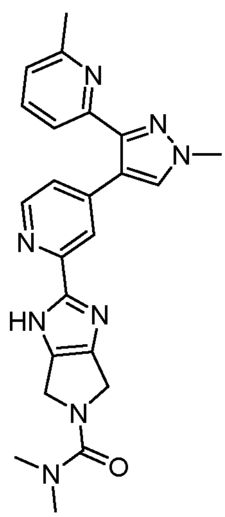

58,

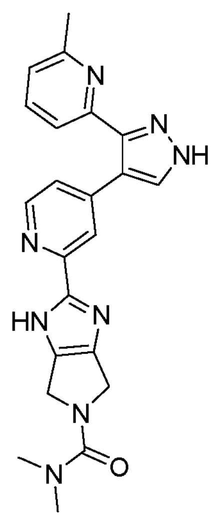

59,

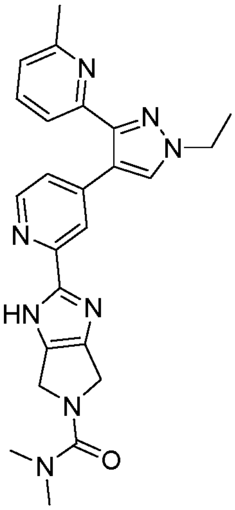
60,

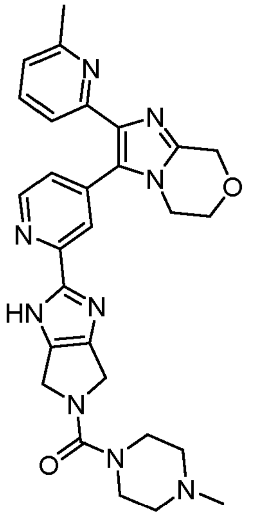
61
,

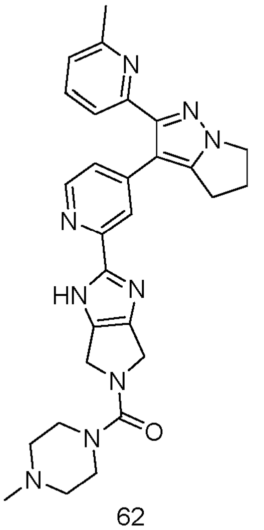
62
,

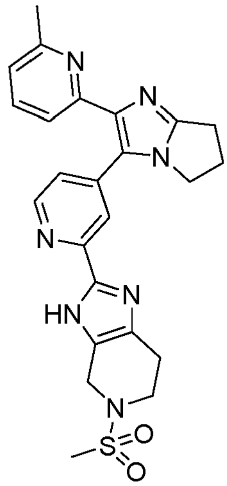
63
,

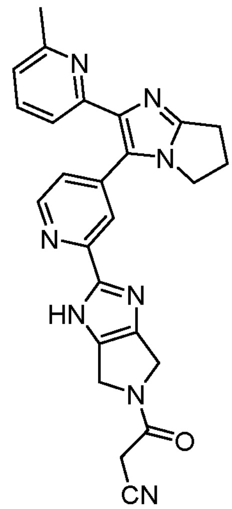
64,

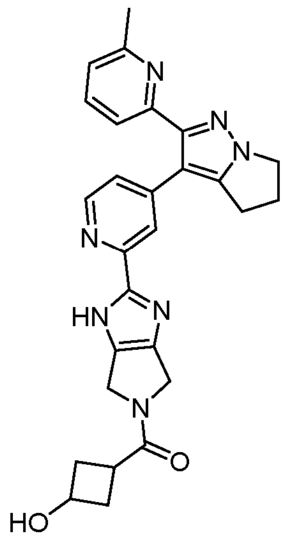
65,

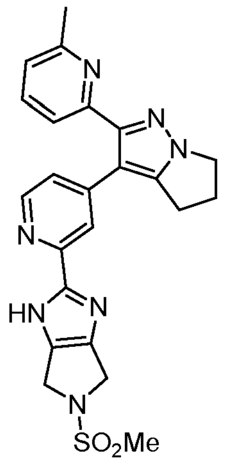
66,

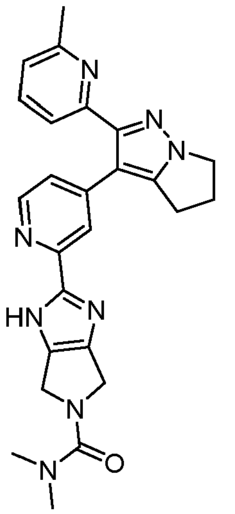
67,

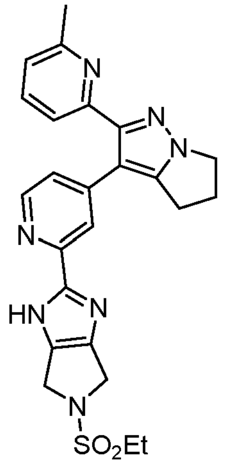
68,

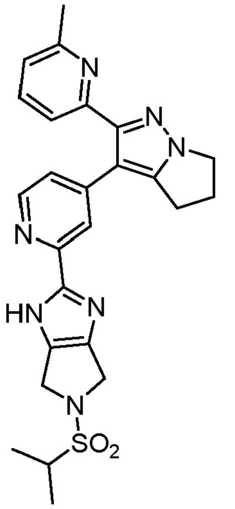
69,

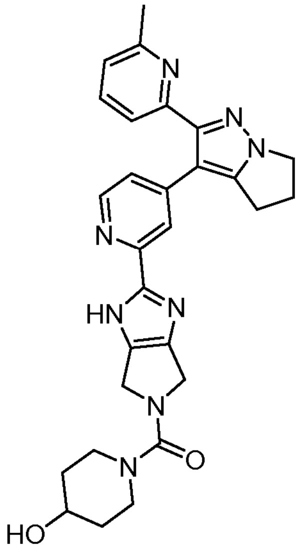

70,

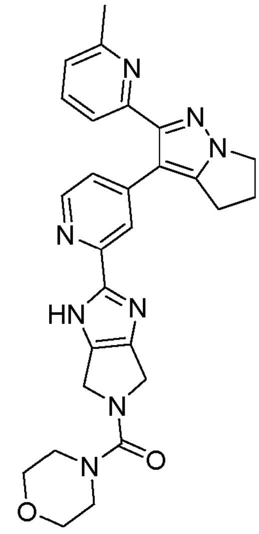

71,

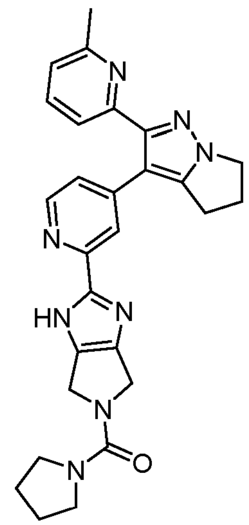

72,

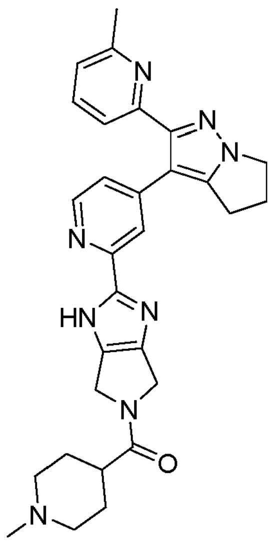

73,

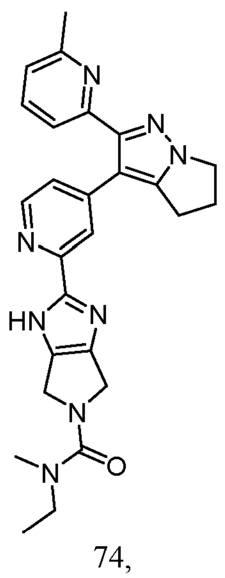

74,

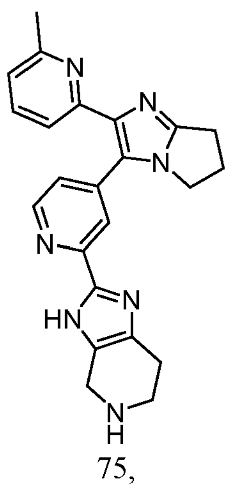

75,

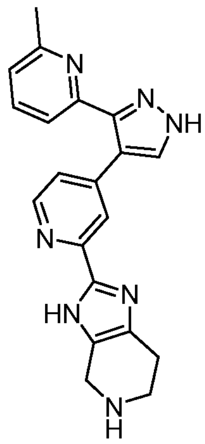

76.

6. A pharmaceutical composition comprising the compound as claimed in any one of claims 1 to 5, or an isotopically labeled compound thereof, or an optical isomer thereof, a geometric isomer thereof, a tautomer thereof or a mixture of various isomers, or a pharmaceutically acceptable salt thereof, and one or more pharmaceutically acceptable carriers, adjuvants, or excipients.

7. The compound as claimed in any one of claims 1 to 5, or an isotopically labeled compound thereof, or an optical isomer thereof, a geometric isomer thereof, a tautomer thereof or a mixture of various isomers, or a pharmaceutically acceptable salt thereof for use as a medicament for the treatment of diseases or conditions associated with TGF-β, preferably TGF-β1, wherein the diseases or conditions associated with TGF-β are selected from cancer, viral infections, chronic glomerulonephritis, acute glomerulonephritis, diabetic nephropathy, osteoporosis, arthritis, wound healing, scarring, ulcers, corneal trauma, heart valve stenosis, congestive cardiac necrosis, neurological damage, Alzheimer's disease, peritoneal or subcutaneous adhesions, atherosclerosis, skin fibrosis and skin aging due to fat loss, skeletal or chondrocyte disorders, hypophosphatemia disorders, and organ fibrosis diseases, preferably, the diseases or conditions associated with TGF-β are selected from hepatocellular carcinoma, breast cancer, bladder cancer, colorectal cancer, melanoma, mesothelioma, lung cancer, prostate cancer, pancreatic cancer, testicular cancer, thyroid cancer, squamous cell carcinoma, glioblastoma, neuroblastoma, cervical cancer, rhabdomyosarcoma, renal fibrosis, liver fibrosis, pulmonary fibrosis, skin scarring, skin fibrosis and skin aging due to fat loss.

8. The pharmaceutical composition as claimed in claim 6 for use as a medicament for the treatment of diseases or conditions associated with TGF-β, preferably TGF-β1, wherein the diseases or conditions associated with TGF-β are selected from cancer, viral infections, chronic glomerulonephritis, acute glomerulonephritis, diabetic nephropathy, osteoporosis, arthritis, wound healing, scarring, ulcers, corneal trauma, heart valve stenosis, congestive cardiac necrosis, neurological damage, Alzheimer's disease, peritoneal or subcutaneous adhesions, atherosclerosis, skin fibrosis and skin aging due to fat loss, skeletal or chondrocyte disorders, hypophosphatemia disorders, and organ fibrosis diseases, preferably, the diseases or conditions associated with TGF-β are selected from hepatocellular carcinoma, breast cancer, bladder cancer, colorectal cancer, melanoma, mesothelioma, lung cancer, prostate cancer, pancreatic cancer, testicular cancer, thyroid cancer, squamous cell carcinoma, glioblastoma, neuroblastoma, cervical cancer, rhabdomyosarcoma, renal fibrosis, liver fibrosis, pulmonary fibrosis, skin scarring, skin fibrosis and skin aging due to fat loss.

## Patentansprüche

1. Verbindung der Formel (I):

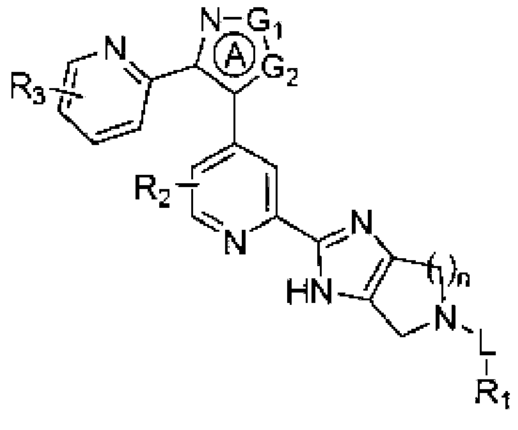

(I)

oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon,

wobei

n gleich 1 ist; und

L für (C=O), (O=S=O), ((C=O)-$CH_2$), $CH_2$ oder eine Bindung steht; und

$R_1$ ausgewählt ist aus H, Halogen, -OH, -$NO_2$, -CN, -$SF_5$, -SH, -S-$C_{1-4}$-Alkyl, $C_{1-6}$-Alkyl, $C_{1-6}$-Haloalkyl, $C_{1-6}$-Alkoxy, $C_{1-6}$-Haloalkoxy, $C_{2-8}$-Alkenyl, $C_{2-8}$-Alkinyl, $C_{3-7}$-alicyclische Gruppe, 3- bis 10-gliedrige heteroalicyclische Gruppe, $C_{6-12}$-bicyclische alicyclische Gruppe, 6- bis 12-gliedrige bicyclische heteroalicyclische Gruppe, $C_{8-15}$-tricyclische alicyclische Gruppe, 8- bis 15-gliedrige tricyclische heteroalicyclische Gruppe, $C_{5-8}$-Aryl, 5- bis 10-gliedriges Heteroaryl, $C_{7-11}$-bicyclisches Aryl, 7- bis 11-gliedriges bicyclisches Heteroaryl, - $C_{1-4}$-Alkyl-($C_{3-7}$-alicyclische Gruppe), -$C_{1-4}$-Alkyl-(3- bis 10-gliedrige heteroalicyclische Gruppe), -$C_{1-4}$-Alkyl-($C_{6-12}$-bicyclische alicyclische Gruppe), -$C_{1-4}$-Alkyl-(6- bis 12-gliedrige bicyclische heteroalicyclische Gruppe), -$C_{1-4}$-Alkyl-($C_{8-15}$-tricyclische alicyclische Gruppe), - $C_{1-4}$-Alkyl-(8- bis 15-gliedrige tricyclische heteroalicyclische Gruppe), -$C_{1-4}$-Alkyl-($C_{5-8}$-Aryl), -$C_{1-4}$-Alkyl-(5- bis 10-gliedriges Heteroaryl), -N($R_{10}$)($R_{11}$), -N($R_{10}$)(C(=O)$R_{11}$), - N($R_{10}$)(C=O)-O$R_{11}$), -N($R_{12}$)(C(=O)-N($R_{10}$)($R_{11}$)), -C(=O)-N($R_{10}$)($R_{11}$), -C(=O)-$R_{12}$, -C(=O)-O$R_{12}$, -OC(=O)$R_{12}$, -N($R_{10}$)(S(=O)$_2$$R_{11}$), -S(=O)$_2$-N($R_{10}$)($R_{11}$), -S$R_{12}$ und -O$R_{12}$,

wobei die Reste -S-$C_{1-4}$-Alkyl, $C_{1-6}$-Alkyl, $C_{1-6}$-Haloalkyl, $C_{1-6}$-Alkoxy, $C_{1-6}$-Haloalkoxy, $C_{2-8}$-Alkenyl, $C_{2-8}$-Alkinyl, $C_{3-7}$-alicyclische Gruppe, 3- bis 10-gliedrige heteroalicyclische Gruppe, $C_{6-12}$-bicyclische alicyclische Gruppe, 6- bis 12-gliedrige bicyclische heteroalicyclische Gruppe, $C_{8-15}$-tricyclische alicyclische Gruppe, 8- bis 15-gliedrige tricyclische heteroalicyclische Gruppe, $C_{5-8}$-Aryl, 5- bis 10-gliedriges Heteroaryl, $C_{7-11}$-bicyclisches Aryl, 7- bis 11-gliedriges bicyclisches Heteroaryl, -$C_{1-4}$-Alkyl-($C_{3-7}$-alicyclische Gruppe), -$C_{1-4}$-Alkyl-(3- bis 10-gliedrige heteroalicyclische Gruppe), -$C_{1-4}$-Alkyl-($C_{6-12}$-bicyclische alicyclische Gruppe), -$C_{1-4}$-Alkyl-(6- bis 12-gliedrige bicyclische heteroalicyclische Gruppe), -$C_{1-4}$-Alkyl-($C_{8-15}$-tricyclische alicyclische Gruppe), -$C_{1-4}$-Alkyl-(8- bis 15-gliedrige tricyclische heteroalicyclische Gruppe), -$C_{1-4}$-Alkyl-($C_{5-8}$-Aryl) und -$C_{1-4}$-Alkyl-(5- bis 10-gliedriges Heteroaryl) jeweils gegebenenfalls mit 0, 1, 2, 3 oder 4 Resten $R_{1a}$ substituiert sind; und $R_{1a}$ jeweils unabhängig voneinander ausgewählt ist aus Halogen, -OH, - $NO_2$, -CN, -$SF_5$, -SH, -S-$C_{1-4}$-Alkyl, Oxo, $C_{1-4}$-Alkyl, $C_{1-4}$-Haloalkyl, $C_{1-4}$-Alkoxy, $C_{1-4}$-Haloalkoxy, $C_{2-8}$-Alkenyl, $C_{2-8}$-Alkinyl, $C_{3-7}$-alicyclischer Gruppe, 3- bis 10-gliedriger heteroalicyclischer Gruppe, $C_{5-6}$-Aryl, 5- bis 7-gliedriges Heteroaryl, -N($R_{13}$)($R_{14}$), - N($R_{13}$)(C(=O)$R_{15}$), -N($R_{13}$)(C(=O)-O$R_{15}$), -N($R_{15}$)(C(=O)-N($R_{13}$)($R_{14}$)), -C(=O)-N($R_{13}$)($R_{14}$), - C(=O)-$R_{15}$, -C(=O)-O$R_{15}$, -OC(=O)$R_{15}$, -N($R_{13}$)(S(=O)$_2$$R_{14}$), -S(=O)$_2$-N($R_{13}$)($R_{14}$), -S$R_{15}$ und - O$R_{15}$; und

$R_{10}$, $R_{11}$, $R_{12}$, $R_{13}$, $R_{14}$ und $R_{15}$, bei jedem Auftreten jeweils unabhängig voneinander ausgewählt sind aus H, $C_{1-6}$-Alkyl, $C_{1-6}$-Haloalkyl, $C_{3-7}$-alicyclischer Gruppe, 3- bis 10-gliedriger heteroalicyclischer Gruppe, $C_{5-6}$-Aryl, 5- bis 7-gliedriges Heteroaryl, $C_{7-11}$-bicyclisches Aryl, 7- bis 11-gliedriges bicyclisches Heteroaryl, -$C_{1-4}$-Alkyl-($C_{3-7}$-alicyclische Gruppe), -$C_{1-4}$-Alkyl-(3- bis 10-gliedrige heteroalicyclische Gruppe), -$C_{1-4}$-Alkyl-($C_{6-12}$-bicyclische alicyclische Gruppe), -$C_{1-4}$-Alkyl-(6- bis 12-gliedrige bicyclische heteroalicyclische Gruppe), -$C_{1-4}$-Alkyl-($C_{8-15}$-tricyclische alicyclische Gruppe), -$C_{1-4}$-Alkyl-(8- bis 15-gliedrige tricyclische heteroalicyclische Gruppe), -$C_{1-4}$-Alkyl-($C_{5-6}$-Aryl) und -$C_{1-4}$-Alkyl-(5- bis 10-gliedriges Heteroaryl),

wobei jeder in der Gruppe aufgeführte Substituent gegebenenfalls mit 0, 1, 2, 3 oder 4 Substituenten substituiert ist, die jeweils unabhängig voneinander ausgewählt sind aus einer Gruppe bestehend aus: Halogen, -OH, -$NH_2$, -NH($CH_3$), -N($CH_3$)$_2$, -CN, -$NO_2$, -$SF_5$, -SH, -S-$C_{1-4}$-Alkyl, Oxo, $C_{1-4}$-Alkyl, $C_{2-6}$-Alkenyl, $C_{2-6}$-Alkinyl, $C_{3-7}$-alicyclischer Gruppe, 3- bis 10-gliedriger heteroalicyclischer Gruppe, $C_{5-6}$-Aryl, 5- bis 7-gliedriges Heteroaryl, $C_{7-11}$-bicyclisches Aryl, 7- bis 11-gliedriges bicyclisches Heteroaryl, $C_{1-4}$-Hydroxyalkyl, -S-$C_{1-4}$-Alkyl, -C(=O)H,

-C(=O)-$C_{1-4}$-Alkyl, -C(=O)-O-$C_{1-4}$-Alkyl, -C(=O)-$NH_2$, -C(=O)-N($C_{1-4}$-Alkyl)$_2$, $C_{1-4}$-Haloalkyl, $C_{1-4}$-Alkoxy und $C_{1-4}$-Haloalkoxy;
oder $R_{10}$, $R_{11}$ und das/die daran gebundene(n) Atom(e) gemeinsam einen 3- bis 14-gliedrigen Ring bilden;
oder $R_{12}$, $R_{13}$ und das/die daran gebundene(n) Atom(e) gemeinsam einen 3- bis 14-gliedrigen Ring bilden;
1, 2 oder 3 $R_2$ in der Formel (I) vorhanden sind, und jedes $R_2$ H ist;
1, 2 oder 3 $R_3$ in der Formel (I) vorhanden sind, und jedes $R_3$ Methyl ist;
und
der Ring A ein Stickstoff enthaltender aromatischer Ring ist und erfüllt: 1) $G_1$ = $NR_4$, $G_2$ = $CR_5$; oder 2) $G_1$ = $CR_4$, $G_2$ = $NR_5$; oder 3) $G_1$ = $CR_4$, $G_2$ = O oder S;
wobei $R_4$ und $R_5$ jeweils unabhängig voneinander ausgewählt sind aus H, Halogen, -CN, -OH, -$NO_2$, $C_{1-6}$-Alkyl, $C_{1-6}$-Haloalkyl, $C_{1-6}$-Alkoxy;
oder $R_4$ und $R_5$ zusammen mit dem/den Atom(en), an den/die sie gebunden sind, einen 3- bis 8-gliedrigen Ring bilden.

**2.** Verbindung nach Anspruch 1 oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, wobei die Verbindung der Formel (I) eine der folgenden Strukturen aufweist:

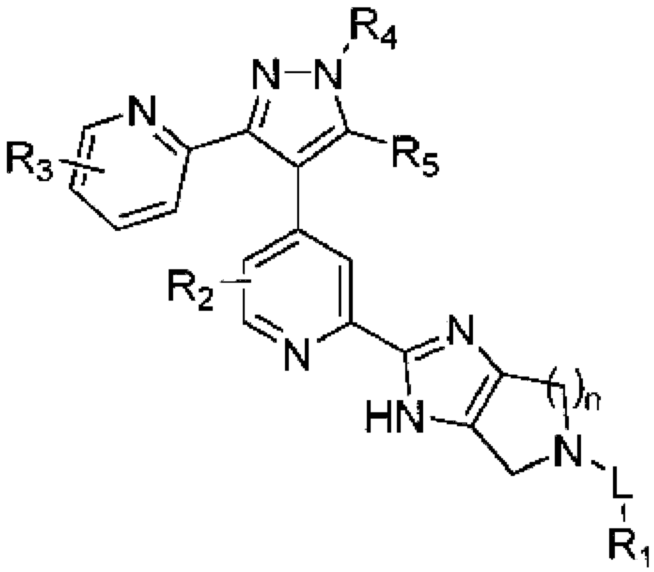

(I-1)

wobei n, L, $R_1$, $R_2$, $R_3$, $R_4$ und $R_5$ wie in Anspruch 1 definiert sind;

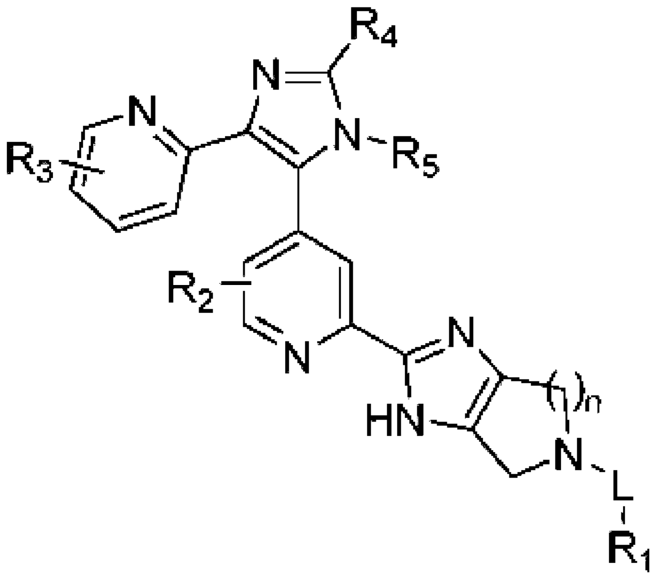

(I-2)

wobei n, L, $R_1$, $R_2$, $R_3$, $R_4$ und $R_5$ wie in Anspruch 1 definiert sind;

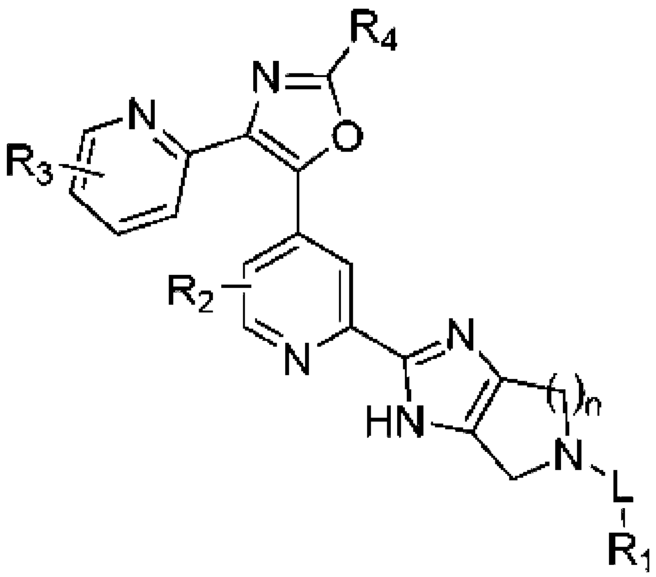

(I-3)

wobei n, L, $R_1$, $R_2$, $R_3$ und $R_4$ wie in Anspruch 1 definiert sind;

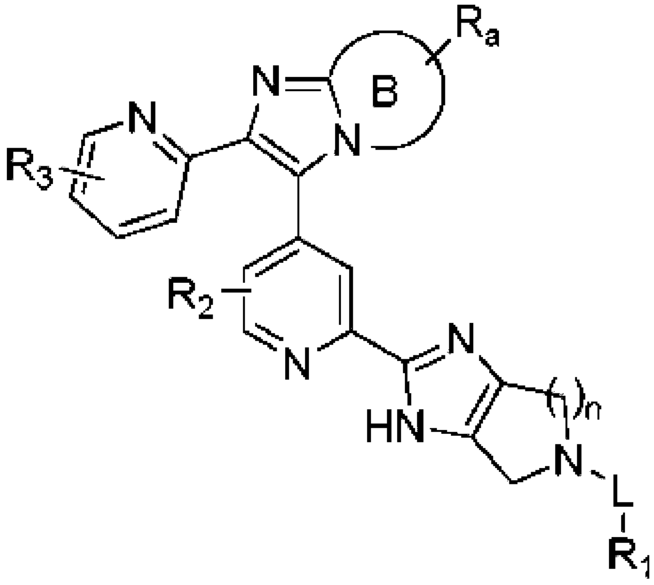

(I-4)

wobei n, L, $R_1$, $R_2$, $R_3$, $R_4$ und $R_5$ die in Anspruch 1 angegebene Bedeutung haben; und für (1-4) der Ring B ein 3- bis 8-gliedriger Ring ist, der 1 N-Atom enthält, und $R_6$ ausgewählt ist aus H, Halogen, -CN, -OH, $-NO_2$, $C_{1-6}$-Alkyl, $C_{1-6}$-Haloalkyl und $C_{1-6}$-Alkoxy.

**3.** Verbindung nach einem der Ansprüche 1 bis 2 oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, wobei $R_1$ ausgewählt ist aus H, Halogen, -OH, $-NO_2$, -CN, $-SF_5$, -SH, -S-$C_{1-4}$-Alkyl, $C_{1-6}$-Alkyl, $C_{1-6}$-Haloalkyl, $C_{1-6}$-Alkoxy, $C_{1-6}$-Haloalkoxy, $C_{3-6}$-alicyclische Gruppe, 4-bis 6-gliedrige heteroalicyclische Gruppe, $C_{5-6}$-Aryl, 5- bis 10-gliedriges Heteroaryl, -$C_{1-4}$-Alkyl-($C_{3-7}$-alicyclische Gruppe), -$C_{1-4}$-Alkyl-(3- bis 10-gliedrige heteroalicyclische Gruppe), -$C_{1-4}$-Alkyl-($C_{5-6}$-Aryl), -$C_{1-4}$-Alkyl-(5- bis 10-gliedriges Heteroaryl) und -N($R_{10}$)($R_{11}$), wobei die Reste -S-$C_{1-4}$-Alkyl, $C_{1-6}$-Alkyl, $C_{1-6}$-Haloalkyl, $C_{1-6}$-Alkoxy, $C_{1-6}$-Haloalkoxy, $C_{3-7}$-alicyclische Gruppe, 4- bis 6-gliedrige heteroalicyclische Gruppe, $C_{5-6}$-Aryl, 5- bis 10-gliedriges Heteroaryl, -$C_{1-4}$-Alkyl-($C_{3-7}$-alicyclische Gruppe), -$C_{1-4}$-Alkyl-(3- bis 10-gliedrige heteroalicyclische Gruppe), -$C_{1-4}$-Alkyl-($C_{5-6}$-Aryl) und -$C_{1-4}$-Alkyl-(5- bis 10-gliedriges Heteroaryl) jeweils gegebenenfalls mit 0, 1, 2, 3 oder 4 Resten $R_{1a}$ substituiert sind, und $R_{1a}$ jeweils unabhängig voneinander ausgewählt ist aus Halogen, -OH, $-NO_2$, -CN, Oxo, $C_{1-4}$-Alkyl, $C_{1-4}$-Haloalkyl, $C_{1-4}$-Alkoxy, $C_{1-4}$-Haloalkoxy, $C_{3-7}$-alicyclischer Gruppe, 3- bis 10-gliedriger heteroalicyclischer Gruppe, $C_{5-6}$-Aryl, 5- bis 7-gliedriges Heteroaryl; und

$R_{10}$, $R_{11}$ und $R_{12}$ jeweils unabhängig voneinander ausgewählt sind aus: H, $C_{1-6}$-Alkyl, $C_{1-6}$-Haloalkyl, $C_{3-7}$-alicyclischer Gruppe und 3- bis 10-gliedriger heteroalicyclischer Gruppe, wobei jeder in der Gruppe aufgeführte Substituent gegebenenfalls mit 0, 1, 2, 3 oder 4 Substituenten substituiert ist, die unabhängig voneinander ausgewählt sind aus einer Gruppe bestehend aus Halogen, -OH, $-NH_2$, Oxo, $C_{1-4}$-Alkyl, $C_{1-4}$-Hydroxyalkyl, $C_{1-4}$-Haloalkyl, $C_{1-4}$-Alkoxy und $C_{1-4}$-Haloalkoxy;
oder $R_{10}$, $R_{11}$ und die Atome, an die sie gebunden sind, gemeinsam einen 3- bis 8-gliedrigen Ring bilden.

**4.** Verbindung nach einem der Ansprüche 1 bis 3 oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, wobei $R_1$ ausgewählt ist aus H, Halogen, -OH, Methyl, Ethyl, Propyl, Butyl, Pentyl, Hexyl, Methoxy, Ethoxy, Propoxy, Butoxy, Pentoxy, Hexyloxy, Cyclopropyl, Cyclobutyl, Cyclopentyl, Cyclohexyl, Cycloheptyl, Phenyl, Piperazinyl, Piperidinyl, Morpholinyl, Pyrrolidinyl, Pyrrolyl, Pyridinyl und Pyrazolyl, wobei die Reste Methyl, Ethyl, Propyl, Butyl, Pentyl, Hexyl, Methoxy, Ethoxy, Propoxy, Butoxy, Pentoxy, Hexyloxy, Cyclopropyl, Cyclobutyl, Cyclopentyl, Cyclohexyl, Cycloheptyl, Phenyl, Piperazinyl, Piperidinyl, Morpholinyl, Pyrrolidinyl, Pyrrolyl, Pyridinyl und Pyrazolyl jeweils gegebenenfalls mit 1 oder 2 Resten $R^{1a}$ substituiert sind, und $R^{1a}$ jeweils unabhängig voneinander ausgewählt ist aus Halogen, -OH, $-NO_2$, -CN, Oxo, $C_{1-4}$-Alkyl, $C_{1-4}$-Haloalkyl und $C_{1-4}$-Alkoxy;
vorzugsweise ist $R_1$ ausgewählt ist aus H, Methyl, Methylpiperazinyl, Pyrrolidinyl, Methylpiperazinyl, Hydroxycyclobutyl, Methylpyrazolyl, Hydroxyethyl, Hydroxypropyl, Methylpiperidinyl, Morpholinyl und Hydroxypyrrolidinyl.

**5.** Verbindung nach einem der Ansprüche 1 bis 4 oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, wobei die Verbindung ausgewählt ist aus den Verbindungen 1-4, 6-7, 9-15, 22-23, 25-34 und 37-76.

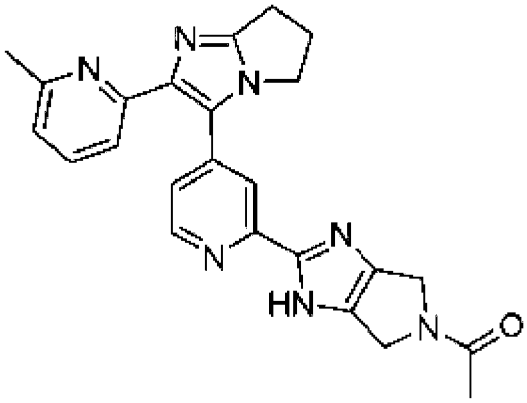

1 ,

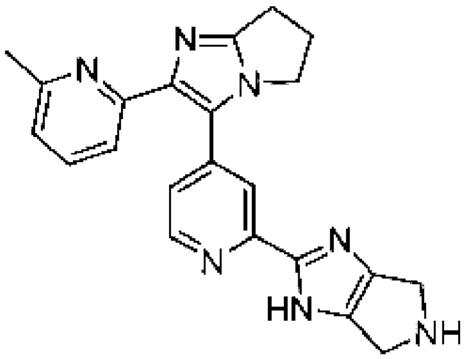

2 ,

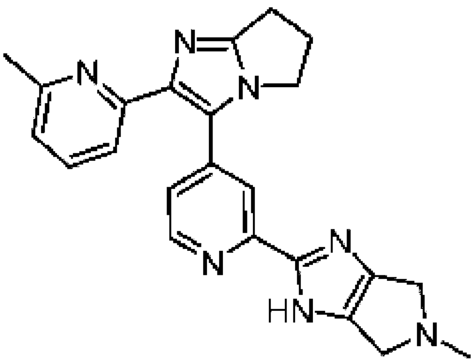

3 ,

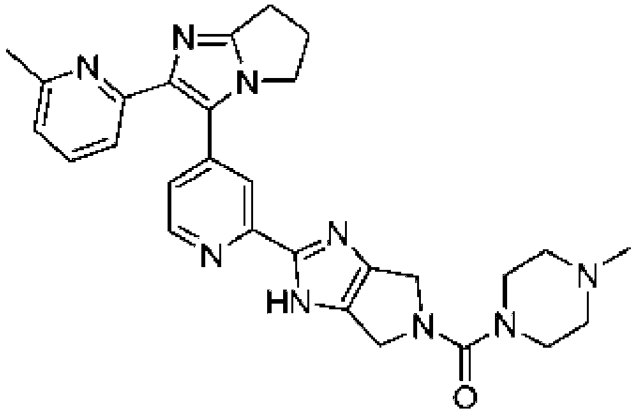

4 ,

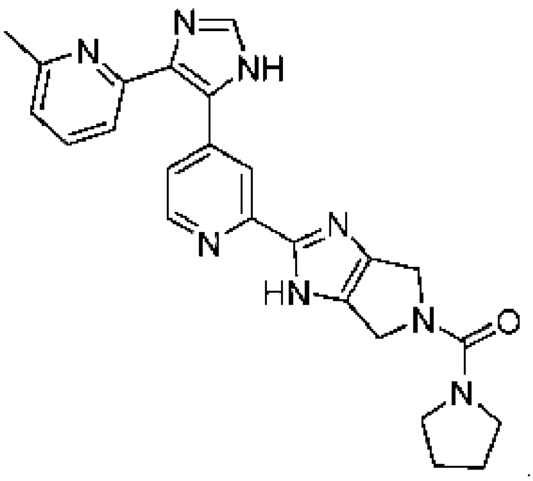

6 ,

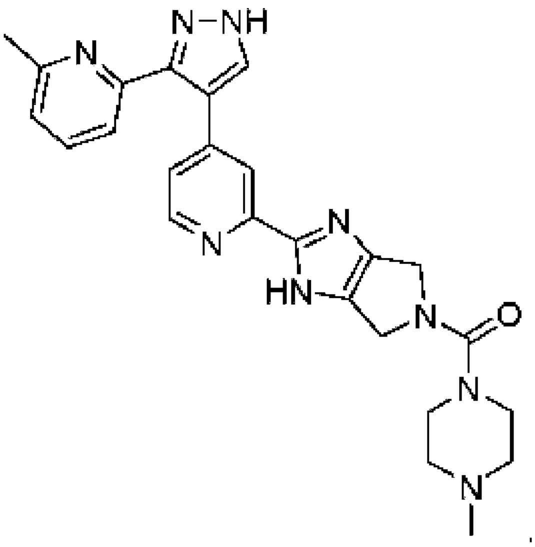

7 ,

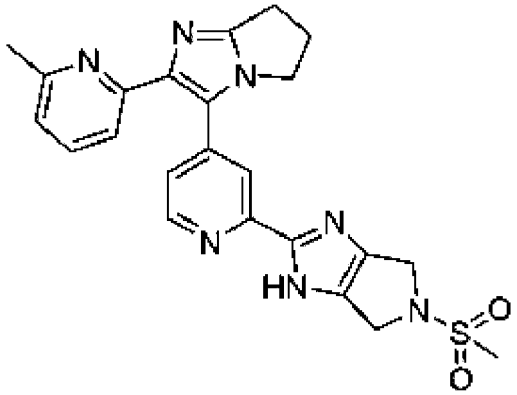

9 ,

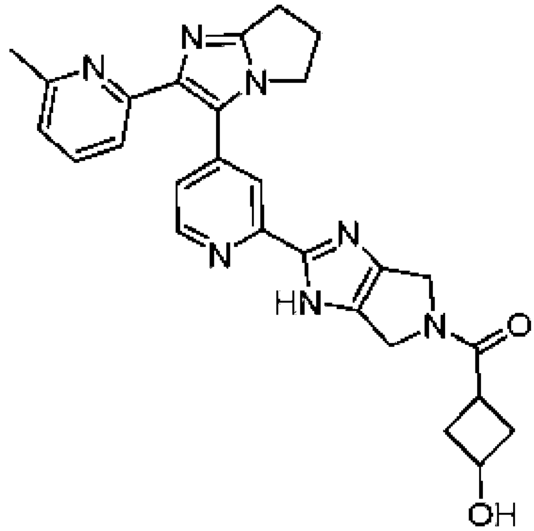

10 ,

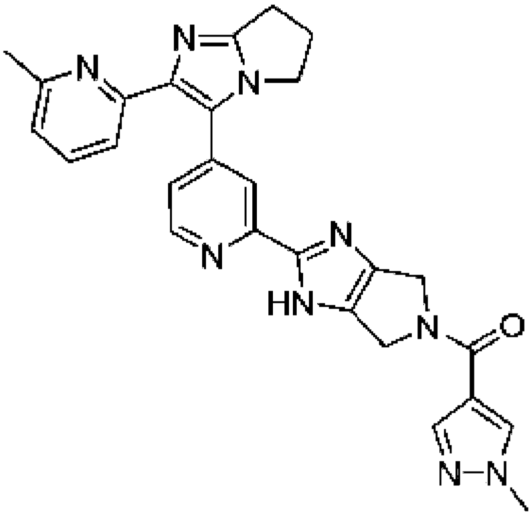

11 ,

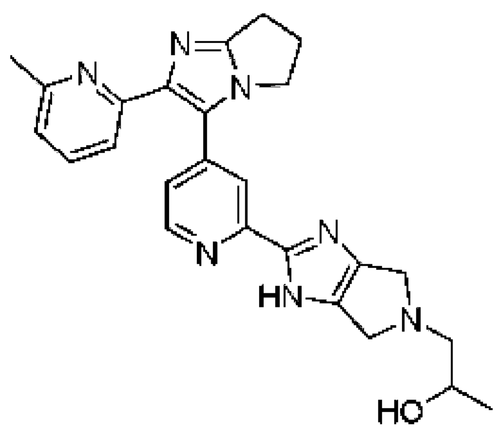

12 ,

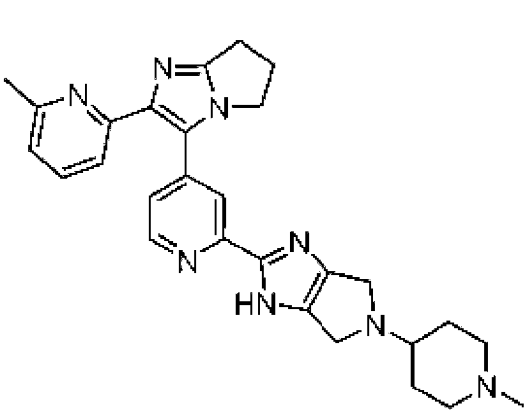

13 ,

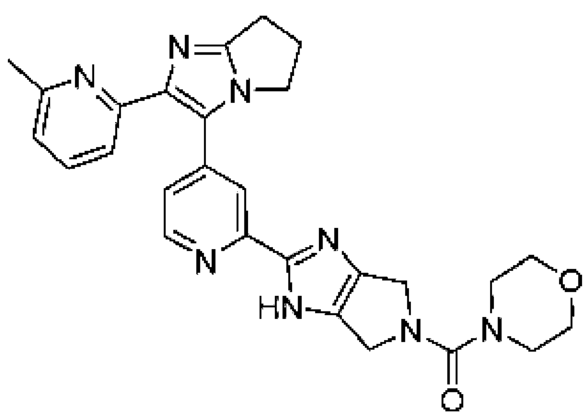

14 ,

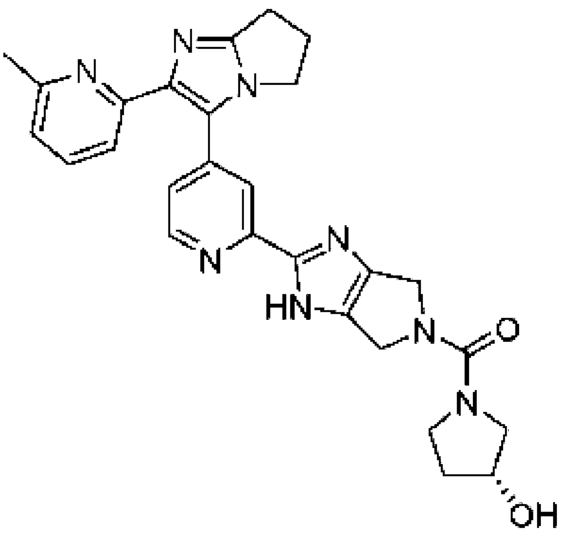
15 ,

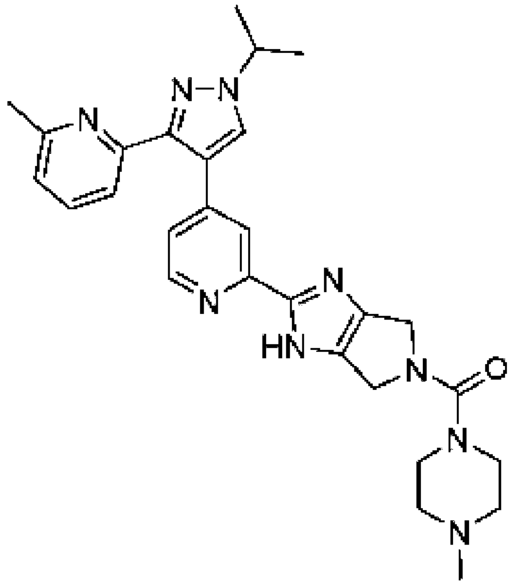
22 ,

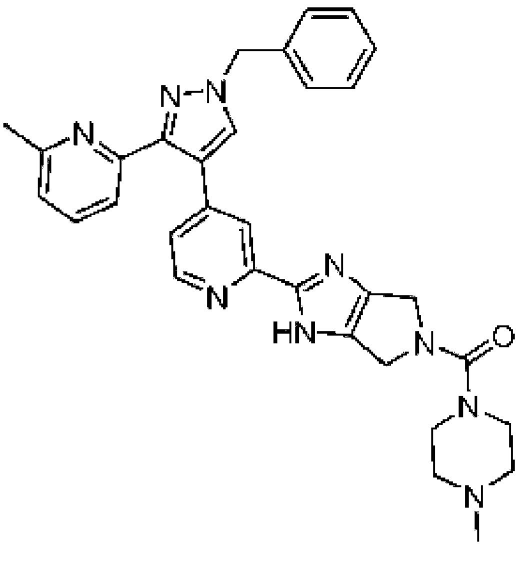
23,

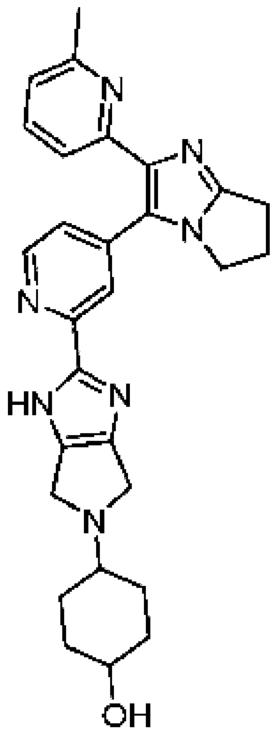
25,

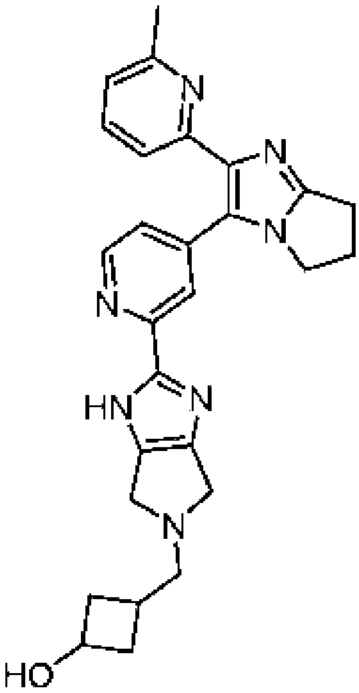
26,

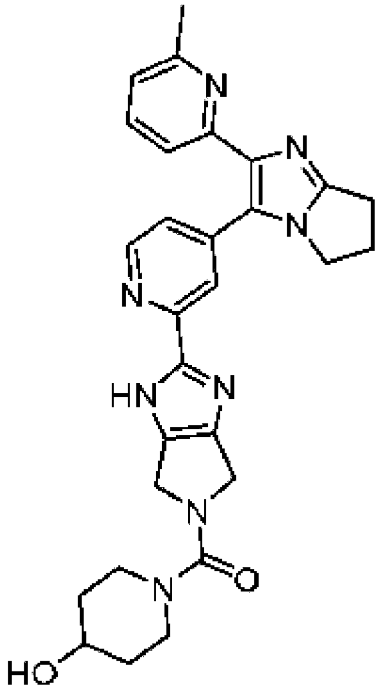
27,

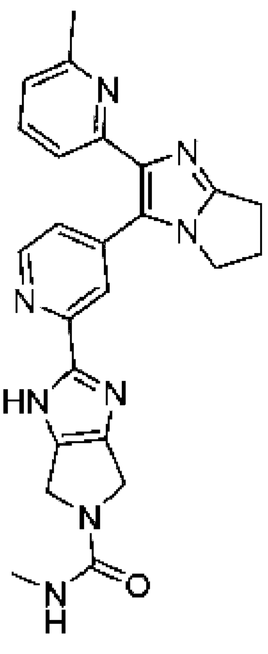
28,

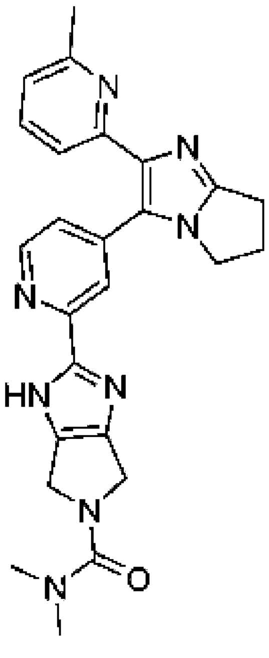
29,

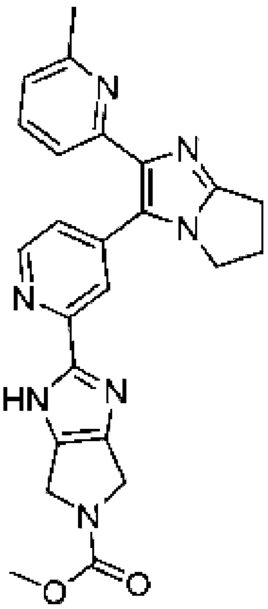
30,

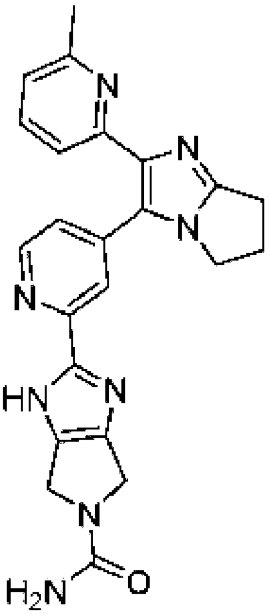
31,

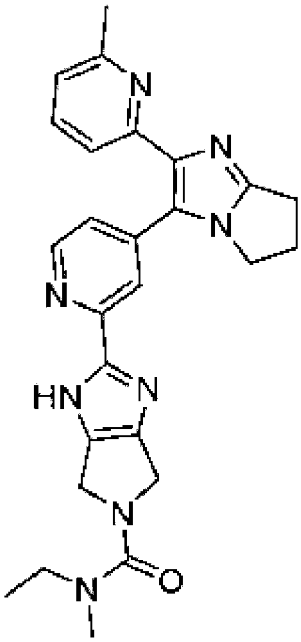
32,

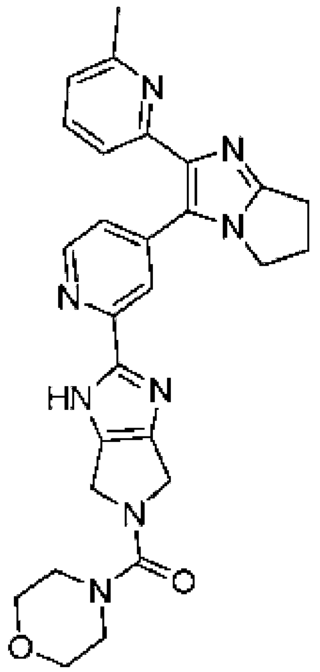
33,

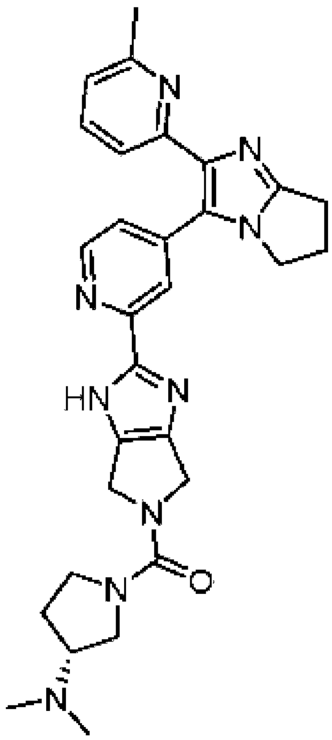

34,

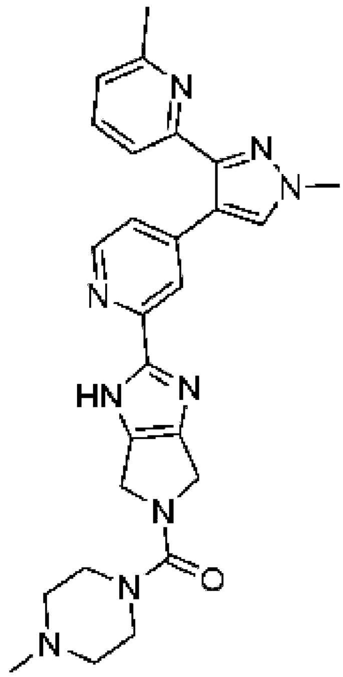

37,

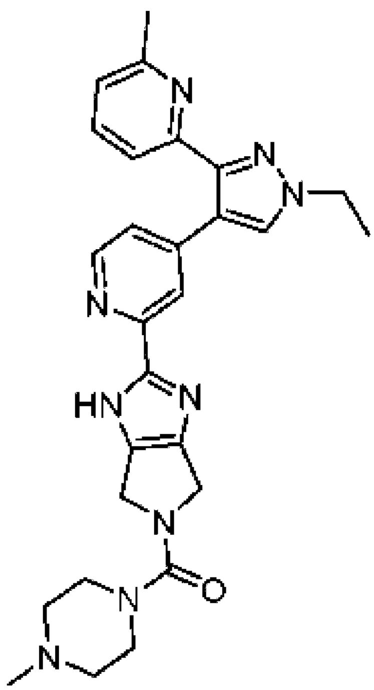

38,

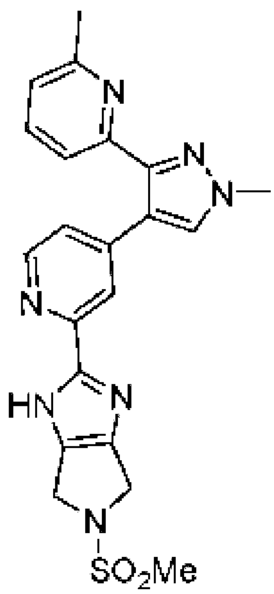

39,

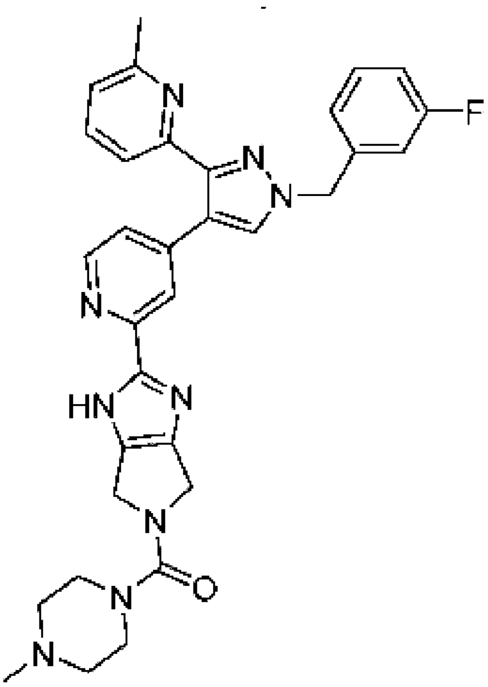

40,

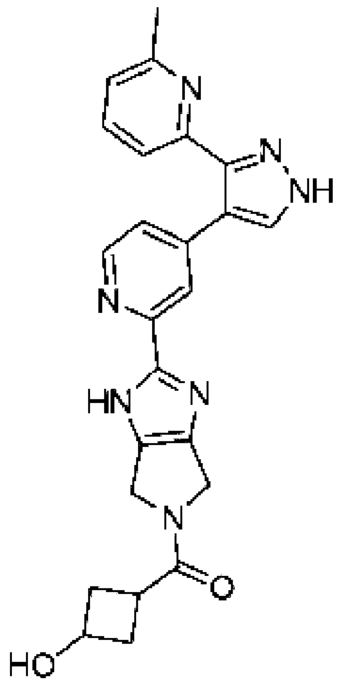

41,

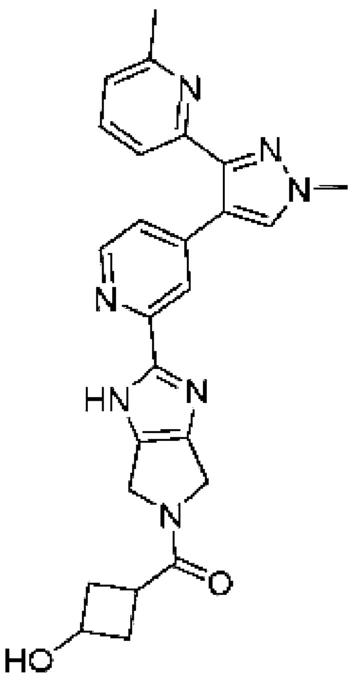

42,

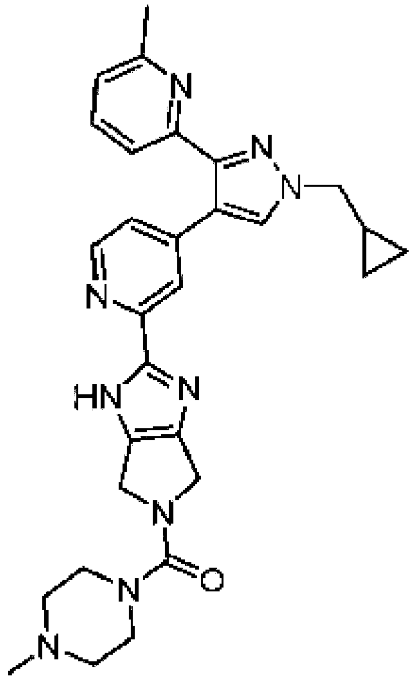

43,

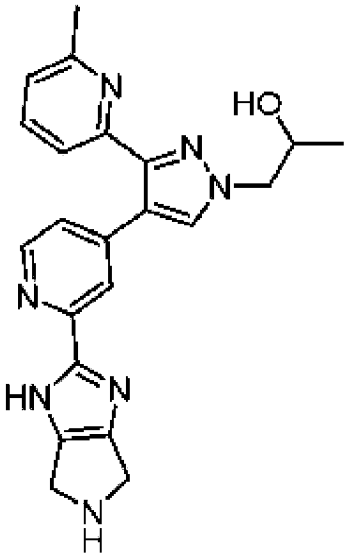

44,

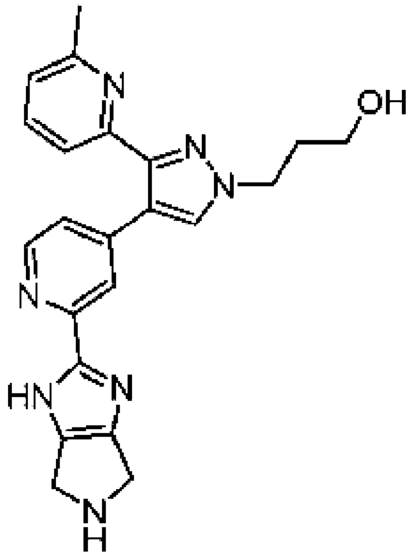

45,

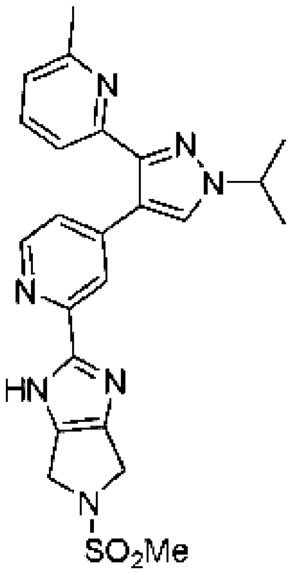

46,

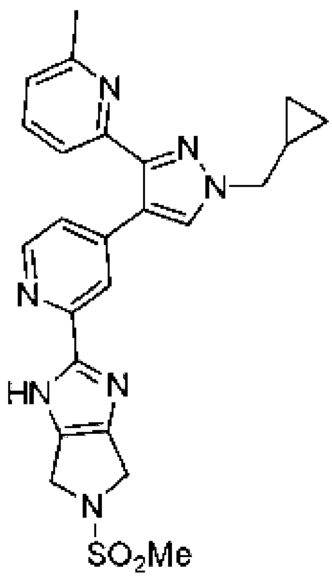

47,

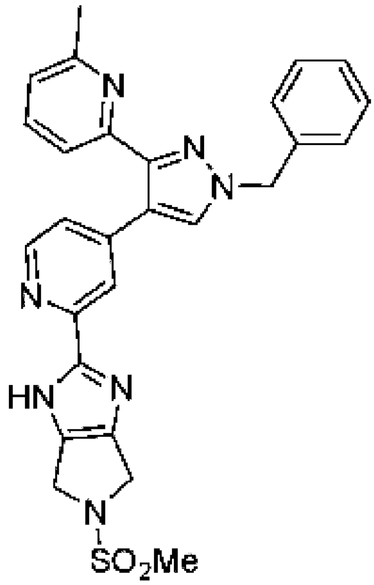

48,

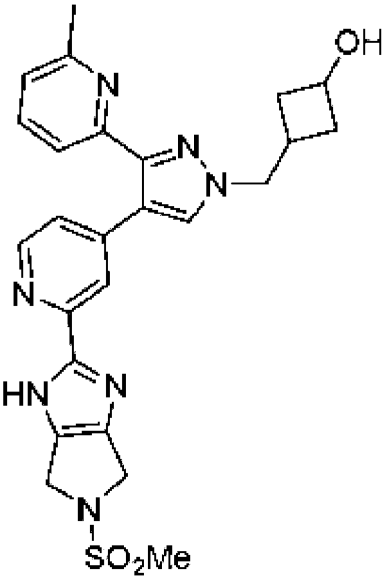

49,

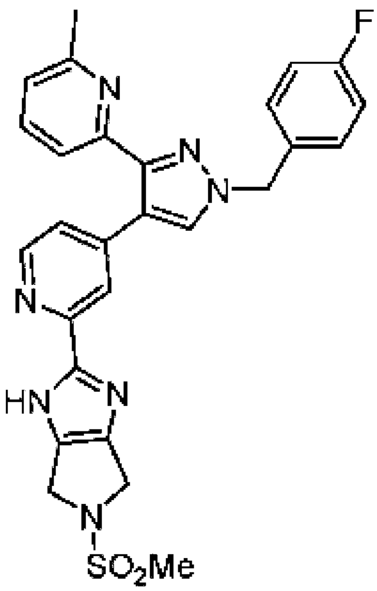

50,

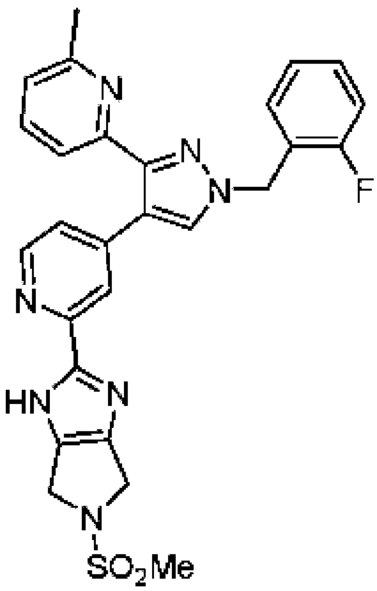

51,

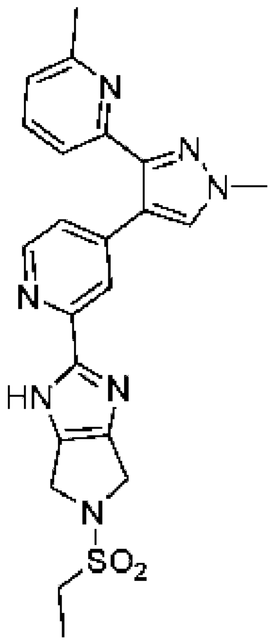

52,

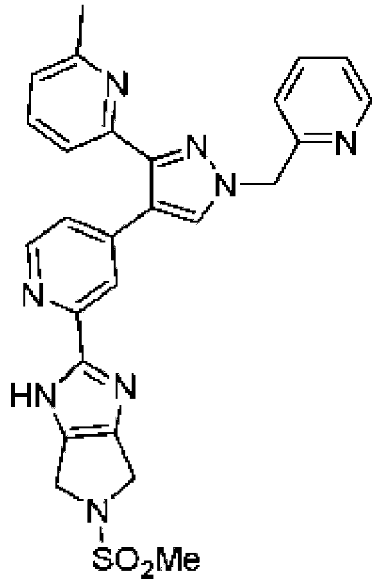

53,

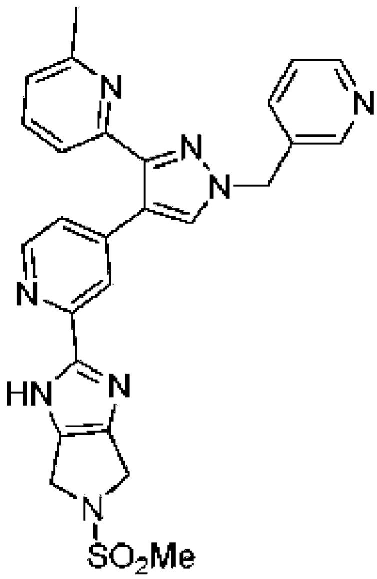

54,

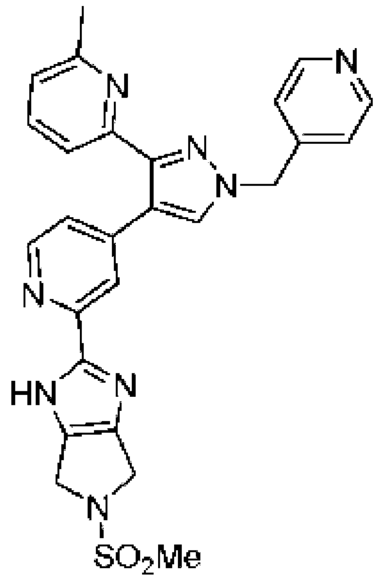

55,

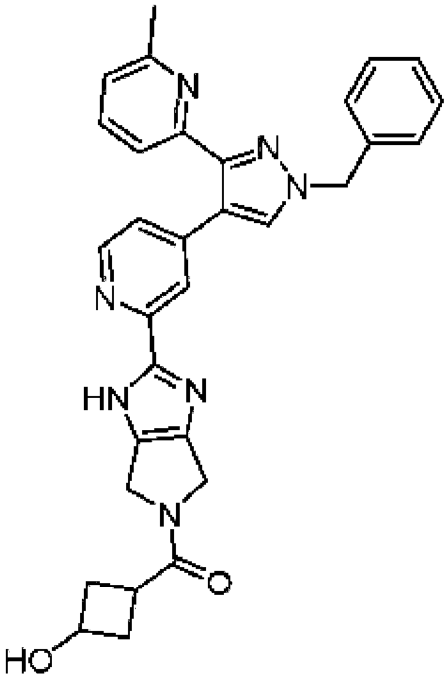

56,

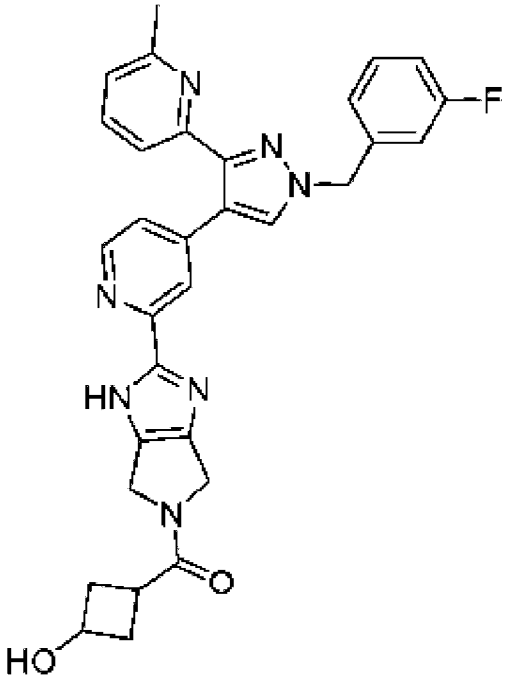

57,

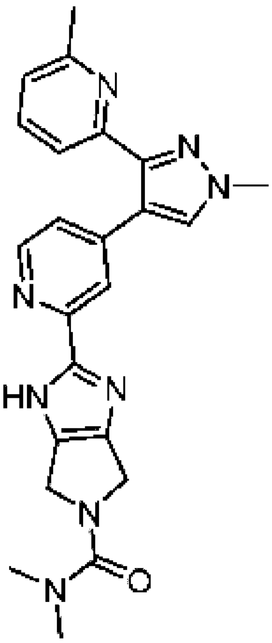

58,

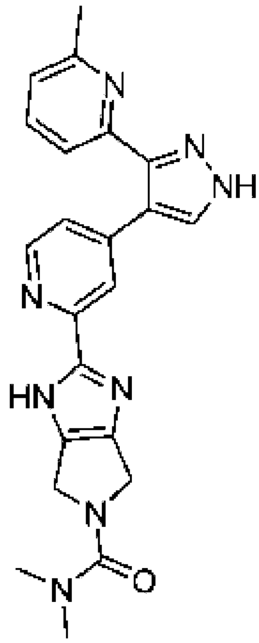

59,

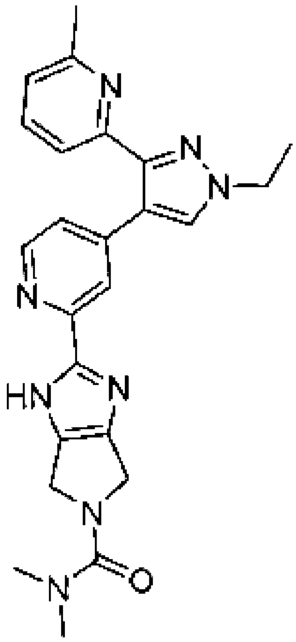

60,

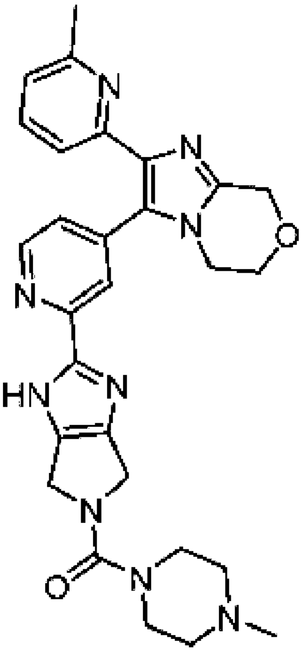

61,

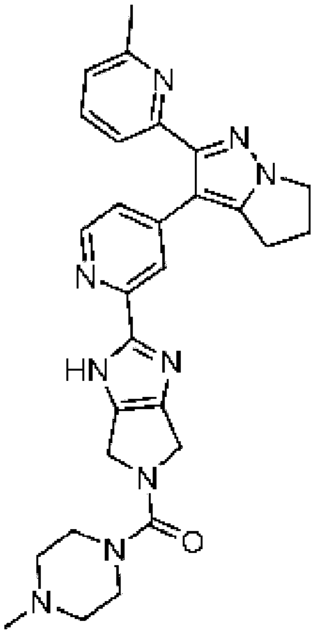

62,

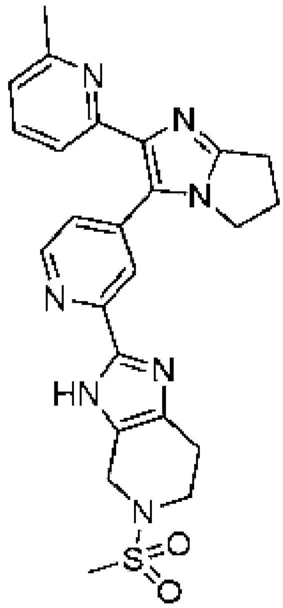

63,

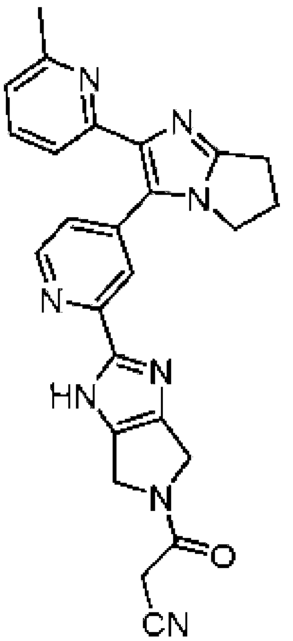

64,

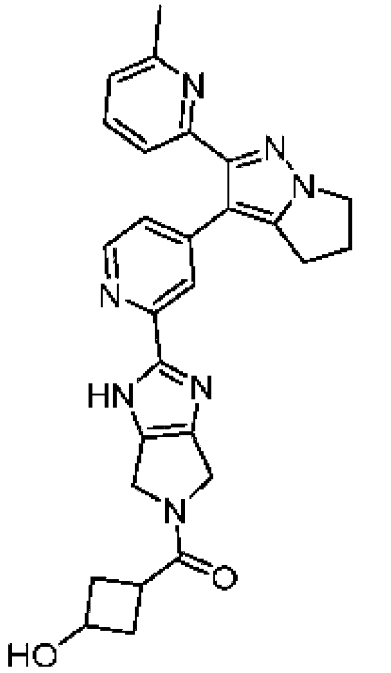

65,

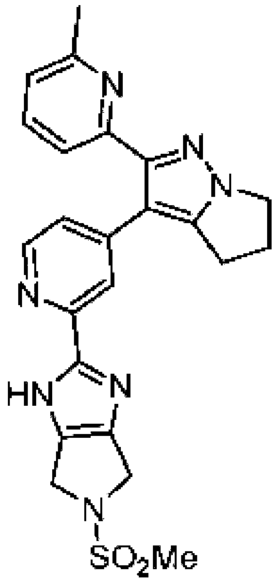

66,

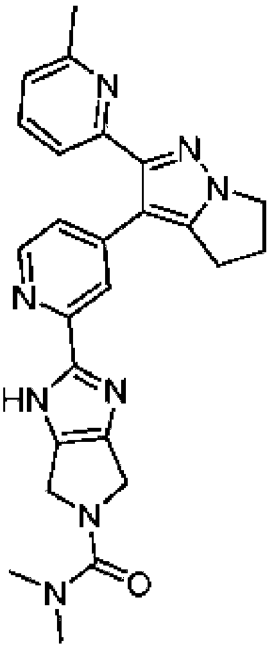

67,

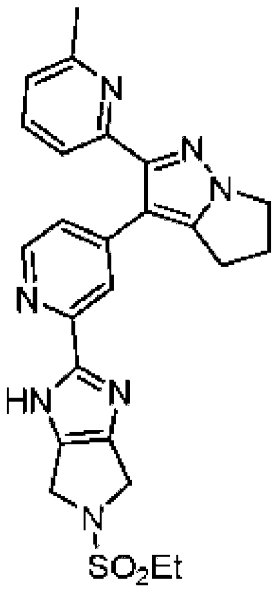

68,

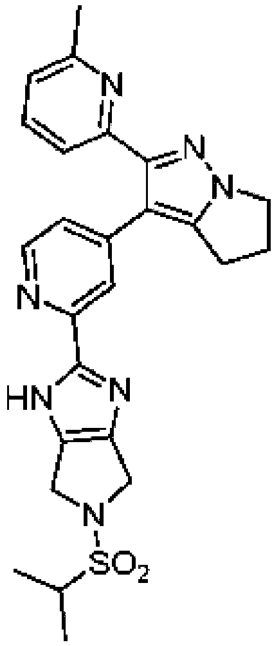

69,

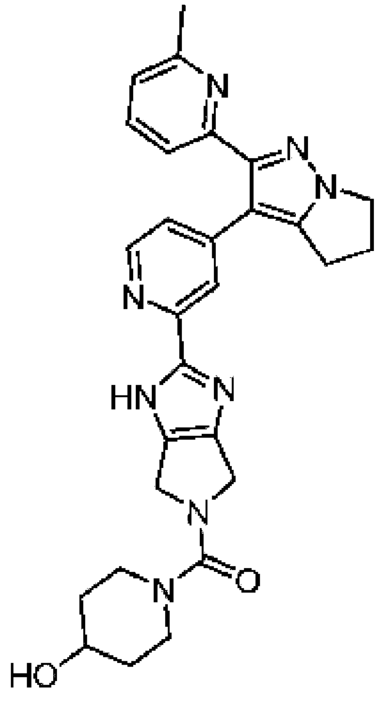

70,

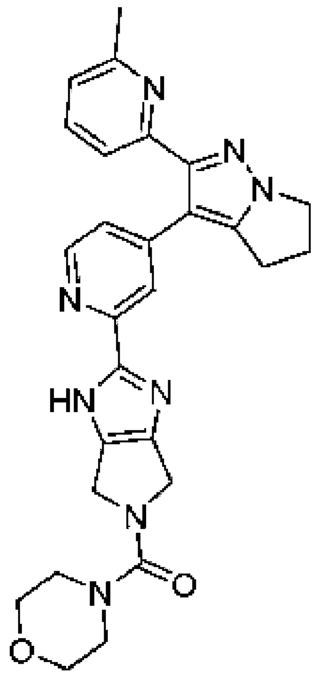

71,

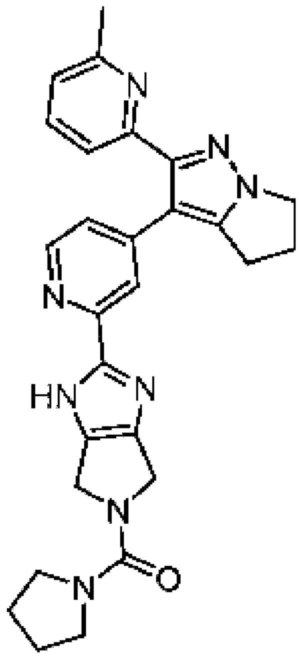

72,

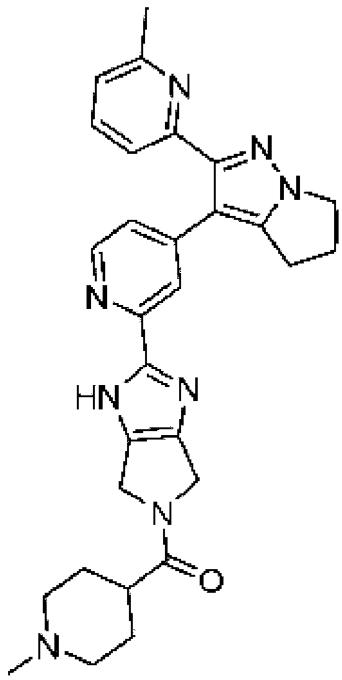

73,

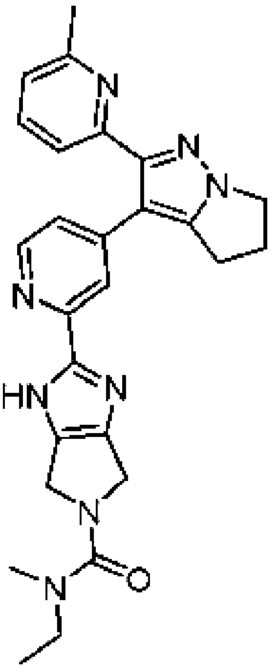

74,

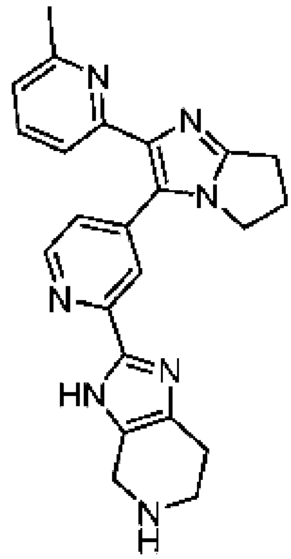

75,

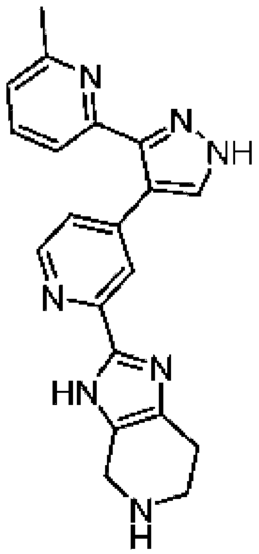

76.

6. Pharmazeutische Zusammensetzung, umfassend die Verbindung nach einem der Ansprüche 1 bis 5, oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, und einen oder mehrere pharmazeutisch verträgliche Träger, Hilfsstoffe oder Exzipienten.

7. Verbindung nach einem der Ansprüche 1 bis 5, oder eine isotopenmarkierte Verbindung davon, oder ein optisches Isomer davon, ein geometrisches Isomer davon, ein Tautomer davon oder ein Gemisch verschiedener Isomere davon, oder ein pharmazeutisch verträgliches Salz davon, zur Verwendung als Arzneimittel zur Behandlung von mit TGF-β, vorzugsweise TGF-$\beta_1$, assoziierten Krankheiten oder Zuständen, wobei die mit TGF-β assoziierten Krankheiten oder Zustände ausgewählt sind aus Krebs, Virusinfektionen, chronischer Glomerulonephritis, akuter Glomerulonephritis, diabetischer Nephropathie, Osteoporose, Arthritis, Wundheilung, Narbenbildung, Ulzera, Korneatrauma, Herzklappenstenose, kongestiver Herznekrose, neurologischen Schäden, Alzheimer-Krankheit, peritonealen oder subkutanen Adhäsionen, Arteriosklerose, Hautfibrose und Hautalterung aufgrund von Fettverlust, Skelett- oder Chondrozyten-Störungen, Hypophosphatämie-Störungen und Organfibrose-Krankheiten, vorzugsweise die mit TGF-β assoziierten Krankheiten oder Zustände ausgewählt sind aus hepatozellulärem Karzinom, Brustkrebs, Blasenkrebs, kolorektalem Krebs, Melanom, Mesotheliom, Lungenkrebs, Prostatakrebs, Pankreaskrebs, Hodenkrebs, Schilddrüsenkrebs, Plattenepithelkarzinom, Glioblastom, Neuroblastom, Zervixkarzinom, Rhabdomyosarkom, Nierenfibrose, Leberfibrose, Lungenfibrose, Hautnarbenbildung, Hautfibrose und Hautalterung aufgrund von Fettverlust.

8. Pharmazeutische Zusammensetzung nach Anspruch 6, zur Verwendung als Arzneimittel zur Behandlung von mit TGF-β, vorzugsweise TGF-$\beta_1$, assoziierten Krankheiten oder Zuständen, wobei die mit TGF-β assoziierten Krankheiten oder Zustände ausgewählt sind aus Krebs, Virusinfektionen, chronischer Glomerulonephritis, akuter Glomerulonephritis, diabetischer Nephropathie, Osteoporose, Arthritis, Wundheilung, Narbenbildung, Ulzera, Korneatrauma, Herzklappenstenose, kongestiver Herznekrose, neurologischen Schäden, Alzheimer-Krankheit, peritonealen oder subkutanen Adhäsionen, Arteriosklerose, Hautfibrose und Hautalterung aufgrund von Fettverlust, Skelett- oder Chondrozyten-Störungen, Hypophosphatämie-Störungen und Organfibrose-Krankheiten, vorzugsweise die mit TGF-β assoziierten Krankheiten oder Zustände ausgewählt sind aus hepatozellulärem Karzinom, Brustkrebs, Blasenkrebs, kolorektalem Krebs, Melanom, Mesotheliom, Lungenkrebs, Prostatakrebs, Pankreaskrebs, Hodenkrebs, Schilddrüsenkrebs, Plattenepithelkarzinom, Glioblastom, Neuroblastom, Zervixkarzinom, Rhabdomyosarkom, Nierenfibrose, Leberfibrose, Lungenfibrose, Hautnarbenbildung, Hautfibrose und Hautalterung aufgrund von Fettverlust.

## Revendications

1. Composé de Formule (I),

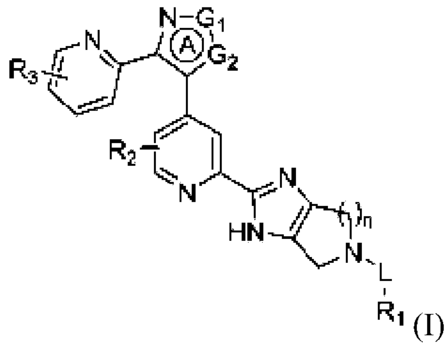

(I)

ou un composé isotopiquement marqué de celui-ci, ou un isomère optique de celui-ci, un isomère géométrique de celui-ci, un tautomère de celui-ci ou un mélange de l'isomère optique, de l'isomère géométrique et du tautomère, ou un sel pharmaceutiquement acceptable de celui-ci,
dans lequel
n est égal à 1 ;
L est (C=O), (O=S=O), ((C=O)-$CH_2$), $CH_2$ ou une liaison ; et
$R_1$ est choisi parmi H, halogène, -OH, -$NO_2$, -CN, -$SF_5$, -SH, -S-alkyle en C1-4, alkyle en C1-6, haloalkyle en C1-6, alcoxyle en C1-6, haloalcoxyle en C1-6, alcényle en C2-8, alcynyle en C2-8, un groupe alicyclique en C3-7, un groupe hétéroalicyclique à 3-10 chaînons, un groupe alicyclique bicyclique en C6-12, un groupe hétéroalicyclique bicyclique à 6-12 chaînons, un groupe alicyclique tricyclique en C8-15, un groupe hétéroalicyclique tricyclique à 8-15 chaînons, aryle en C5-8, hétéroaryle à 5-10 chaînons, aryle bicyclique en C7-11, hétéroaryle bicyclique à 7-11 chaînons, -alkyle en C1-4-(groupe alicyclique en C3-7), -alkyle en C1-4-(groupe hétéroalicyclique à 3 à 10 chaînons), -alkyle en C1-4-(groupe alicyclique bicyclique en C6-12), -alkyle en C1-4-(groupe hétéroalicyclique bicyclique à 6 à 12 chaînons), -alkyle en C1-4-(groupe alicyclique tricyclique en C8-15), -alkyle en C1-4-(groupe hétéroalicyclique tricyclique à 8 à 15 chaînons), -alkyle en C1-4-(aryle en C5-8), -alkyle en C1-4-(hétéroaryle à 5-10 chaînons), -N($R_{10}$)($R_{11}$), -N($R_{10}$)(C(=O)$R_{11}$), -N($R_{10}$)(C(=O)-O$R_{11}$), -N($R_{12}$)(C($_F$O)-N($R_{10}$)($R_{11}$)), -C(=O)-N($R_{10}$)($R_{11}$), -C(=O)-$R_{12}$, -C(=O)-O$R_{12}$, -OC(=O)$R_{12}$, -N($R_{10}$)(S(=O)$_2R_{11}$), -S(=O)$_2$-N($R_{10}$)($R_{11}$), -S$R_{12}$, et -O$R_{12}$, dans lesquels -S-alkyle en C1-4, alkyle en C1-6, haloalkyle en C1-6, alcoxyle en C1-6, haloalcoxyle en C1-6, alcényle en C2-8, alcynyle en C2-8, le groupe alicyclique en C3-7, le groupe hétéroalicyclique à 3-10 chaînons, le groupe alicyclique bicyclique en C6-12, le groupe hétéroalicyclique bicyclique à 6-12 chaînons, le groupe alicyclique tricyclique en C8-15, le groupe hétéroalicyclique tricyclique à 8-15 chaînons, aryle en C5-8, hétéroaryle à 5-10 chaînons, aryle bicyclique en C7-11, hétéroaryle bicyclique à 7-11 chaînons, -alkyle en C1-4-(groupe alicyclique en C3-7), -alkyle en C1-4-(groupe hétéroalicyclique à 3 à 10 chaînons), -alkyle en C1-4-(groupe alicyclique bicyclique en C6-12), -alkyle en C1-4-(groupe hétéroalicyclique bicyclique à 6 à 12 chaînons), -alkyle en C1-4-(groupe alicyclique tricyclique en C8-15), -alkyle en C1-4-(groupe hétéroalicyclique tricyclique à 8 à 15 chaînons), -alkyle en C1-4-(aryle en C5-8) et -alkyle en C1-4-(hétéroaryle à 5-10 chaînons) sont éventuellement substitués chacun par 0, 1, 2, 3 ou 4 $R^{1a}$ ; et $R^{1a}$ est indépendamment choisi parmi halogène, -OH, - $NO_2$, -CN, -$SF_5$, -SH, -S-alkyle en C1-4, oxo, alkyle en C1-4, haloalkyle en C1-4, alcoxyle en C1-4, haloalcoxyle en C1-4, alcényle en C2-8, alcynyle en C2-8, un groupe alicyclique en C3-7, un groupe hétéroalicyclique à 3-10 chaînons, aryle en C5-8, hétéroaryle à 5-7 chaînons, -N($R_{13}$)($R_{14}$), -N($R_{13}$)(C(=O)$R_{14}$), -N($R_{13}$)(C(=O)-O$R_{14}$), -N($R_{15}$)(C(=O)-N($R_{13}$)($R_{14}$)), -C(=O)-N($R_{13}$)($R_{14}$), -C(=O)-$R_{15}$, -C(=O)-O$R_{15}$, -OC(=O)$R_{15}$, -N($R_{13}$)(S(=O)$_2R_{14}$), -S(=O)$_2$-N($R_{13}$)($R_{14}$), -S$R_{15}$, et -O$R_{15}$ ; et
$R_{10}$, $R_{11}$, $R_{12}$, $R_{13}$, $R_{14}$ et $R_{15}$, à chaque événement, sont choisis chacun indépendamment parmi H, alkyle en C1-6, haloalkyle en C1-6, un groupe alicyclique en C3-7, un groupe hétéroalicyclique à 3-10 chaînons, aryle en C5-8, hétéroaryle à 5-7 chaînons, bicycloaryle en C7-11, hétéroaryle bicyclique à 7-11 chaînons, -alkyle en C1-4-(groupe alicyclique C3-7), -alkyle en C1-4 (groupe hétéroalicyclique à 3-10 chaînons), -alkyle en C1-4 -(groupe alicyclique bicyclique en C6-12), -alkyle en C1-4-(groupe heteroalicyclique bicyclique à 6-12 chaînons), -alkyle en C1-4 -(groupe alicyclique tricyclique en C8-15), -alkyle en C1-4-(groupe hetéroalicyclique tricyclique à 8-15 chaînons), -alkyle en C1-4-(aryle en C5-8), et -alkyle en C1-4-(hétéroaryle à 5-10 chaînons), dans lesquels chacun listé dans le groupe est éventuellement substitué par 0, 1, 2, 3 ou 4 substituants chacun choisi indépendamment dans un groupe consistant en halogène, -OH, -$NH_2$, -NH($CH_3$), -N($CH_3$)$_2$, -CN, -$NO_2$, -$SF_5$, -SH, -S-$C_{1-4}$ alkyle, oxo, alkyle en C1-4, alcényle en C2-6, alcynyle en C2-6, un groupe alicyclique en C3-7, un groupe hétéroalicyclique à 3-10 chaînons, aryle en C5-8, hétéroaryle à 5-7 chaînons, bicycloaryle en C7-11, hétéroaryle bicyclique à 7-11 chaînons, hydroxyalkyle en C1-4, -S-$C_{1-4}$ alkyle, -C(=O)H, -C(=O)-$C_{1-4}$ alkyle, -C(=O)-O-$C_{1-4}$ alkyle, -C(=O)-$NH_2$, -C(=O)-N($C_{1-4}$ alkyle)$_2$, haloalkyle en C1-4, alcoxyle en C1-4 et haloalcoxyle en C1-4 ; ou $R_{10}$, $R_{11}$, et les atomes attachés à ceux-ci forment ensemble un cycle à 3 à 14 chaînons ;

ou $R_{13}$, $R_{14}$, et les atomes attachés à ceux-ci forment ensemble un cycle à 3 à 14 chaînons ;
1, 2, ou 3 $R_2$(s) se présentent dans la formule (I), et chaque $R_2$ est H ;
1, 2, ou 3 $R_3$(s) se présentent dans la formule (I), et chaque $R_3$ est méthyle ;
et
le cycle A est un cycle aromatique contenant l'azote et satisfait à : 1) G1 = $NR_4$, G2 = $CR_5$ ; ou 2) G1 = $CR_4$, G2 = $NR_5$ ; ou 3) G1 = $CR_4$, G2 = O ou S ; dans lesquels $R_4$ et $R_5$ sont choisis chacun indépendamment parmi H, halogène, -CN, -OH, $-NO_2$, alkyle en C1-6, haloalkyle en C1-6, alcoxyle en C1-6 ; ou $R_4$ et $R_5$ et les atomes attachés à ceux-ci forment ensemble un cycle à 3 à 8 chaînons.

**2.** Composé selon la revendication 1, ou un composé isotopiquement marqué de celui-ci, ou un isomère optique de celui-ci, un isomère géométrique de celui-ci, un tautomère de celui-ci ou un mélange de l'isomère optique, de l'isomère géométrique et du tautomère, ou un sel pharmaceutiquement acceptable de celui-ci, dans lequel le composé de formule (I) présente l'une des structures suivantes :

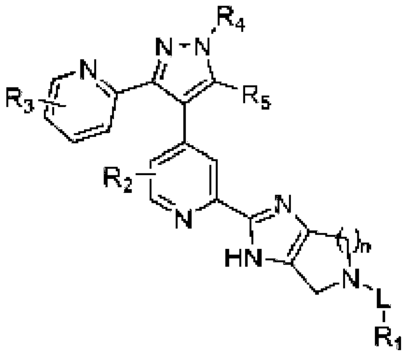
(I-1)

où n, L, $R_1$, $R_2$, $R_3$, $R_4$, et $R_5$ sont définis dans la revendication 1 ;

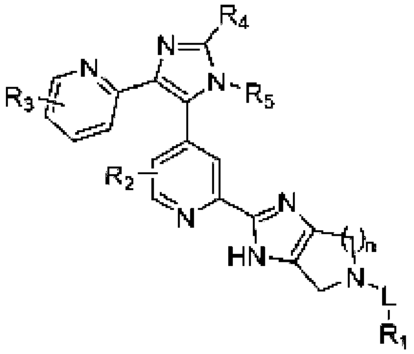
(I-2)

où n, L, $R_1$, $R_2$, $R_3$, $R_4$, et $R_5$ sont définis dans la revendication 1 ;

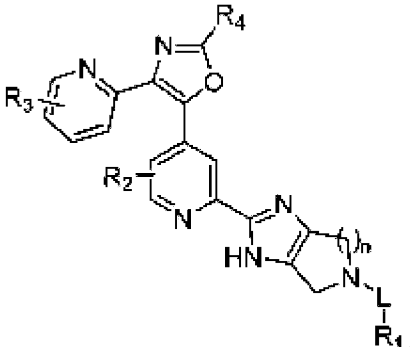
(I-3)

où n, L, $R_1$, $R_2$, $R_3$ et $R_4$ sont définis dans la revendication 1 ;

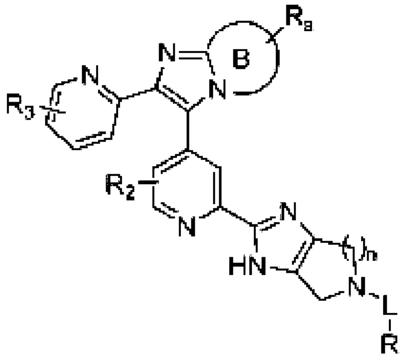

(I-4)

où n, L, $R_1$, $R_2$, et $R_3$ sont comme définis dans la revendication 1 ; le cycle B est un cycle à 3 à 8 chaînons contenant 1 atome de N, et $R_a$ est choisi parmi H, halogène, -CN, -OH, -$NO_2$, alkyle en C1-6, haloalkyle en C1-6, alcoxyle en C1-6.

**3.** Composé selon l'une quelconque des revendications 1 à 2, ou un composé isotopiquement marqué de celui-ci, ou un isomère optique de celui-ci, un isomère géométrique de celui-ci, un tautomère de celui-ci ou un mélange de l'isomère optique, de l'isomère géométrique et du tautomère, ou un sel pharmaceutiquement acceptable de celui-ci, dans lesquels $R_1$ est choisi parmi H, halogène, -OH,- $NO_2$, -CN, -$SF_5$, -SH, -S-alkyle en C1-4, alkyle en C1-6, haloalkyle en C1-6, alcoxyle en C1-6, haloalcoxyle en C1-6, un groupe alicyclique en C3-6, un groupe hetéroalicyclique à 4-6 chaînons, aryle en C5-8, hétéroaryle à 5-10 chaînons, -alkyl en C1-4 -(groupe alicyclique en C3-7), -alkyle en C1-4-(groupe hétéroalicyclique à 3-10 chaînons), -alkyle en C1-4- (aryle en C5-8), -alkyle en C1-4-(hétéroaryle à 5-10 chaînons), -N($R_{10}$)($R_{11}$), dans lesquels -S-alkyle en C1-4, alkyle en C1-6, haloalkyle en C1-6, alcoxyle en C1-6, haloalcoxyle en C1-6, un groupe alicyclique en C3-6, un groupe hetéroalicyclique à 4-6 chaînons, aryle en C5-8, hétéroaryle à 5-10 chaînons, -alkyl en C1-4 -(groupe alicyclique en C3-7), -alkyle en C1-4-(groupe hétéroalicyclique à 3-10 chaînons), -alkyle en C1-4- (aryle en C5-8) et -alkyle en C1-4-(hétéroaryle à 5-10 chaînons) sont éventuellement substitués chacun par 0, 1, 2, 3 ou 4 $R^{1a}$ groupes ; et $R^{1a}$ est indépendamment choisi parmi halogène, -OH, -$NO_2$, -CN, oxo, alkyle en C1-4, haloalkyle en C1-4, alcoxyle en C1-4, haloalcoxyle en C1-4, un groupe alicyclique en C3-7, un groupe hétéroalicyclique à 3-10 chaînons, aryle en C5-8 et hétéroaryle à 5-7 chaînons ; et
$R_{10}$, $R_{11}$, et $R_{12}$ sont chacun indépendamment choisi parmi : H, alkyle en C1-6, haloalkyle en C1-6, un groupe alicyclique en C3-7 et un groupe hétéroalicyclique à 3-10 chaînons, dans lesquels chacun listé dans le groupe est éventuellement substitué par 0, 1, 2, 3 ou 4 substituants choisis indépendamment dans un groupe consistant en halogène, -OH, -$NH_2$, oxo, alkyle en C1-4, hydroxyalkyle en C1-4, haloalkyle en C1-4, alcoxyle en C1-4 et haloalcoxyle en C1-4 ; ou $R_{10}$, $R_{11}$, et les atomes attachés à ceux-ci forment ensemble un cycle à 3 à 8 chaînons.

**4.** Composé selon l'une quelconque des revendications 1 à 3, ou un composé isotopiquement marqué de celui-ci, ou un isomère optique de celui-ci, un isomère géométrique de celui-ci, un tautomère de celui-ci ou un mélange de l'isomère optique, de l'isomère géométrique et du tautomère, ou un sel pharmaceutiquement acceptable de celui-ci, dans lequel $R_1$ est choisi parmi H, halogène, -OH, méthyle, éthyle, propyle, butyle, pentyle, hexyle, méthoxy, éthoxy, propoxy, butoxy, pentyloxy, hexyloxy, cyclopropyle, cyclobutyle, le cyclopentyle, cyclohexyle, cycloheptyle, phényle, pipérazinyle, pipéridinyle, morpholinyle, pyrrolidinyle, pyrrolyle, morpholinyle, pyridinyle et pyrazolyle, dans lesquels le méthyle, l'éthyle, le propyle, le butyle, le pentyle, l'hexyle, le méthoxy, l'éthoxy, le propoxy, le butoxy, le pentyloxy, l'hexyloxy, le cyclopropyle, le cyclobutyle, le cyclopentyle, le cyclohexyle, le cycloheptyle, le phényle, le pipérazinyle, le pipéridinyle, le morpholinyle, le pyrrolidinyle, le pyrrolyle, le morpholinyle, le pyridinyle et le pyrazolyle sont chacun éventuellement substitués par 1 ou 2 $R^{1a}$, et $R^{1a}$ est choisi indépendamment parmi halogène, -OH, -$NO_2$, -CN, oxo, alkyle en C1-4, haloalkyle en C1-4 et alcoxyle en C1-4 ; de préférence, $R_1$ est choisi H, méthyle, méthylpipérazinyle, pyrrolidinyle, méthylpipérazinyle, hydroxycyclobutyle, méthylpyrazolyle, hydroxyéthyle, hydroxypropyle, méthylpipéridinyle, morpholinyle et hydroxypyrrolidinyle.

**5.** Composé selon l'une quelconque des revendications 1 à 4, ou un composé isotopiquement marqué de celui-ci, ou un isomère optique de celui-ci, un isomère géométrique de celui-ci, un tautomère de celui-ci ou un mélange de l'isomère optique, de l'isomère géométrique et du tautomère, ou un sel pharmaceutiquement acceptable de celui-ci, dans lequel le composé est choisi parmi des composés 1-4, 6-7, 9-15, 22-23, 25-34, et 37-76 :

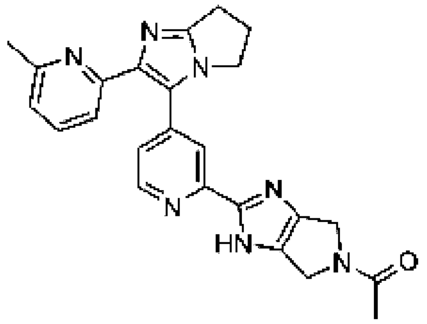

1,

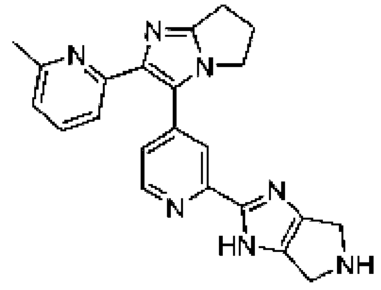

2,

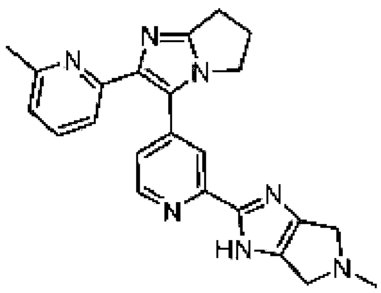

3,

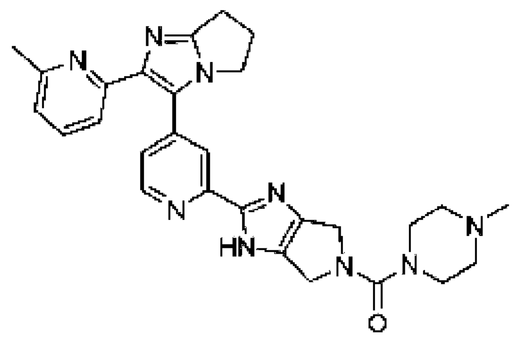

4,

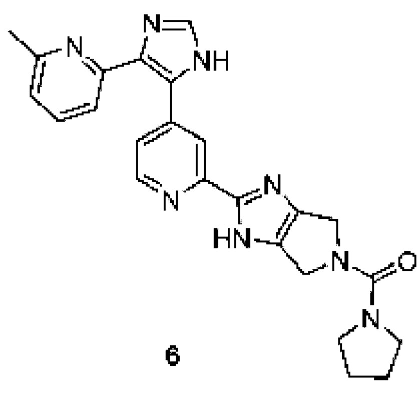

6,

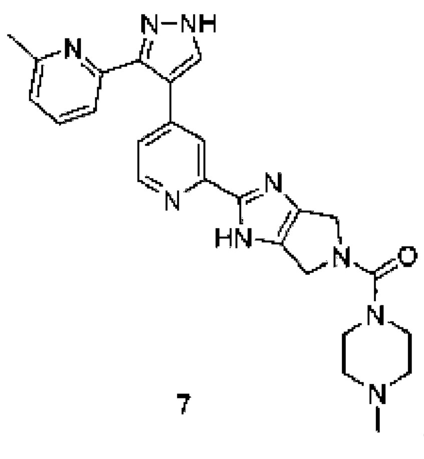

7,

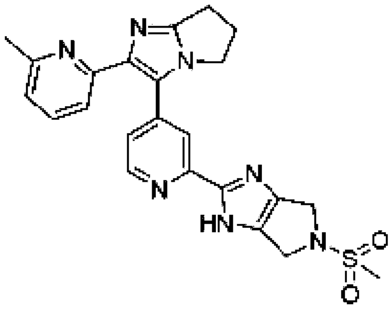

9,

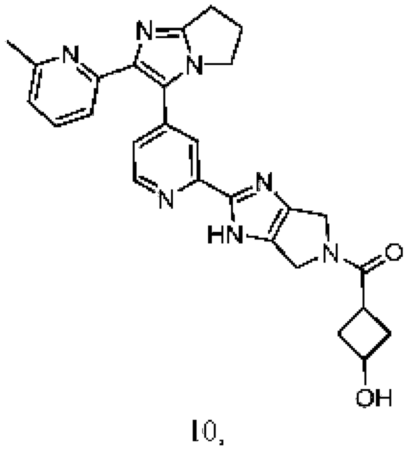

10,

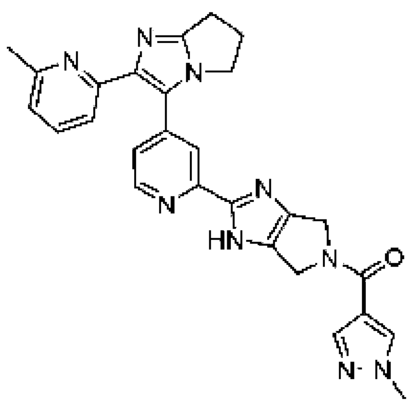

11,

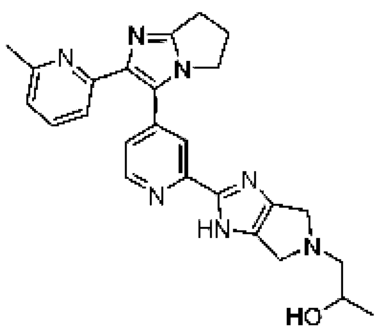

12,

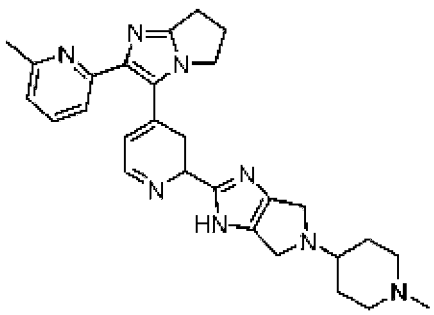

13,

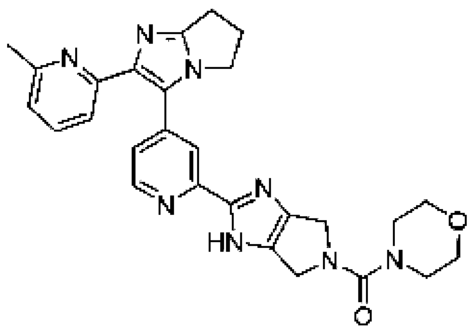

14,

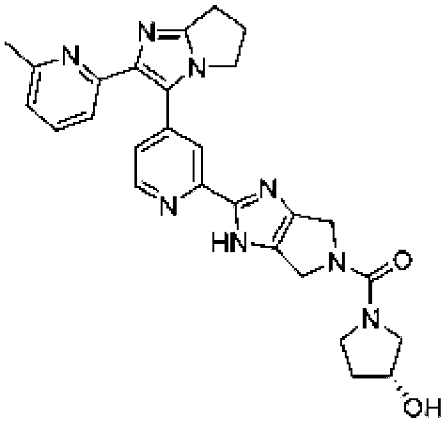

15,

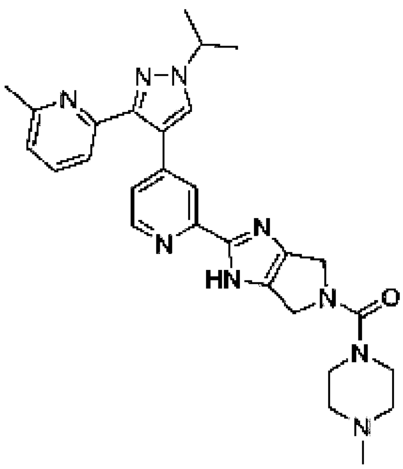

22,

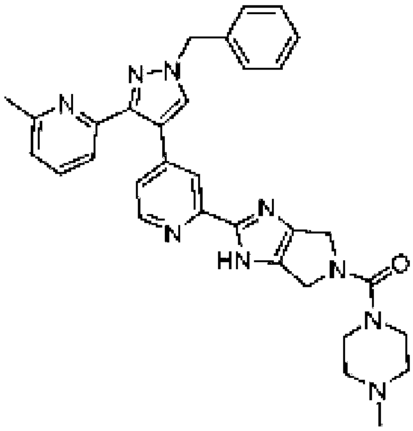
23,

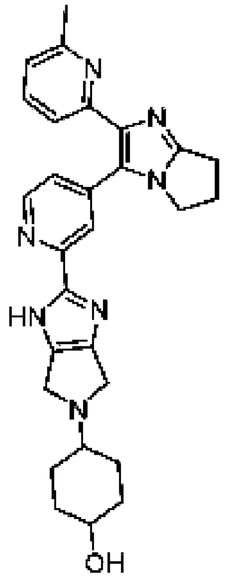
25,

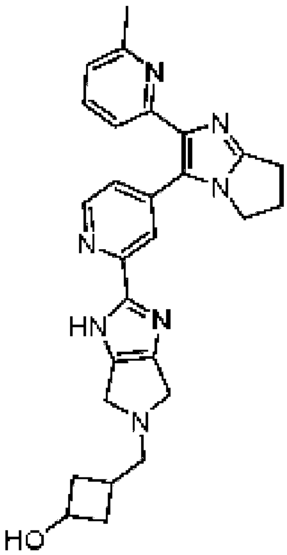
26,

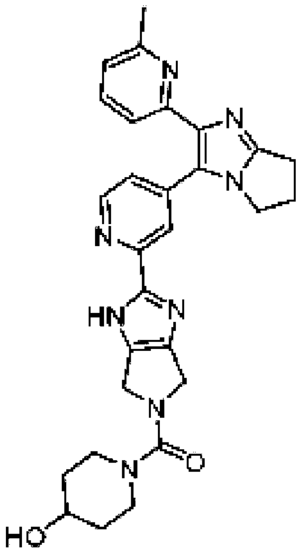
27,

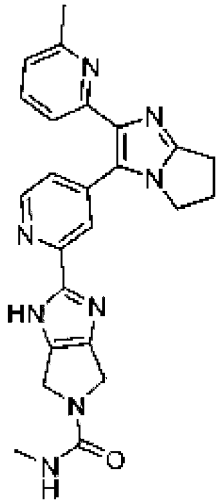

28,

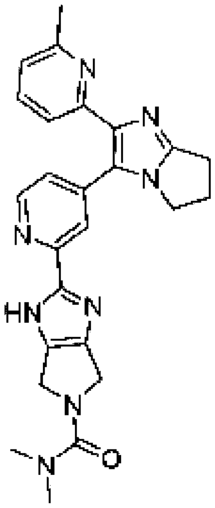

29,

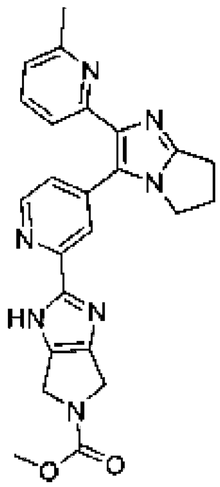

30,

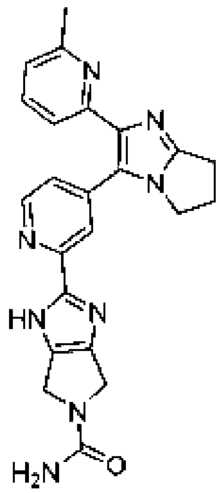

31,

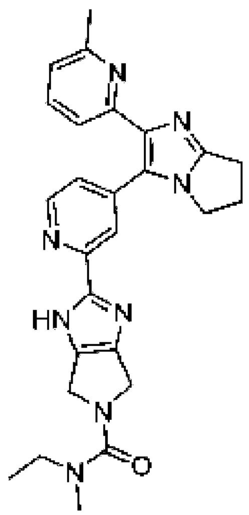

32,

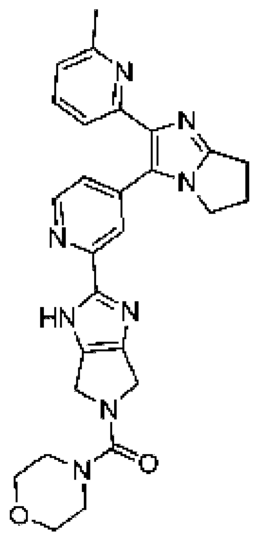

33,

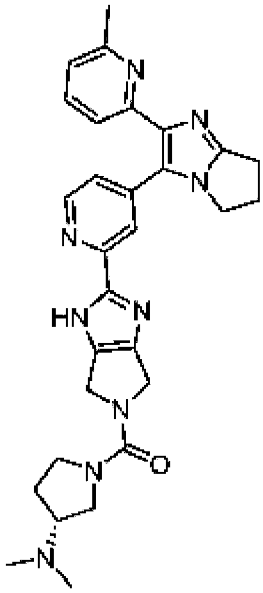

34,

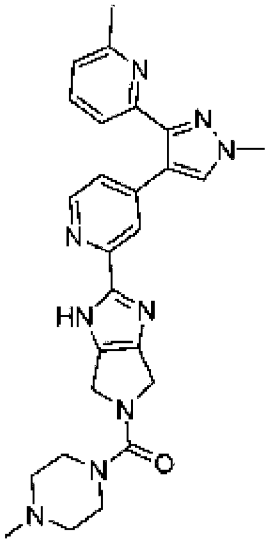

37,

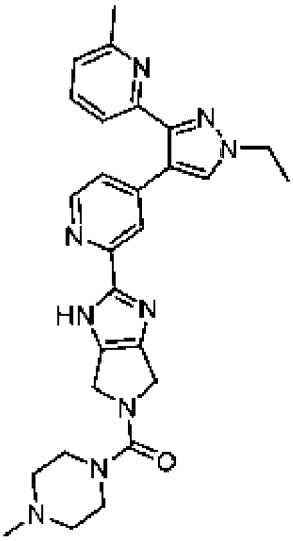

38,

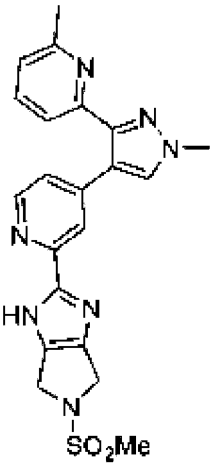

39,

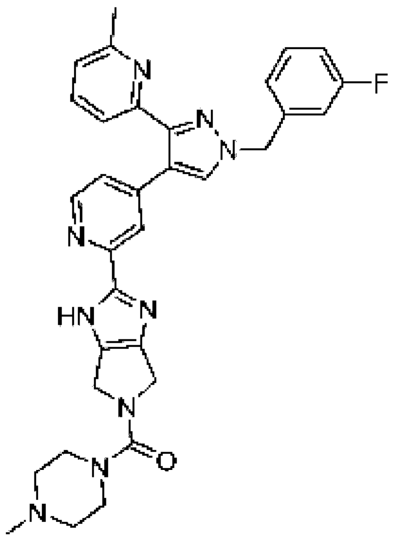

40,

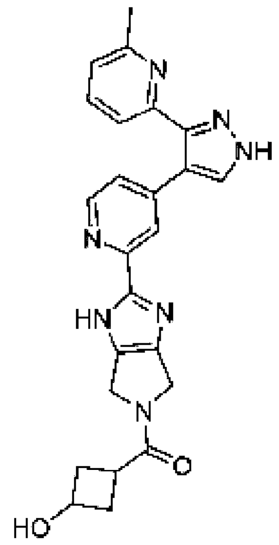

41,

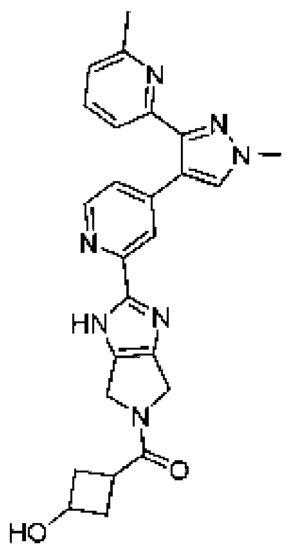

42,

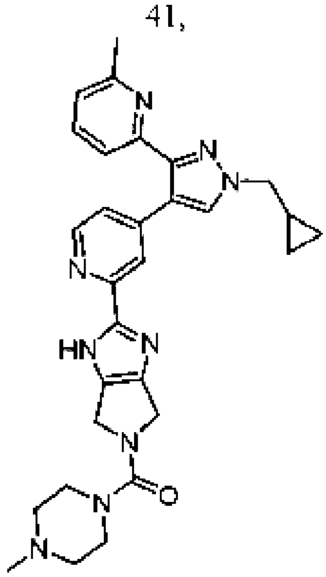

43,

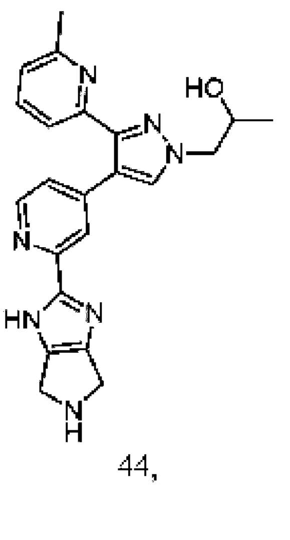

44,

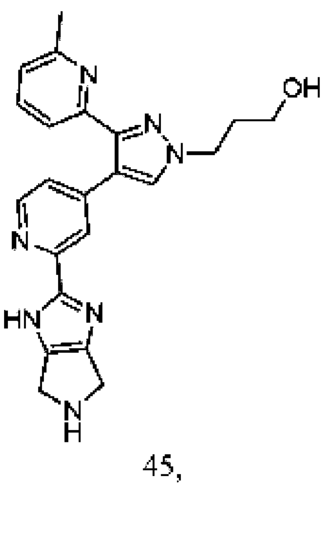

45,

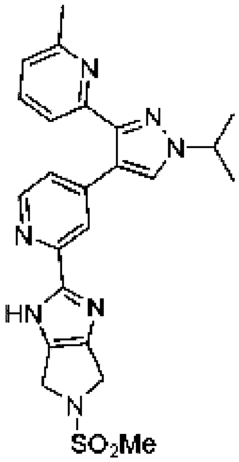

46,

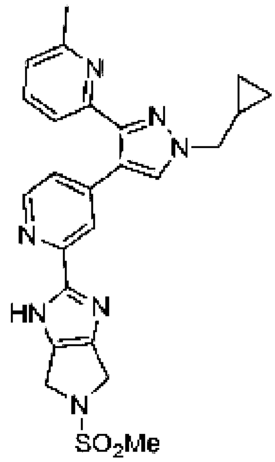

47,

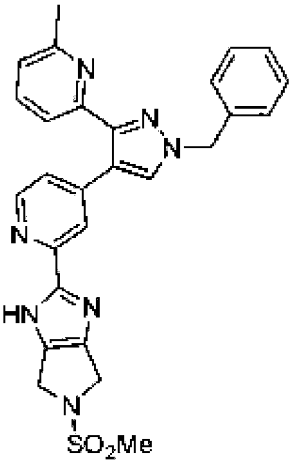

48,

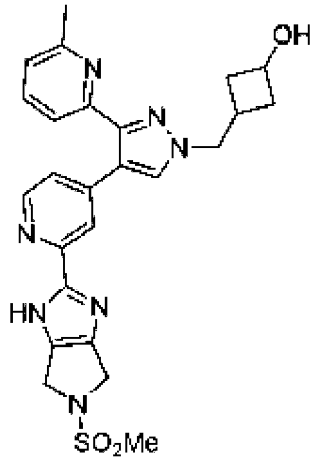

49,

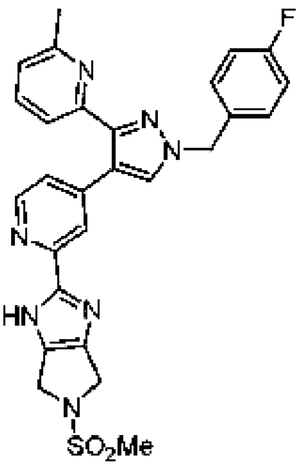

50,

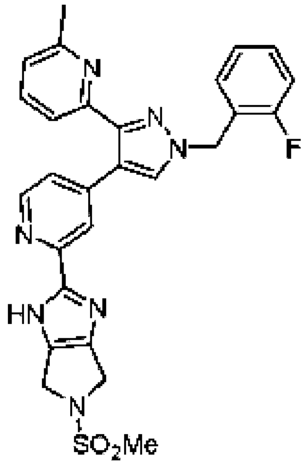

51,

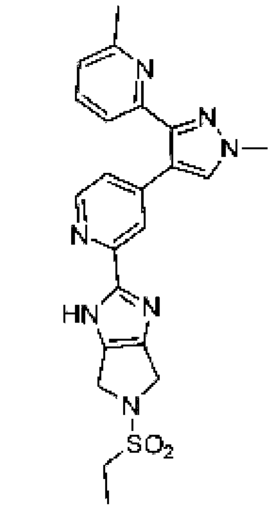

52,

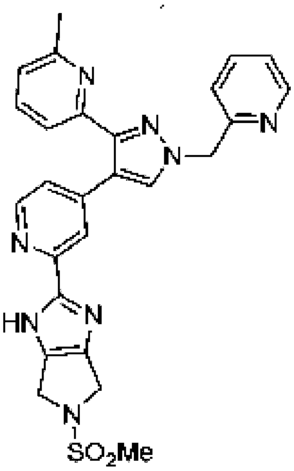

53,

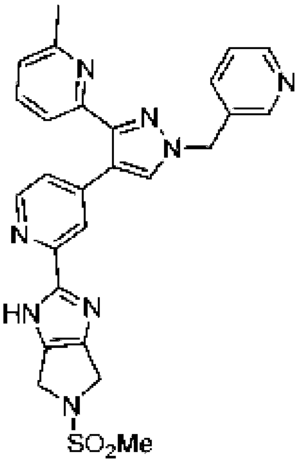

54,

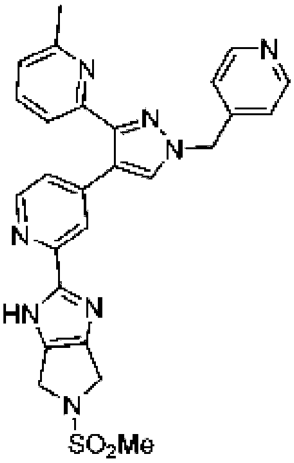

55,

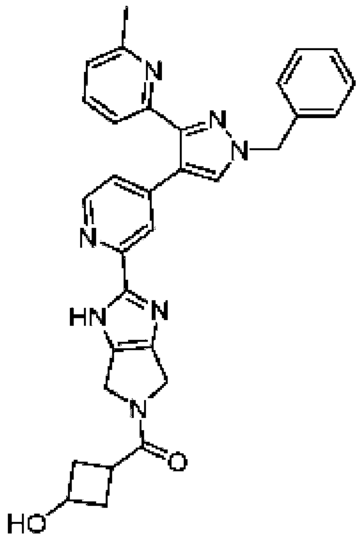

56,

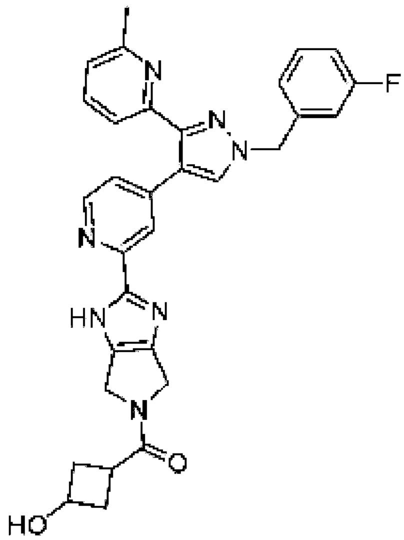

57

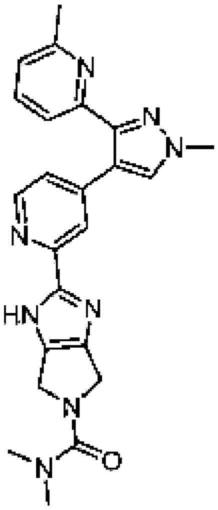

58,

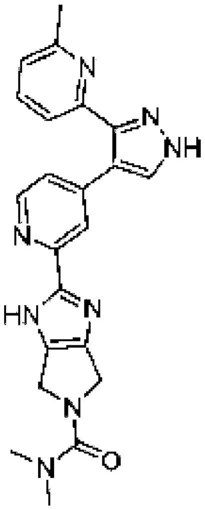

59,

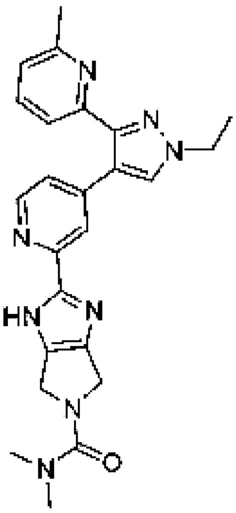

60,

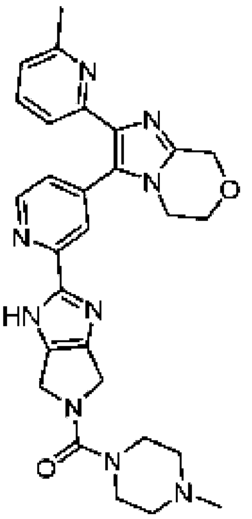

61 ,

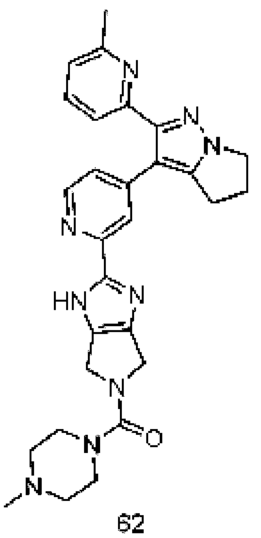

62 ,

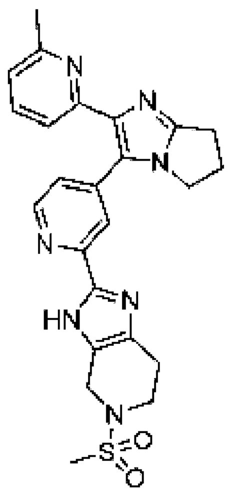

63 ,

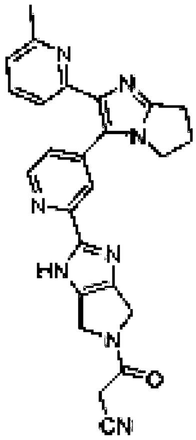
64,

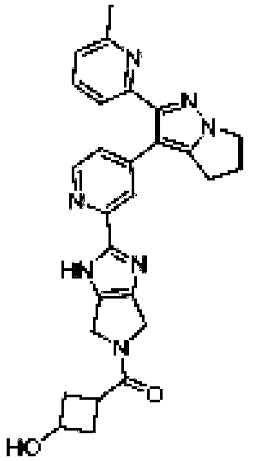
65,

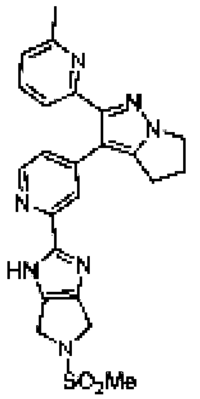
66,

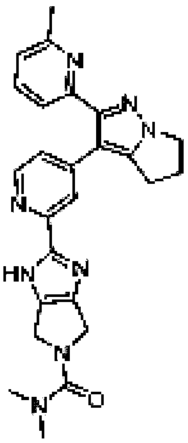
67,

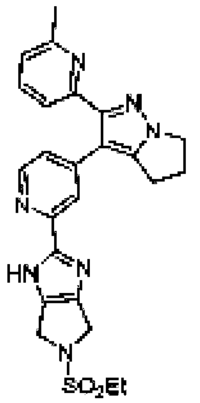
68,

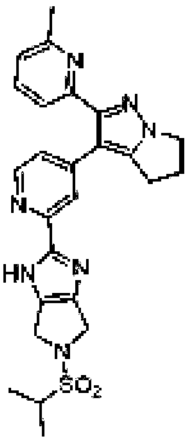
69,

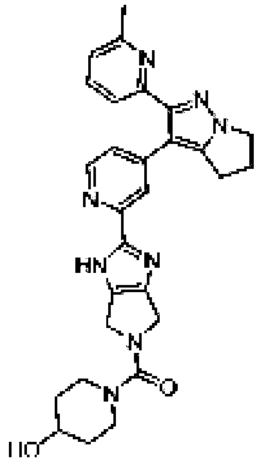

70,

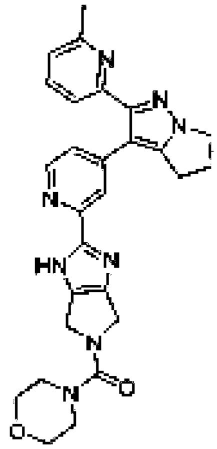

71,

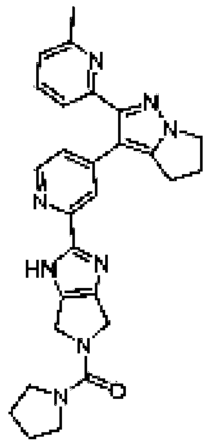

72,

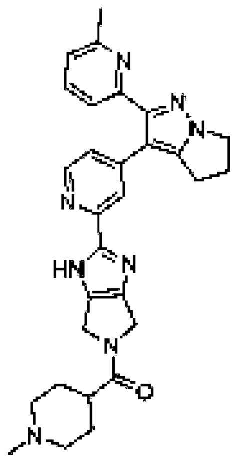

73,

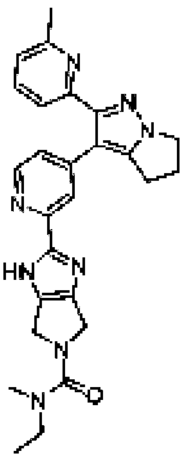

74,

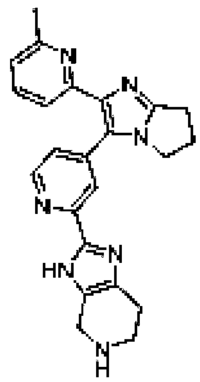

75,

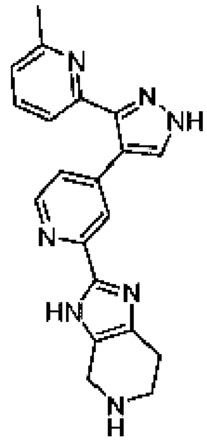

76.

6. Composition pharmaceutique comprenant le composé selon l'une quelconque des revendications 1 à 5, ou un composé isotopiquement marqué de celui-ci, ou un isomère optique de celui-ci, un isomère géométrique de celui-ci, un tautomère de celui-ci ou un mélange de divers isomères, ou un sel pharmaceutiquement acceptable de celui, et un ou plusieurs véhicules, adjuvants ou excipients pharmaceutiquement acceptables.

7. Composé selon l'une quelconque des revendications 1 à 5, ou un composé isotopiquement marqué de celui-ci, ou un isomère optique de celui-ci, un isomère géométrique de celui-ci, un tautomère de celui-ci ou un mélange de divers isomères, ou un sel pharmaceutiquement acceptable de celui pour utilisation comme médicament pour le traitement de maladies ou de conditions liées au TGF-β, de préférence au TGF-β1, dans lequel les maladies ou conditions liées au TGF-β sont choisies parmi les cancers, les infections virales, la glomérulonéphrite chronique, la glomérulonéphrite aiguë, la néphropathie diabétique, l'ostéoporose, l'arthrite, la cicatrisation des plaies, la formation de cicatrices, les ulcères, les traumatismes cornéens, la sténose des valves cardiaques, la nécrose cardiaque congestive, les dommages neurologiques, la maladie d'Alzheimer, les adhérences péritonéales ou sous-cutanées, l'athérosclérose, la fibrose cutanée et le vieillissement cutané dû à la perte de graisse, les troubles squelettiques ou des chondrocytes, les troubles hypophosphatégiques et les maladies de fibrose des organes, de préférence, les maladies ou conditions liées au TGF-β sont choisies parmi le carcinome hépatocellulaire, le cancer du sein, le cancer de la vessie, le cancer colorectal, le mélanome, le mésothéliome, le cancer du poumon, le cancer prostatique, le cancer du pancréas, le cancer testiculaire, le cancer de la thyroïde, le carcinome à cellules squameuses, le glioblastome, le neuroblastome, le cancer du col utérin, le rhabdomyosarcome, la fibrose rénale, la fibrose hépatique, la fibrose pulmonaire, la cicatrisation cutanée, la fibrose cutanée et le vieillissement cutané dû à la perte de graisse.

8. Composition pharmaceutique selon la revendication 6 pour utilisation comme médicament pour le traitement de maladies ou de conditions liées au TGF-β, de préférence au TGF-β1, dans laquelle les maladies ou conditions liées au TGF-β sont choisies parmi les cancers, les infections virales, la glomérulonéphrite chronique, la glomérulonéphrite aiguë, la néphropathie diabétique, l'ostéoporose, l'arthrite, la cicatrisation des plaies, la formation de cicatrices, les ulcères, les traumatismes cornéens, la sténose des valves cardiaques, la nécrose cardiaque congestive, les dommages neurologiques, la maladie d'Alzheimer, les adhérences péritonéales ou sous-cutanées, l'athérosclérose, la fibrose cutanée et le vieillissement cutané dû à la perte de graisse, les troubles squelettiques ou des chondrocytes, les troubles hypophosphatégiques et les maladies de fibrose des organes, de préférence, les maladies ou conditions liées au TGF-β sont choisies parmi le carcinome hépatocellulaire, le cancer du sein, le cancer de la vessie, le cancer colorectal, le mélanome, le mésothéliome, le cancer du poumon, le cancer prostatique, le cancer du pancréas, le cancer testiculaire, le cancer de la thyroïde, le carcinome à cellules squameuses, le glioblastome, le neuroblastome, le cancer du col utérin, le rhabdomyosarcome, la fibrose rénale, la fibrose hépatique, la fibrose pulmonaire, la cicatrisation cutanée, la fibrose cutanée et le vieillissement cutané dû à la perte de graisse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2004013135 A
- WO 2004016606 A
- WO 2018086609 A


### Non-patent literature cited in the description


- **STAHL** ; **WERMUTH**. Handbook of Pharmaceutical Salts: Properties, Selection and Use. Wiley-VCH, 2002